# EUROPEAN PATENT APPLICATION

(11) **EP 3 511 332 A1**
(43) Date of publication of application: **17.07.2019**
(21) Application number: 17848080.2
(22) Date of filing: 31.08.2017
(51) Int. Cl.: C07D 487/04, C07D 471/04, C07D 487/10, C07D 487/08, C07D 471/08, A61K 31/4545, A61P 7/02

(54) **NOVEL 2-ACYLAMINOTHIAZOLE DERIVATIVE AND PREPARATION METHOD THEREFOR AND USE THEREOF**

(30) Priority: 08.09.2016 CN 201610810656
(71) Applicant: Sichuan Kelun-Biotech Biopharmaceutical Co., Ltd., Chengdu, Sichuan 611138 (CN)
(72) Inventor: CAI, Jiaqiang, Sichuan 611138 (CN); YU, Nan, Sichuan 611138 (CN); ZENG, Hong, Sichuan 611138 (CN); SONG, Hongmei, Sichuan 611138 (CN); QING, Yan, Sichuan 611138 (CN); SONG, Shuai, Sichuan 611138 (CN); DENG, Hanwen, Sichuan 611138 (CN); TANG, Zujian, Sichuan 611138 (CN); DUAN, Xiaofan, Sichuan 611138 (CN); HUANG, Haitao, Sichuan 611138 (CN); YE, Hong, Sichuan 611138 (CN); LIU, Gang, Sichuan 611138 (CN); WANG, Lichun, Sichuan 611138 (CN); WANG, Jingyi, Sichuan 611138 (CN)
(74) Representative: Epping - Hermann - Fischer
(86) International application number: PCT/CN2017/099945
(87) International publication number: WO 2018/045907

(57) **Abstract**

The present invention relates to novel 2-acylaminothiazole derivatives. The present invention also relates to the preparation method for these novel 2-acylaminothiazole derivatives and intermediates thereof, and a pharmaceutical composition containing these compounds. Moreover, the present invention also relates to these novel 2-acylaminothiazole derivatives and the pharmaceutical composition containing the same for use in treatment and/or prevention of thrombopoietin receptor mediated diseases.

## Description

### Technical Field

The present invention relates to a novel 2-acylaminothiazole derivative. The present invention also relates to the preparation these novel 2-acylaminothiazole derivatives and intermediate thereof, and a pharmaceutical composition containing these compounds. Moreover, the present invention also relates to use of these novel 2-acylaminothiazole derivatives and the pharmaceutical composition containing the same in manufacture of a medicament for treatment and/or prevention of diseases mediated by thrombopoietin receptor agonist.

### Background

Thrombopoietin (TPO) receptor is one of the members of the thrombopoietin growth factor receptor family, which is characterized by a common structure of the extracellular domain, including for conserved C residues in the N-terminal portion and a WSXWS motif close to the transmembrane region (see Bazan, Proc. Natl. Acad. Sci. USA, 87: 6934-6938 (1990)). Evidence that thrombopoietin (TPO) plays a functional role in hematopoiesis includes observations that its expression is restricted to spleen, bone marrow, or fetal liver in mice (see Souyri et al., Cell, 63: 1137-1147 (1990)) and to megakaryocytes, platelets, and CD34+ cells in humans (See Methia et al., Blood, 82: 1395-1401 (1993)).

Platelet is very important in physiological hemostasis and pathological thrombosis. It is continuously produced by megakaryocytes in the living body. Megakaryocyte is a large bone marrow cell, which undergoes a process known as endomitosis whereby they replicate their nuclei but without undergoing cell division and thereby give rise to polyploid cells. In response to a decreased platelet count, the endomitotic rate increases, higher ploidy of megakaryocytes are formed, and the number of megakaryocytes may increase up to 3-fold (Harker J. Clin. Invest. 47: 458-465 (1968)). In contrast, in response to an elevated platelet count, the endomitotic rate decreases, lower ploidy megakaryocytes are formed, and the number of megakaryocytes may decrease by 50%. The exact physiological feedback mechanism by which the mass of circulating platelets regulate the endomitotic rate and number of bone marrow megakaryocytes is not known. The circulating hematopoietic factor involved in mediating this feedback loop is now thought to be thrombopoietin (TPO). More specifically, TPO has been shown to be the main humoral regulator in situations involving thrombocytopenia (Metcalf, Nature. 369:519-520 (1994)). TPO has been shown in several studies to increase platelet counts and increase platelet size. Specifically, TPO is thought to affect megakaryocyte in several ways: (1) it produces increases in megakaryocyte size and number; (2) it produces an increase in DNA content, in the form of polyploidy of megakaryocytes; (3) it increases endomitosis in megakaryocyte; (4) it produces increased maturation of megakaryocytes; and (5) it produces an increase in the percentage of precursor cells, in the form of acetylcholinesterase-positive cells, in the bone marrow.

Because platelets (thrombocytes) are necessary for blood clotting and when their numbers are very low, a patient is at risk of death from catastrophic hemorrhage. TPO has potential useful application in both the diagnosis and the treatment of various hematological disorders, for example, diseases primarily due to platelet defects (Harker et al., Blood, 91: 4427-4433 (1996)). To this end, researchers have developed a series of compounds that target thrombopoietin receptors, and it is desirable to prevent or treat diseases or conditions caused by platelet defects or reduction by promoting platelet production.

For example, WO2005/014561, WO2007/004038 and WO2009/017098 all disclose different types of 2-aminothiazole derivatives. These compounds all exhibit a certain degree of pharmacological activity of preventing the thrombocytopenia in varying degrees, but their activities have not yet reached a satisfactory level. Moreover, some of the drugs that have been approved for marketing have also shown a certain degree of side effects, which has hindered the widespread clinical application of these drugs. For example, recently approved small molecule oral TPO receptor agonist drugs, eltrombopag and lusutrombopag, produce a therapeutic effect on severe targeted aplastic anemia and chronic primary immune thrombocytopenia (ITP) through TPO receptors (Ali et al.; Blood Coagulation & Fibrinolysis, 27 (1), 4-52, (2016)). However, although eltrombopab is well tolerated by human, it has severe hepatotoxic side effects, which greatly limits its clinical application. Therefore, it is urgent need and great importance in the clinical practice to seek for a TPO receptor agonist with better effect and less side effects.

### Summary of the Invention

The object of the present invention is to provide 2-acylaminothiazole derivative that is an excellent agonist to the thrombopoietin (TPO) receptor. 2-acylaminothiazole derivative of the present invention has a low side-effect on human or the human's tissue or organ.

The present invention provides 2-acylaminothiazole derivative represented by formula (I) and an isomer or racemate thereof, a salt thereof, an ester thereof, a solvate thereof, a chemically protected form thereof, a prodrug or metabolite thereof, a crystal form thereof, and a mixture thereof. 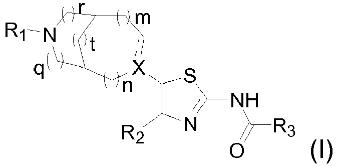 wherein,
m, n, q, and r are individually and separately selected from an integer of 0-4;
t is selected from an integer of 0-3;
X is N or C;
-̅-̅-̅ represents a single or double bond; when X is N, -̅-̅-̅ is a single bond;
wherein R₁ is selected from hydrogen, optionally substituted C₁-C₁₂alkyl, optionally substituted C₂-C₁₂alkenyl, optionally substituted C₂-C₁₂alkynyl, optionally substituted C₃-C₁₂cycloalkyl, optionally substituted C₅-C₁₂cycloalkenyl, optionally substituted C₅-C₁₂polycycliccycloalkyl, optionally substituted C₆-C₁₂polycycliccycloalkenyl, optionally substituted C₄-C₁₂fused cycloalkyl, optionally substituted C₆-C₁₂fused cycloalkenyl, optionally substituted C₆-C₁₀aryl, optionally substituted C₃-C₁₀heterocyclyl, wherein the above "optionally substituted" in the definition of R₁ refers to being unsubstituted or substituted by one or more identical or different groups selected from : C₁-C₆alkyl, C₁-C₆alkyl substituted by one or more halogens, cyano, halogen, C₁-C₆alkoxy, C₁-C₆alkoxy substituted by one or more halogens;
R₂ is selected from C₆-C₁₀aryl optionally substituted by R₄, 5- or 6-membered heteroaryl containing 1-3 identical or different heteroatoms selected from N, O and S and optionally substituted by R₄, 8- to 10-membered heteroaryl containing 1-4 identical or different heteroatoms selected from N, O and S and optionally substituted by R₄,
R₄ is selected from hydrogen, substituted or unsubstituted C₁-C₁₂alkyl, substituted or unsubstituted C₃-C₁₂cycloalkyl, halogen, cyano, nitro, substituted or unsubstituted C₁-C₁₂alkoxy, substituted or unsubstituted C₂-C₁₂alkoxyalkyl, carboxyl, carboxyl-substituted C₂-C₆alkenyl, ester group, ester group-substituted C₂-C₁₂alkenyl, R₅R₆N-, (C₁-C₁₂alkyl) C(=O)N(R₅)-, R₅R₆NC(=O)-, R₅SO, R₅SO₂, R₅R₆NSO₂; where two or more R₄ groups are present, each of R₄ groups can be identical to or different from each other;
wherein R₅ and R₆ are each independently selected from hydrogen, C₁-C₁₂alkyl and C₃-C₁₂cycloalkyl;
R₃ is selected from an aryl or heteroaryl represented by formula (II); or
R₃ is selected from a heteroaryl represented by formula (III): 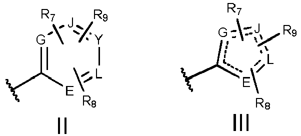
wherein J, L, G, E and Y are each independently selected from N, O, S, CH or C, wherein R₇, R₈ and R₉ are each independently selected from hydrogen, halogen, OH, cyano, nitro, carboxyl, ester group, substituted or unsubstituted C₃-C₁₀cycloalkyl, substituted or unsubstituted C₁-C₄alkyl (in particular, C₁-C₄alkyl substituted by one or more halogens), substituted or unsubstituted C₂-C₄alkenyl, C₁-C₄alkoxy (in particular, C₁-C₄alkoxy substituted by one or more halogens), R₅R₆N, substituted or unsubstituted 4-8 membered saturated heterocyclyl containing at least one atom selected from N, O, S and S(O)ₑ, e is 1 or 2;
said "substituted" refers to being substituted by one or more identical or different groups selected from : C₁-C₆alkyl, cyano, halogen, carboxyl, ester group, phosphoric acid group, phosphate ester group.

The present invention provides a compound represented by formula (I'), or an isomer or racemate thereof, a salt thereof, an ester thereof, a solvate thereof, a chemically protected form thereof, a prodrug or metabolite thereof, a crystal form thereof, and a mixture thereof: 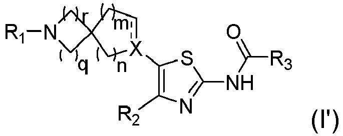 wherein m, n, q, and r are individually and separately selected from an integer of 0-4; R₁, R₂ and R₃ are defined as in formula (I).

### Detailed Description of the Invention

The embodiments of the present invention will be described below in more detail with reference to specific examples. However, those skilled in the art will understand that the following specific examples are only used to illustrate the present invention and should not be regarded as being limited to the protection scope of the present invention. In contrast, the present invention is intended to cover all alternatives, modifications, and equivalents that may be included within the scope of the present invention as defined by the claims.

### Definition

In the present invention, the expression of "Cₐ-C_{b}group" (a and b represent an integer of 1 or more, a<b) represents a-b carbon atoms are present in the "group".For example, C₁-C₄alkyl represents an alkyl having 1-4 carbon atoms; C₁-C₄alkoxy represents an alkoxy having 1-4 carbon atoms;C₃-C₁₀cycloalkyl represents a cycloalkyl having 3-10 carbon atoms;C₁-C₄alkoxyC₁-C₄alkyl represents a group obtained by bonding an alkyl having 1-4 carbon atoms and an alkoxy having 1-4 carbon atoms.

As used in the present invention, the term "alkyl" refers to a saturated linear or branched monovalent hydrocarbyl that may have 1-12 carbon atoms (C₁-C₁₂), wherein said alkyl can be optionally and independently substituted with one or more substituents as described in the context. Preferably, the alkyl can have 1-8 carbon atoms (C₁-C₈), or 1-6 carbon atoms (C₁-C₆). The example of alkyl includes but is not limited to: methyl, ethyl, 1-propyl(n-propyl), 2-propyl (isopropyl), 1-butyl(n-butyl), 2-methyl-1-propyl (isobutyl), 2-butyl (sec-butyl), 2-methyl-2-propyl (tert-butyl), 1-pentyl(n-pentyl), 2-pentyl, 3-pentyl, 2-methyl-2-butyl, 3-methyl-2-butyl, 3-methyl-1-butyl, 2-methyl-1-butyl, 1-hexyl, 2-hexyl, 3-hexyl, 2-methyl-2-pentyl, 3-methyl-2-pentyl, 4-methyl-2-pentyl, 3-methyl-3-pentyl, 2-methyl-3-pentyl, 2, 3-dimethyl-2-butyl, 3,3-dimethyl-2-butyl, 1-heptyl, 1-octyl, 1-nonyl, 1-decyl and the like. The term "alkenyl" refers to a linear or branched monovalent hydrocarbyl that can have 2-12 carbon atoms (C₂-C₁₂) and have at least one unsaturatedsite, i.e. carbon-carbon double bond (sp2 hybridization), wherein said alkenyl can be optionally and independently substituted with one or more substituents as described in the context, and comprise the groups having "cis" and "trans" orientations or "E" and "Z" orientations. Preferably, the alkenyl has 2-8 carbon atoms or 2-6 carbon atoms. The example includes but is not limited to ethenyl, propenyl, 1-butenyl, 2-butenyl, 2-methylpropenyl, pentenyl, hexenyl or the like.

The term "alkynyl" refers to a linear or branched monovalent hydrocarbyl that can have 2-12 carbon atoms and have at least one unsaturatedsite, i.e., carbon-carbon triple double (sp hybridization), wherein said alkynyl can be optionally and independently substituted with one or more substituents as described in the context. Preferably, the alkynyl has 2-8 carbon atoms or 1-6 carbon atoms. The example includes but is not limited to ethynyl, propynyl, butynyl, pentynyl, hexynyl or the like.

The term "alkoxy" refers to a group of alkyl-O-, for example it can be C₁-C₁₂alkoxy, i.e., a group of C₁-C₁₂alkyl-O-, or can be a group of C₁-C₄alkyl-O-, i.e., a group of C₁-C₄alkyl-O-. Its example includes but is not limited to methoxy, ethoxy, propoxy, iso-propoxy, butoxy, iso-butoxy, sec-butoxy, tert-butoxy, pentoxy, iso-pentoxy, neo-pentoxy, hexoxy or the like.

The term "alkoxyalkyl" refers to a group of alkyl-O-alkyl; C₂-C₁₂alkoxyalkyl refers to a group in which the total number of carbon atoms is 2-12, i.e., the sum of carbon atoms in alkoxy and alkyl is 2-12. The example of C₂-C₁₂alkoxyalkyl comprises CH₃OCH₂-, CH₃(CH₂)₃OCH₂-, CH₃OCH(CH₃)- or the like.

The term "alkylthio" refers to a group of alkyl-S-, and for example it can be C₁-C₁₂alkylthio, i.e. a group of C₁-C₁₂alkyl-S-.

The term "halogen" refers to Cl, F, Br or I.

The term "cycloalkyl" refers to a saturated monovalent hydrocarbyl that can have one or more C₃-C₁₂monocycles (e.g., monocyclic ring, fused ring, bridged ring, spiro ring or the like). The example of cycloalkyl comprises cyclopropyl, cyclobutyl, cyclopentyl, cyclohexyl, bicyclo[2.1.1]hexyl, bicyclo[2.2.1]heptyl, bicyclo[2.2.2]octyl, bicyclo[3.2.1]octyl, bicyclo[4.3.1]decyl, bicyclo[3.3.1]nonyl, bornyl, bornylenyl, norbornyl, norbornylenyl, 6,6-dimethylbicyclo[3.1.1]heptyl, tricyclobutyl and adamantanyl or the like.

The term "polycyclic cycloalkyl" refers to a group obtained by removing one hydrogen atom attached to a carbon atom from a structure having a total carbon atom number of 5-12 and formed by connecting a bridge between at least one pair of nonadjacent carbon atoms of a cycloalkane, and for example, adamantanyl, norbornyl, and cubanyl can be exemplified; or
a cycloalkyl having a total carbon atom number of 5-12 and formed from two cycloalkyl groups connected by one shared carbon atom, and for example, spiropentyl, and spiro[3.5]nonyl can be exemplified.

The term "fused cycloalkyl" refers to a group obtained by removing one hydrogen atom attached to a carbon atom from a system formed by fusing two or more cycloalkanes through sharing one pair of adjacent carbon atoms. The carbon atom number can be 4-12(C₄-C₁₂fused cycloalkyl), and for example decahydronaphthalene or the like can be exemplified.

The term "cycloalkenyl" refers to a monovalent hydrocarbyl that contains one or more C₃-C₁₂monocycles (e.g., monocyclic ring, fused ring, bridged ring, spiro ring or the like) having one or more carbon-carbon double bonds (sp2 hybridization). The example of cycloalkenyl comprises cyclopentenyl and cyclohexenyl or the like.

The term "polycyclic cycloalkenyl" refers to a group that is the same to the above polycycliccycloalkyl except for having at least one double bond. It can be C₆-C₁₂polycycliccycloalkenyl. Its example includes but is not limited to norbornylenyl, norbornylenyl, indenyl or the like.

The term "fused cycloalkenyl" refers to a group that is the same to the above fused cycloalkyl except for having at least one double bond. Its carbon atom number can be 6-12(C₆-C₁₂fused cycloalkenyl), and for example, hexahydronaphthyl or the like can be exemplified.

The term "hydrogen" and the hydrogen in various groups comprise various isotopes of hydrogen, e.g. protium (H), deuterium (D), and tritium (T).

The term "aryl" refers to a monovalent aromatic hydrocarbyl that can have 6-20 carbon atoms (C₆-C₂₀) and is derived by removing one hydrogen atom from a single carbon atom of a parent aromatic ring system. The aryl comprises bicyclic or polycyclic group containing an aromatic ring fused to a saturated or partially unsaturatedring or an aromatic carbocyclic ring. The typical aryl includes but is not limited to: phenyl, naphthyl, anthryl, biphenylyl, indenyl, indanyl, 1,2-dihydronaphthyl, 1,2,3,4-tetrahydronaphthyl or the like. The aryl can be optionally and independently substituted with one or more substituents as described in the context.

The term "heterocyclyl" comprises aliphatic heterocyclyl and heteroaryl. Wherein, the term "aliphatic heterocyclyl" refers to a cyclic group, which is completely saturated or can contain one or more unsaturated units (in order to avoid the doubt, the degree of unsaturation will not result in the formation of aromatic ring system), and can have 3-20 carbon atoms and 1-3 hetero atoms such as N, O or S. It includes but is not limited to fused ring, bridged ring or spiro ring. It is also called as saturated heterocyclyl. The example of aliphatic heterocyclyl includes: azepinyl, azetidinyl, indolinyl, isoindolinyl, morpholinyl, piperazinyl, piperidinyl, pyrrolidinyl, quinuclidinyl, thiomorpholinyl, tetrahydropyranyl, teterhydrofuryl, tetrahydroindolyl, thiomorpholinyl, azanorbornyl, quinuclidinyl, isoquinuclidinyl, tropanyl, azabicyclo[3.2.1]octyl, azabicyclo[2.2.1]heptyl, 2-azabicyclo[3.2.1]octyl, azabicyclo[3.2.1]octyl, azabicyclo[3.2.2]nonyl, azabicyclo[3.3.0]nonyl and azabicyclo[3.3.1]nonyl.

The term "heteroaryl" refers to a monovalent aromatic group that can be a 5-, 6- or 7-membered ring, and a fused ring system that can comprise 5-20 atoms (in which at least one ring is aromatic). It contains 1-3 heteroatoms independently selected from N, O and S. The example of heteroaryl comprises: pyridinyl(including, for example, 2-hydroxypyridinyl), imidazolyl, imidazopyridinyl, pyrimidinyl(including, for example, 4-hydroxypyrimidinyl), pyrazolyl, triazolyl, pyrazinyl, tetrazolyl, furyl, thienyl, isoxazolyl, thiazolyl, oxadiazolyl, oxazolyl, isothiazolyl, pyrrolyl, quinolinyl, isoquinolinyl, tetrahydroisoquinolinyl, indolyl, benzoimidazolyl, benzofuryl, cinnolinyl, indazolyl, indolizinyl, phthalazinyl, pyridazinyl, triazinyl, isoindolyl, pteridinyl, purinyl, oxadiazolyl, triazolyl, thiadiazolyl, furazanyl, benzofurazanyl, benzothienyl, benzothiazolyl, benzoxazolyl, quinazolinyl, quinoxalinyl, naphthyridinyl, and furopyridinyl. The heteroaryl is optionally and independently substituted with one or more substituents as described in the context.

The term "amino protection group" refers to a chemical group which is attached to the amino group and is easily removed in a certain condition. It includes but is not limited to alkoxycarbonyls, acyls, alkyls; for example tert-butyloxycarbonyl, benzyloxycarbonyl, fluorene-methoxycarbonyl, allylloxycarbonyl, phthalyl, benzyl, para-methoxybenzyl, triphenylmethyl or the like. It can be appropriately selected and manipulated by those skilled in the art with reference to the conventional textbook in the art, such as Greene's Protective Groups in Organic Synthesis (4th editi*on).*

The term "optionally substituted" or "(being) optionally substituted" represents any part of a moiety to be ready for substitution as known by those skilled in the art can be unsubstituted or substituted by the substituent as described herein. If more than one substiuent is present, each substitutent is independently selected.

The term "treatment and/or prevention" refers to therapeutic treatment or prophylactic or preventative or preventive measure, wherein the goal is to prevent or alleviate (mitigate) undesired pathological changes or conditions, such as the development or spread of cancer. For the purposes of the present invention, beneficial or desirable clinical outcomes include, but are not limited to, mitigation of symptoms, reduction in disease severity, delay or slowing of disease progression, amelioration or mitigation of disease states, and remission (either in part or in whole), regardless of being detectable or undetectable.

The phrase "therapeutically effective amount" refers to an amount of the compound according to the present invention, which is capable of (i) treating or preventing diseases or conditions described herein, (ii) attenuating, ameliorating or eliminating one or more diseases or conditions described herein, or (iii) preventing or delaying the onset of one or more symptoms of diseases or conditions described herein.

As used herein, the phrase "pharmaceutically acceptable salt" refers to a pharmaceutically acceptable organic or inorganic salt of the compound of the present invention. Exemplary salts include, but are not limited to, hydrochloride, phosphate; or ammonium salt (e.g., primary amine salt, secondary amine salt, tertiary amine salt), metal salt (e.g., sodium salt, potassium salt).

If the compound of the present invention is a base, the desired pharmaceutically acceptable salt can be prepared by any suitable method available in the art. For example, the free base is treated with an inorganic acid such as hydrochloric acid, phosphoric acid and the like, or an organic acid such as trifluoroacetic acid, and the like.

If the compound of the present invention is an acid, the desired pharmaceutically acceptable salt can be prepared by any suitable method. For example, the free acid is treated with an inorganic or organic base such as an amine, an alkali metal hydroxide or an alkaline earth metal hydroxide or the like. The example of suitable salt includes, but is not limited to, an organic salt derived from ammonia, primary amine, secondary amine, tertiary amine, cyclic amine such as piperidine, morpholine and piperazine, and an inorganic salt derived from sodium and potassium.

The phrase "pharmaceutically acceptable" means that the substance or composition must be pharmaceutically and/or toxicologically compatible with other ingredients contained in the formulation and/or pharmaceutical composition.

"Solvate" refers to a complex formed from one or more solvent molecules and the compound of the present invention. The example of the solvent that forms the solvate includes, but is not limited to, water, isopropanol, ethanol, methanol, dimethyl sulfoxide (DMSO), ethyl acetate, acetic acid. When the solvent is water, a hydrate is formed.

In particular, the present invention provides a compound of formula (I) and an isomer or racemate thereof, a salt thereof, an ester thereof, a solvate thereof, a chemically protected form thereof, a prodrug or metabolite thereof, a crystal form thereof, and a mixture thereof, 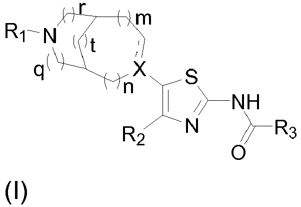 wherein,
m, n, q, and r are individually and separately selected from an integer of 0-4;
t is selected from an integer of 0-3;
X is N or C;
-̅-̅-̅ represents a single or double bond; when X is N, -̅-̅-̅ is a single bond;
wherein R₁ is selected from hydrogen, optionally substituted C₁-C₁₂alkyl, optionally substituted C₂-C₁₂alkenyl, optionally substituted C₂-C₁₂alkynyl, optionally substituted C₃-C₁₂cycloalkyl, optionally substituted C₅-C₁₂cycloalkenyl, optionally substituted C₅-C₁₂polycycliccycloalkyl, optionally substituted C₆-C₁₂polycycliccycloalkenyl, optionally substituted C₄-C₁₂fused cycloalkyl, optionally substituted C₆-C₁₂fused cycloalkenyl, optionally substituted C₆-C₂₀aryl, optionally substituted C₃-C₂₀heterocyclyl, wherein the above "optionally substituted" in the definition of R₁ refers to being unsubstituted or substituted by one or more identical or different groups selected from : C₁-C₆alkyl, C₁-C₆alkyl substituted with one or more halogens, cyano, halogen, C₁-C₆alkoxy or C₁-C₆alkoxy substituted with one or more halogens;
R₂ is selected from C₆-C₁₀aryl optionally substituted by R₄, 5- or 6-membered heteroaryl optionally substituted by R₄ and containing 1-3 identical or different heteroatoms selected from N, O and S, 8- to 10-membered heteroaryl optionally substituted by R₄ and containing 1-4 identical or different heteroatoms selected from N, O and S,
R₄ is selected from hydrogen, substituted or unsubstituted C₁-C₁₂alkyl, substituted or unsubstituted C₃-C₁₂cycloalkyl, halogen, cyano, nitro, substituted or unsubstituted C₁-C₁₂alkoxy, substituted or unsubstituted C₂-C₁₂alkoxyalkyl, carboxyl, carboxyl-substituted C₂-C₆alkenyl, ester group, ester group-substituted C₂-C₁₂alkenyl, R₅R₆N-, (C₁-C₁₂alkyl) C(=O)N(R₅)-, R₅R₆NC(=O)-, R₅SO, R₅SO₂, R₅R₆NSO₂; where two or more R₄ groups are present, each of R₄ groups can be identical to or different from each other,
wherein R₅ and R₆ are each independently selected from hydrogen, C₁-C₁₂alkyl and C₃-C₁₂cycloalkyl,
R₃ is selected from an aryl or heteroaryl represented by formula (II);
alternatively, R₃ is selected from a heteroaryl represented by formula (III): 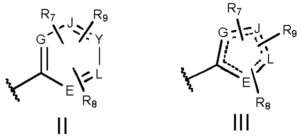
wherein J, L, G, E and Y are each independently selected from N, O, S, CH or C wherein R₇, R₈ and R₉ are each independently selected from hydrogen, halogen, OH, cyano, nitro, carboxyl, ester group, substituted or unsubstituted C₃-C₁₀cycloalkyl, substituted or unsubstituted C₁-C₄alkyl (in particular, C₁-C₄alkyl substituted by one or more halogens), substituted or unsubstituted C₂-C₄alkenyl, C₁-C₄alkoxy (in particular, C₁-C₄alkoxy substituted by one or more halogens), R₅R₆N, substituted or unsubstituted 4-8 membered saturated heterocyclyl containing at least one atom selected from N, O, S and S(O)ₑ, e is 1 or 2;
said "substituted" refers to being substituted by one or more identical or different groups selected from : C₁-C₆alkyl, cyano, halogen, carboxyl, ester group, phosphoric acid group, phosphate ester group.

The present invention provides a compound represented by formula (I'), or an isomer or racemate thereof, a salt thereof, an ester thereof, a solvate thereof, a chemically protected form thereof, a prodrug or metabolite thereof, a crystal form thereof, and a mixture thereof: 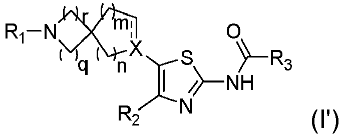 wherein m, n, q, and r are individually and separately selected from an integer of 0-4; R₁, R₂ and R₃ are defined as in formula (I).

In a preferable embodiment of the present invention, X is N, and -̅-̅-̅ is a single bond.

In an embodiment, the group 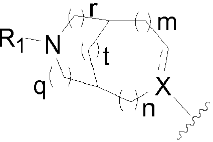 is as shown in formula (IV): 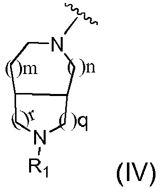 wherein m, n, q, and r are individually and separately selected from an integer of 0-4, e.g. 0, 1, 2, 3 or 4; R₁ is defined as in formula (I).

In another embodiment, the group 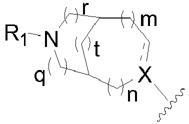 is as shown in formula (V): 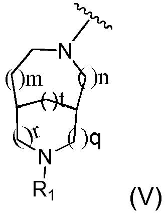 wherein m, n, q, and r are independently selected from an integer of 0-4, e.g. 0, 1, 2, 3 or 4; t is an integer of 1-3, e.g. 1, 2 or 3; R₁ is defined as in formula (I).

In a preferable embodiment of the present invention, R₁ is selected from substituted or unsubstituted C₃-C₁₀alkyl, substituted or unsubstituted C₃-C₁₀alkenyl, substituted or unsubstituted C₃-C₁₀cycloalkyl, substituted or unsubstituted C₅-C₁₀cycloalkenyl, substituted or unsubstituted C₅-C₁₂polycycliccycloalkyl, substituted or unsubstituted C₆-C₁₂polycycliccycloalkenyl, substituted or unsubstituted C₄-C₁₂fused cycloalkyl, substituted or unsubstituted C₆-C₁₂fused cycloalkenyl, substituted or unsubstituted 4-8 membered saturated heterocyclyl containing at least one heteroatom selected from R₇N, O and S, substituted or unsubstituted 5-membered or 6-membered or 8-membered to 10-membered heteroaryl containing 1-4 identical or different heteroatoms selected from N, O and S.

Preferably, R₁ is selected from substituted or unsubstituted C₁-C₁₀alkyl, substituted or unsubstituted C₃-C₁₀alkenyl, substituted or unsubstituted C₃-C₁₀cycloalkyl, substituted or unsubstituted C₅-C₁₀cycloalkenyl, substituted or unsubstituted C₅-C₁₂polycycliccycloalkyl, substituted or unsubstituted C₆-C₁₂polycycliccycloalkenyl, substituted or unsubstituted C₄-C₁₂fused cycloalkyl, substituted or unsubstituted C₆-C₁₂fused cycloalkenyl, substituted or unsubstituted 4-8 membered saturated heterocyclyl containing at least one group selected from R₁₀N, O and S, substituted or unsubstituted 5-membered or 6-membered or 8-membered to 10-membered heteroaryl containing 1-4 identical or different heteroatoms selected from N, O and S, R₁₀ can be selected from hydrogen, C₁-C₁₂alkyl or C₃-C₁₂cycloalkyl;
more preferably, R₁ is selected from methyl, ethyl, propyl, iso-propyl, butyl, iso-butyl, tert-butyl, cyclopropyl, cyclobutyl, cyclopentyl, cyclohexyl, 2, 2, 2-trifluoroethyl, 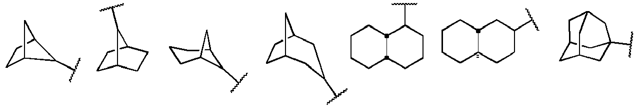 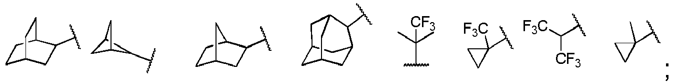 more preferably, R₁ may be further selected from cycloheptyl, piperidinyl, methylpiperidinyl, tetrahydropyranyl, methylcyclopentyl, pyrrolyl,

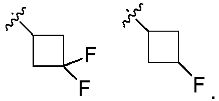

Further preferably, R₁ is selected from methyl, ethyl, cyclopropyl, iso-propyl, butyl, iso-butyl, tert-butyl, cyclopropyl, cyclobutyl, cyclopentyl, cyclohexyl, or

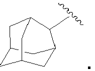

In a preferable embodiment of the present invention, R₂ is selected from C₆-C₁₀aryl optionally substituted by R₄, 5- or 6-membered heteroaryl optionally substituted by R₄ and containing 1-3 identical or different heteroatoms selected from N, O and S, 8- to 10-membered heteroaryl optionally substituted by R₄ and containing 1-4 identical or different heteroatoms selected from N, O and S; R₄ is selected from hydrogen, halogen, cyano, nitro, C₁-C₄alkyl, C₁-C₄alkoxy, C₃-C₁₂cycloalkyl, C₁-C₄alkyl substituted by one or more halogens, C₁-C₄alkoxy substituted by one or more halogens, C₁-C₄alkoxyalkyl, C₂-C₁₂alkoxyalkyl, alkoxyalkyl substituted by C₃-C₁₂cycloalkyl; Preferably, R₂ is selected from the following groups optionally substituted by R₄: phenyl, naphthyl, or the following groups optionally substituted by R₄: pyridinyl, imidazolyl, imidazopyridinyl, pyrimidinyl, pyrazolyl, triazolyl, pyrazinyl, tetrazolyl, furyl, thienyl, isoxazolyl, thiazolyl, oxadiazolyl, oxazolyl, isothiazolyl, pyrrolyl, quinolinyl, isoquinolinyl, tetrahydroisoquinolinyl, indolyl, benzoimidazolyl, benzofuryl, cinnolinyl, indazolyl, indolizinyl, phthalazinyl, pyridazinyl, triazinyl, isoindolyl, pteridinyl, purinyl, oxadiazolyl, triazolyl, thiadiazolyl, furazanyl, benzofurazanyl, benzothienyl, benzothiazolyl, benzoxazolyl, quinazolinyl, quinoxalinyl, naphthyridinyl and furopyridinyl. Wherein, R₄ is selected from hydrogen, halogen, C₁-C₄alkyl, C₁-C₄alkyl substituted by one or more halogens;

Further preferably, R₂ is selected from the following groups optionally substituted by R₄: phenyl, naphthyl; the following groups optionally substituted by R₄: furyl, thienyl, pyrrolyl, oxazolyl, thiazolyl, pyridinyl, pyrimidinyl, pyridazinyl, pyrazinyl, indolyl, quinolinyl, purinyl, wherein R₄ is selected from hydrogen, CI, F, Me, CF₃;

Further preferably, R₂ is selected from

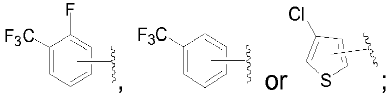

Particularly preferably, R₂ is selected from

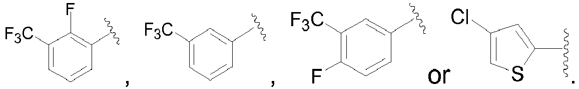

In a preferable embodiment of the present invention, R₃ is selected from formula VI, formula VII, formula VIII 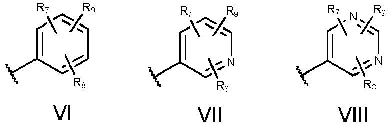 wherein R₇, R₈, and R₉ is selected from hydrogen, halogen, OH, cyano, nitro, carboxyl, ester group, C₃-C₆cycloalkyl, substituted or unsubstituted C₁-C₄alkyl, substituted or unsubstituted C₂-C₄alkenyl, C₁-C₄alkyl substituted by one or more halogens, substituted or unsubstituted C₁-C₄alkoxy, C₁-C₄alkoxy substituted by more than one halogen, R₅R₆N, substituted or unsubstituted 4-8 membered saturated heterocyclyl containing at least one heteroatom selected from N, O, S, and S(O)ₑ, e is 1 or 2; Preferably, R₃ is selected from 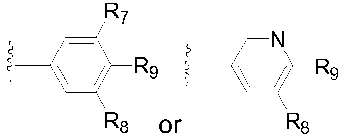 wherein R₇ and R₈ are selected from Me, Et, CF₃, CI, Br, F, cyclopropyl; Rg is selected from carboxyl, ester group, OH, NH₂, halogen, C₁-C₁₀alkyl containing a substituent of carboxyl or ester group, C₂-C₄alkenyl containing a substituent of carboxyl or ester group, C₁-C₁₀alkoxy containing a substituent of carboxyl or ester group, C₁-C₁₀alkylamino containing a substituent of carboxyl or ester group, C₁-C₁₀alkylthio containing a substituent of carboxyl or ester group, C₄-C₁₀heterocyclyl containing a substituent of carboxyl or ester group;

Further preferably, R₃ is selected from

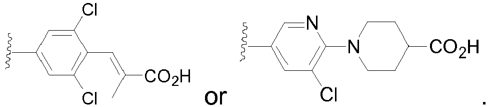

In one embodiment of the present invention, m, n, q and r are not 0 simultaneously.

In a preferable embodiment, the compound of the present invention is selected from : 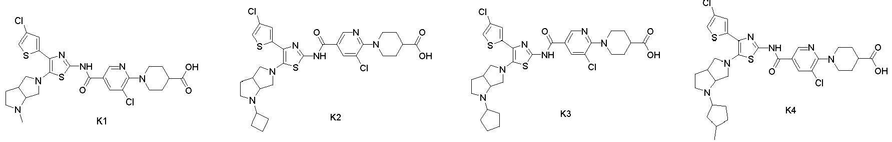 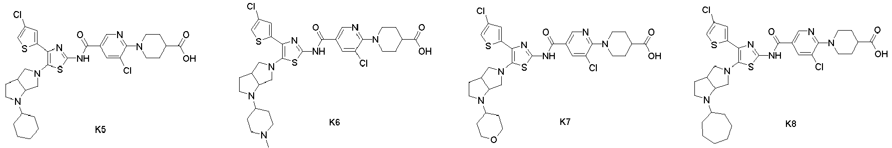 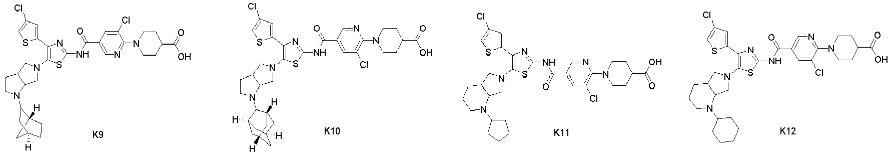 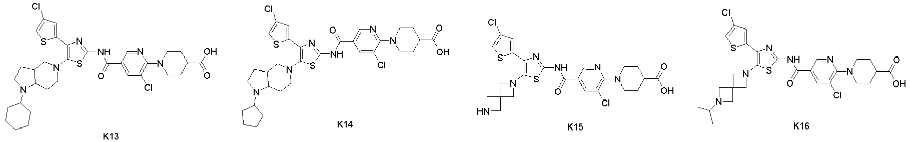 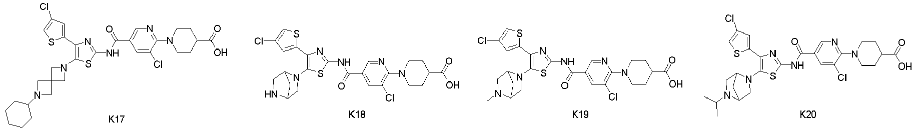 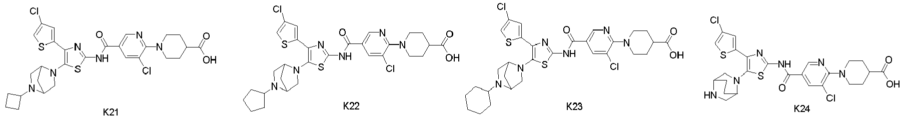 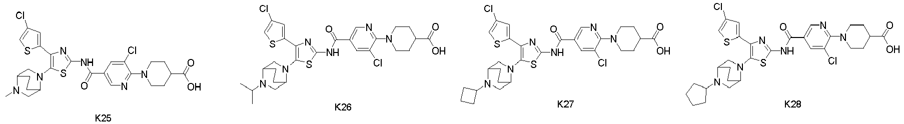 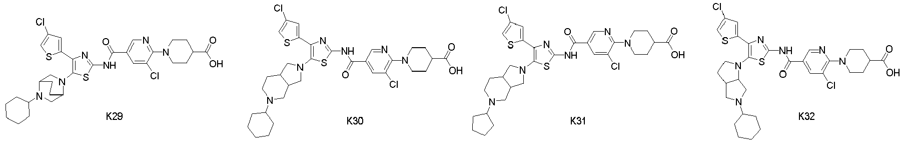 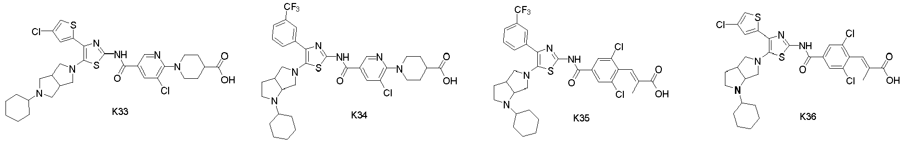 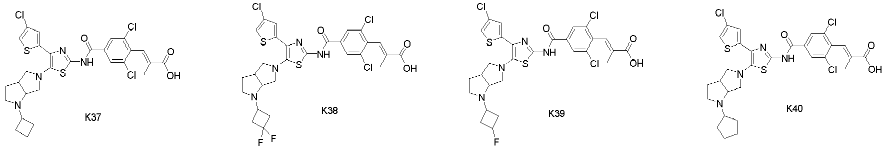 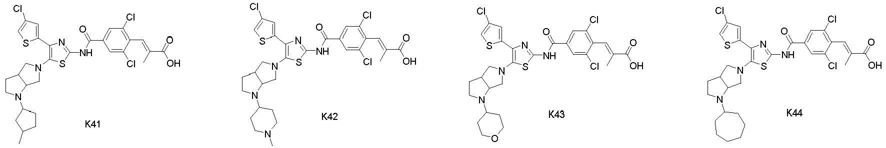 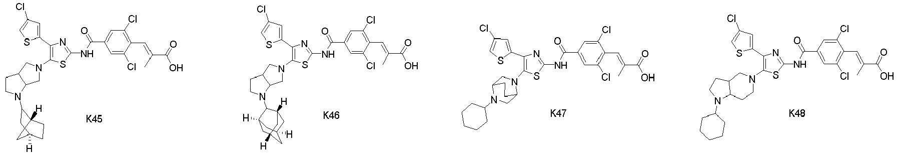 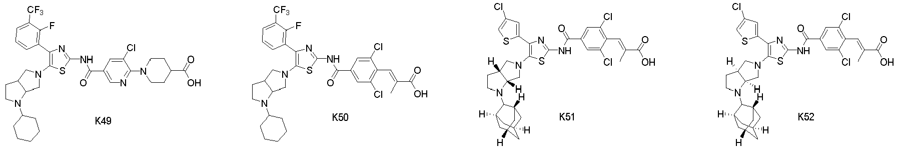 or an isomer or racemate thereof, a salt thereof, an ester thereof, a solvate thereof, a chemically protected form thereof, a prodrug or metabolite thereof, a crystal form thereof, and a mixture thereof.

The present invention provides a compound represented by formula (IX), or an isomer or racemate thereof, a salt thereof, an ester thereof, a solvate thereof, a chemically protected form thereof, a prodrug or metabolite thereof, a crystal form thereof, and a mixture thereof: 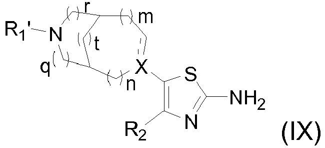
wherein m, n, q, and r are individually and separately selected from an integer of 0-4;
t is selected from an integer of 0-3;
X is N or C;
-̅-̅-̅ represents a single or double bond; when X is N, -̅-̅-̅ is a single bond;
R₁' is an amino protection group or hydrogen;
R₂ is defined as in formula (I).Identical to the above definition;

In an embodiment, the group 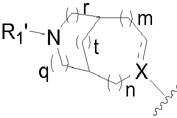 is formula (IV'): 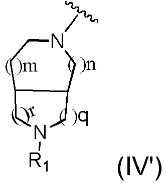 wherein m, n, q, and r are individually and separately selected from an integer of 0-4; R₁' is an amino protection group or hydrogen.

In another embodiment, the group 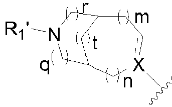 is formula (V'): 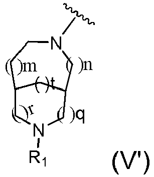 wherein,
m, n, q, and r are independently selected from an integer of 0-4;
t is an integer of 1-3;
R₁' is an amino protection group or hydrogen.

In a preferable embodiment, the compound of the present invention is selected from : 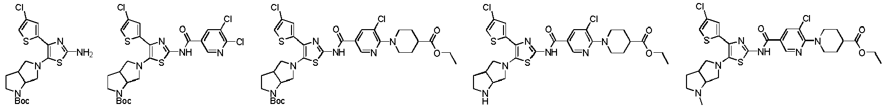 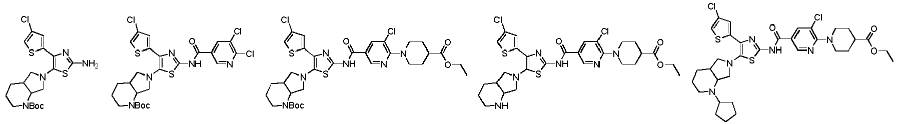 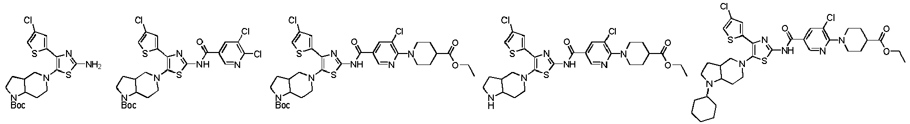 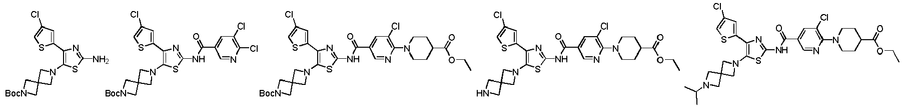 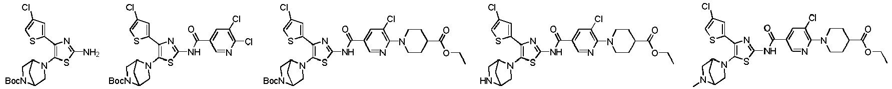 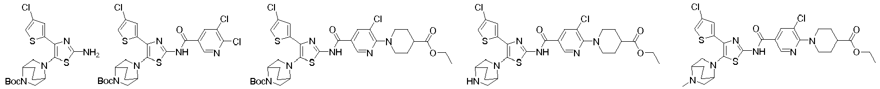 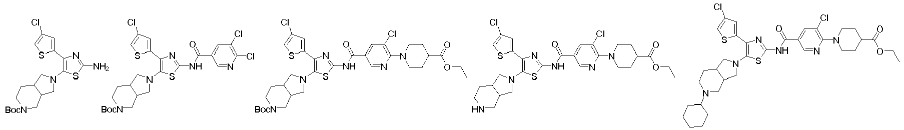 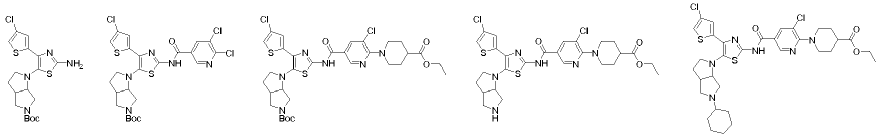 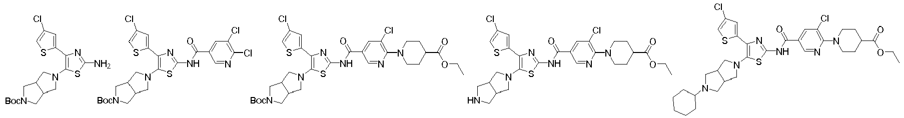 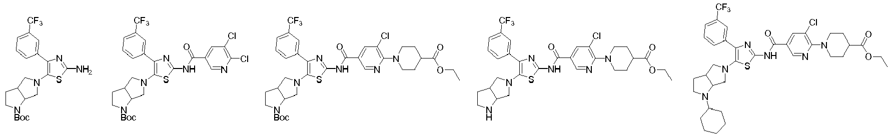 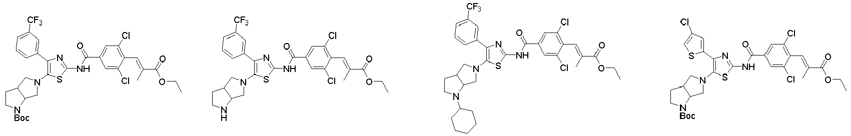 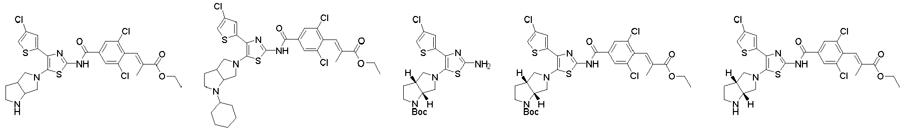 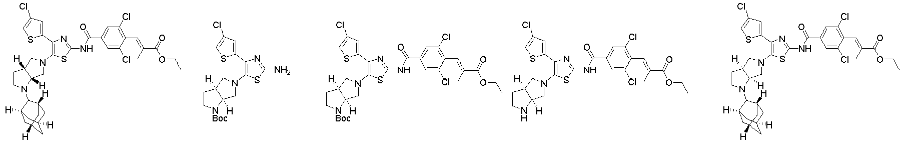 or an isomer or racemate thereof, a salt thereof, an ester thereof, a solvate thereof, a chemically protected form thereof, a prodrug or metabolite thereof, a crystal form thereof, and a mixture thereof.

In another aspect, the present invention provides a preparation method for the compound represented by formula (I), as follows: 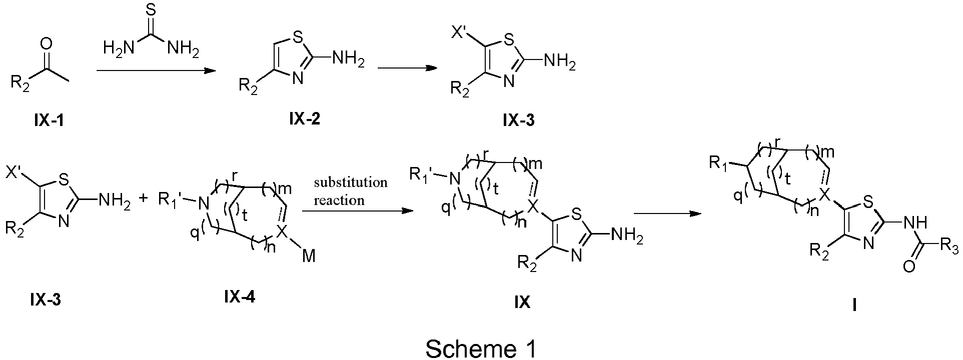 wherein, M is H, Li, Na, K, Si, Mg, Zn, boric acid group or boric acid ester group; R₁, R₂, R₃, m, n, q, r and t are defined as above, X' is halogen, R₁' is an amino protection group(e.g. acyl, Boc, Fmoc, Cbz or the like) or hydrogen.

Compound IX-1 and thiourea are reacted in a suitable solvent, such as ethers, alkanes, haloalkanes, aromatic hydrocarbons, alcohols and water solvent, or a mixture thereof, in presence of a halogenation agent such as iodine, bromine, NBS, NIS, NCS, CBr₄, and dibromohydantoin, to produce 2-aminothiazole IX-2. The above 2-aminothiazole IX-2 is reacted in presence of a halogenation agent such as iodine, bromine, NBS, NIS, NCS, CBr₄, dibromohydantoin, PBr₃, PCl₃, and POCl₃ to produce compound IX-3.

Compound IX-3 and compound IX-4 are subjected to a substitution reaction to produce compound IX. When X is N, the above substitution reaction can be a substitution reaction between compound IX-3 and a secondary amine, which can be carried out in presence or absence of a metal catalyst (preferably in presence of a metal catalyst) to form a C-N bond. Compound IX is subjected to an acylation reaction, a deprotection reaction, a reductive amination reaction, a hydrolysis reaction or a combination thereof to finally produce the desired compound I. R₁, R₂ and R₃ in Compound I can be further chemically modified according to the requirement, and this chemical modification for R₁, R₂ and R₃ can be done with the methods well known in the art, for example, with reference to the textbook such as Handbook of Synthetic Organic Chemistry (2^{nd} edition).

Preferably, the present invention provides a method for preparing the compound represented by formula (I), as follows: 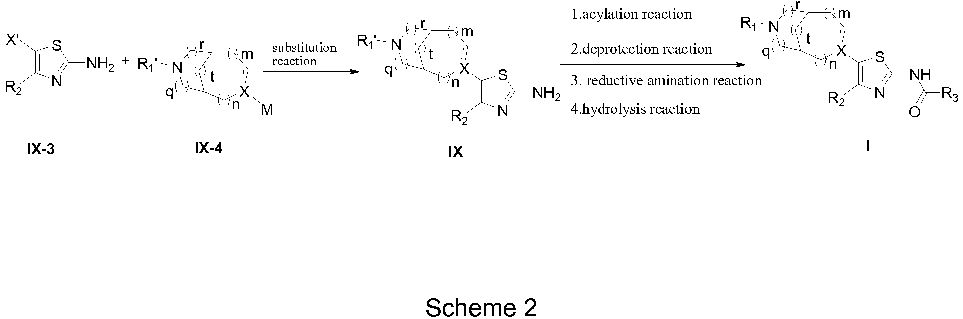 wherein, M is H, Li, Na, K, Si, Mg, Zn, boric acid group or boric acid ester group; R₁, R₂, R₃, m, n, q, r and t are defined as those for the above general formula, X' is halogen, R₁' is an amino protection group (including, for example, tert-butyloxycarbonyl) or hydrogen. Wherein, when R₁' is hydrogen, it is necessary for the formed compound IX to firstly transfer the hydrogen of R₁' to an amino protection group, and then carry out the subsequent acylation reaction, deprotection reaction, reductive amination reaction and hydrolysis reaction.

First, compound IX-3 and compound IX-4 are subjected to a substitution reaction to produce compound IX; the substitution reaction is carried out in presence of a base, said base may be an organic base (e.g. triethylamine, N,N-diisopropylethylamine, DBU, DBN, DABCO, morpholine, N-methylmorpholine, pyridine) or an inorganic base (e.g. sodium hydroxide, potassium hydroxide, calcium hydroxide, barium hydroxide, sodium carbonate, potassium carbonate, magnesium carbonate, sodium bicarbonate, potassium bicarbonate). The substitution reaction can be carried out in an organic solvent, the suitable organic solvent comprises DMSO, NMP, DMF, acetonitrile, alcohols (e.g. methanol, ethanol, isopropanol, n-butyl alcohol, tert-butyl alcohol), halogenated hydrocarbons (e.g. chloroform, dichloromethane, dichloroethane), ketones (e.g. acetone, butanone), ethers (e.g. 1,4-dioxane, tetrahydrofuran, dimethyl ether, ethyl ether, methyl t-butyl ether), esters (e.g. ethyl acetate), hydrocarbons (benzene, toluene, xylene). The reaction temperature for the substitution reaction can be from room tempeature to 150°C, preferably from room tempeature to 100°C, more preferably 50-90°C, most preferably 70-90°C; the reaction time is in a range of 1-24h, preferably 1-16h, more preferably 1-8 hours, most preferably 1 hour. The substitution reaction can be carried out in presence or absence of a metal catalyst, said metal catalyst can be a metal catalyst containing Mg, Fe, Ni, Pd, Ru, Rh or Ti.

Subsequently, the obtained compound IX was successively subjected to an acylation reaction, a deprotection reaction, a reductive amination reaction and a hydrolysis reaction to finally produce the desired compound I; wherein, the acylation reaction is preferably carried out in presence of an activation agent, said activation agent is an agent that can transafer carboxylic acid to more active anhydride, acyl halide, and ester, and the agent is preferably acetic anhydride, oxalyl chloride, NBS, NIS, PCl₃, PBr₃, PPh₃/I₂, 4-nitrophenol, phenol, diphenyl chlorophosphate. Said acylation reaction can be carried out in an organic solvent, and said solvent is defined as above. Said acylation reaction is preferably carried out in presence of a base, and said base is an organic base (e.g. triethylamine, N,N-diisopropylethylamine, DBU, DBN, DABCO, morpholine, N-methylmorpholine, pyridine) or an inorganic base (e.g. sodium hydroxide, potassium hydroxide, calcium hydroxide, barium hydroxide, sodium carbonate, potassium carbonate, magnesium carbonate, sodium bicarbonate, potassium bicarbonate). The reaction temperature of the acylation reaction is usually in the range from room temperature to 100°C, preferably 20-80°C, more preferably 40-60°C, most preferably 50°C; the reaction time is usually 1-10h, preferably 1-5h, more preferably 1-3h, most preferably 3h.

The deprotection reaction is carried out in presence of a deprotection agent at room temperature or under heating. The preferable deprotection agent includes an acidic agent such as trifluoroacetic acid, hydrochloric acid, sulfuric acid or the like, or a basic agent such as sodium hydroxide, potassium hydroxide, lithium hydroxide, piperidine or the like. More detailed operation steps may be found in Greene's Protective Groups in Organic Synthesis (4^{th} Edition) or the like.

The reductive amination reaction is to react the free amino group after deprotection with a ketone or an aldehyde such as paraformaldehyde, acetone, cyclopentanone, cyclobutanone, 3-methylcyclopentanone, cyclohexanone, N-methyl-4-piperidinone, 4-tetrahydropyrone, cycloheptanone, 2-bicyclo[2.2.1]heptanone, adamantanone, 3,3-difluorocyclobutanone or the like in presence of a reducing agent to form a C-N bond, wherein the reducing agent may be sodium borohydride, potassium borohydride, borane, sodium cyanoborohydride, sodium triacetyloxyborohydride. The reductive amination reaction can be carried out in an organic solvent, wherein said organic solvent may comprise acetonitrile, alcohols (e.g. methanol, ethanol, isopropanol, n-butyl alcohol, tert-butyl alcohol), halogenated hydrocarbons (e.g. chloroform, dichloromethane, dichloroethane), ethers (e.g. 1,4-dioxane, tetrahydrofuran, dimethyl ether, ethyl ether, methyl t-butyl ether) or esters (e.g. ethyl acetate). The reductive amination reaction temperature is in a range from room temperature to 100°C, preferably 35-75°C, more preferably 45-65°C, most preferably 50-60°C; the reaction time is usually 1-16h, preferably 1-8h, more preferably 1-5h, most preferably 1-3h.

The hydrolysis reaction is carried out in presence of a base. Said base is an organic base (triethylamine, N,N-diisopropylethylamine, DBU, DBN, DABCO, morpholine, N-methylmorpholine, pyridine) or an inorganic base (e.g. lithium hydroxide, sodium hydroxide, potassium hydroxide, calcium hydroxide, barium hydroxide, sodium carbonate, potassium carbonate, magnesium carbonate, sodium bicarbonate, potassium bicarbonate). The hydrolysis reaction temperature is usually in a range from room temperature to 80°C, preferably from room tempeature to 50°C, more preferably from room tempeature to 30°C; the reaction time is usually 1-24h, preferably 1-12h, more preferably 1-8h, most preferably 1-3h.

Preferably, the present invention provides a method for preparing a compound represented by Formula (I), as follows: 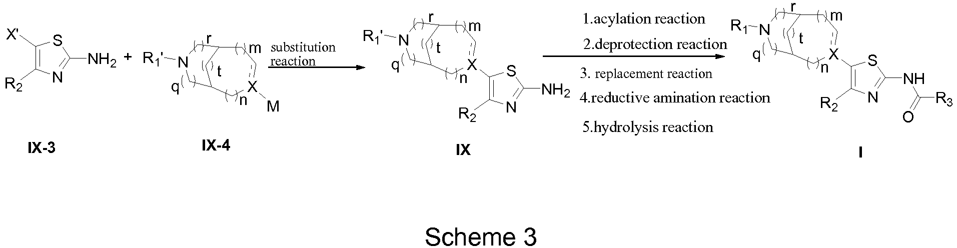 wherein, M is H, Li, Na, K, Si, Mg, Zn, boric acid group or boric acid ester group; R₁, R₂, R₃, m, n, q, r and t are defined as in the above general formula, X' is halogen, R₁' is an amino protection group (e.g. tert-butyloxycarbonyl) or hydrogen. Wherein, when R₁' is hydrogen, it is necessary for the formed compound IX to firstly transfer the hydrogen of R₁' to an amino protection group, and then carry out the subsequent acylation reaction, deprotection reaction, substitution reaction, reductive amination reaction and hydrolysis reaction.

Firstly, compound IX-3 and compound IX-4 are subjected to a substitution reaction to produce compound IX; the formed compound IX is successively subjected to an acylation reaction, a substitution reaction, a deprotection reaction, a reductive amination reaction and a hydrolysis reaction to finally produce the desired compound I; wherein the reaction conditions for the substitution reaction, the acylation reaction, the deprotection reaction, the reductive amination reaction and the hydrolysis reaction are substantially identical to those in Scheme 2.

In another aspect, the present invention provides a pharmaceutical composition in which a compound represented by formula (I) or a pharmaceutically acceptable salt thereof is used as active component. The composition contains the composition of the present invention or an isomer or racemate thereof, a salt thereof, an ester thereof, a solvate thereof, a chemically protected form thereof, a prodrug or metabolite thereof, a crystal form thereof, and a mixture thereof and a pharmaceutically acceptable carrier. The pharmaceutically acceptable carrier can be a solid or a liquid. Wherein, the solid carrier may be one or more materials used as excipients, diluents, sweeting agents, solubilizers, lubricants, binders, tablet disintegrating agents, stabilizers, preservatives or encapsulating materials. The liquid carrier may be solvents or liquid dispersion media. Suitable solid carrier includes but is not limited to, for example, cellulose, glucose, lactose, mannitol, magnesium stearate, magnesium carbonate, sodium carbonate, saccharin sodium, sucrose, dextrin, talc, starch, pectin, gelatin, tragacanth, arabic gum, sodium alginate, parabens, methylcellulose, sodium carboxymethyl cellulose, a low-melting point wax, cocoa butter or the like. Suitable liquid carrier includes but is not limited to water, ethanol, polyhydric alcohol (e.g. glycerol, propylene glycol, liquid polyethylene glycol, etc), a vegetable oil, glyceride and a mixture thereof.

The pharmaceutical composition according to the present invention may be prepared by a known method, including conventional blending, granulating, tableting, coating, dissolving, or lyophilization processes.

The compound or the pharmaceutical composition of the present invention may be administered by any route appropriate to diseases or conditions to be treated. Suitable routes include oral, parenteral (including subcutaneous, intramuscular, intravenous, intraarterial, intradermal, intrathecal and epidural), transdermal, rectal, nasal, topical (including buccal and sublingual), vaginal, intraperitoneal, intrapulmonary and intranasal.

Depending on the administration route, the pharmaceutical composition according to the present invention may be prepared into various dosage forms conventional in the art, such as tablet, capsule, pill, emulsion, injection, pulvis, granule, ointment, patch, powder injection, solution, suspending agent, emulsion, cream, aerosol, drop, lozenge, or the like.

Tablets containing the compound represented by formula (I) of the present invention in admixture with non-toxic pharmaceutically acceptable excipient which are suitable for manufacture of tablets are acceptable. These exemplary excipients may be, for example, inert diluents, such as sodium carbonate, lactose, glucose, cellulose or the like; granulating and disintegrating agents, such as maize starch, glycolate, alginic acid; binders, such as gelatin or ; arabic gum; lubricants, such as silica, magnesium stearate or calcium stearate, stearic acid or talc.

The liquid formulation comprises solutions, suspensions and emulsions, and contains the compound of formula (I) in a mixture of excipients suitable for preparation of aqueous suspensions. The excipient is for example sodium carboxymethylcellulose, methylcellulose, resin, sodium alginate and natural or synthetic gum. According to the requirement, the liquid formulation can also contain suitable coloring agents, flavoring agents, stabilizers, preservatives and thickening agents.

The pharmaceutical formulation is preferably in a unit dosage form, in which the formulation is subdivided into unit dosages containing a suitable amount of active ingredient. The unit dosage form can be packed in a package containing discrete quantities of the formulation, such as packaged tablets, capsules, or powders in vials or ampoules.

The dosage depends on the various factors including age, weight and healthy condition of the patient, and administration route. The precise dosage required is determined based on the attendant physician's judgment. Generally, the dosage of the active compound to be administered may be, for example, about 0.1 to about 100 mg per day, about 0.1 to about 75 mg/day, about 0.1 to about 50 mg/day, or about 5 to about 10 mg/day. The desired dosage depends on the specific compound used, severity of disease, administration route, weight and healthy condition of the patient as well as the attendant physician's judgment.

In another aspect, the present invention also relates to a compound represented by formula (I) or an isomer or racemate thereof, a salt thereof, an ester thereof, a solvate thereof, a chemically protected form thereof, a prodrug or metabolite thereof, a crystal form thereof, and a mixture thereof as a thrombopoietin (TPO) receptor agonist drug for use in treatment and/or prevention of thrombopoietin receptor mediated diseases or conditions. In addition, the present invention provides a method of treatment of a thrombopoietin receptor agonist mediated disease, which comprises administrating the pharmaceutical composition containing the compound of the present invention to a patient. Wherein, said thrombopoietin receptor agonist mediated disease comprises thrombocytopenia, particularly chronic idiopathic thrombocytopenic purpura, the sympotom of which comprises dermatorrhagia, nasal hemorrhage and gingival bleeding, possibly gastrointestinal or intracerebral hemorrhage in a serious patient.

In another aspect, the present invention also relates to use of a compound represented by formula (I) or an isomer or racemate thereof, a salt thereof, an ester thereof, a solvate thereof, a chemically protected form thereof, a prodrug or metabolite thereof, a crystal form thereof, and a mixture thereof in combination with another drug that are compatible to or have no adverse effects on each other, particularly at least one of other thrombopoietin (TPO) receptor agonist drugs, in manufacture of a medicament for prevention and/or treatment of thrombopoietin receptor mediated diseases or conditions. Said other thrombopoietin (TPO) receptor agonist drugs generally refers to a substance having an activation effect on the thrombopoietin (TPO) receptor; said thrombopoietin receptor agonist mediated disease comprises thrombocytopenia, particularly chronic idiopathic thrombocytopenic purpura, the symptom of which comprises dermatorrhagia, nasal hemorrhage and gingival bleeding, possibly gastrointestinal or intracerebral hemorrhage in a serious patient.

Moreover, the present invention relates to use of the compounds involved in all particular or preferable aspects of the present invention or an isomer or racemate thereof, a salt thereof, an ester thereof, a solvate thereof, a chemically protected form thereof, a prodrug or metabolite thereof, a crystal form thereof, and a mixture thereof in combination with another drug that are compatible to or have no adverse effects on each other in manufacture of a medicament for prevention and/or treatment of thrombopoietin receptor mediated diseases or conditions.

The so-called "in combination" comprises the simultaneous, sequential or alternative use, and further comprises preparing a pharmaceutical dosage form or pharmaceutical product correspondingly present in one or more drug units suitably used in combination.

In another aspect, the present invention provides a method for prevention and/or treatment of thrombopoietin receptor mediated diseases or conditions with a compound of formula (I), or an isomer or racemate thereof, a salt thereof, an ester thereof, a solvate thereof, a chemically protected form thereof, a prodrug or metabolite thereof, a crystal form thereof, and a mixture thereof in combination with another drug, particularly in combination with at least one of other thrombopoietin (TPO) receptor agonist drugs.

The compound of the present invention further comprises a compound in which one or more of hydrogen atoms, fluorine atoms, carbon atoms, nitrogen atoms, oxygen atoms, and sulfur atoms are replaced with the corresponding radioactive isotopes or stable isotopes. These labelled compounds can be used in metabolism or pharmacokinetics research, or used as a receptor's ligand in biological assay, or the like.

### Examples

For a reaction for which the specific reaction conditions are not indicated in the example, the reaction is carried out according to the conventional conditions or the conditions recommended by the manufacturer. For a used reagent or a used instrument for which the manufacturer is not indicated, the reagent and the instrument are conventional products which are commercially available.

In the present invention, unless otherwise stated, wherein:
(i) The temperature is expressed in degrees Celsius (°C), and the operation is carried out in a room temperature environment; the room temperature has a meaning well known in the art, specifically is a range of 10-35°C, preferably a range of 15-30°C, most preferably a range of 20-25°C;
(ii) The organic solvent is dried over anhydrous sodium sulfate, and the removal of solvent by evaporation is performed under reduced pressure with a rotary evaporator at a bath temperature of not higher than 60°C;
(iii) The reaction process is traced with a thin layer chromatography (TLC);
(iv) Both of the obtained solid intermediate and the final product are dried in vacuum at a temperature of not higher than 60°C; and
(v) The final product has a clear proton nuclear magnetic resonance spectrum (1H-NMR) and mass spectrometry (MS) data.

The abbreviations used in the present invention have the following meanings:

| Abbreviation | Meaning |
|---|---|
| LC-MS | high performance liquid chromatography-mass spectrum combination |
| Cbz-OSu | N-(benzyloxycarbonyloxy)succinimide |
| -Boc | tert-butyloxycarbonyl |
| -Cbz | Benzyloxycarbonyl |

### Example 1:

### 1-(3-chloro-5-{[4-(4-chlorothiophen-2-yl)-5-(1-methyl-hexahydropyrrolo[3,4-b]pyrrol-5(1 H)-yl)thiazol-2-yl]carbamoyl}pyridin-2-yl)piperidine-4-carboxylic acid (K1)

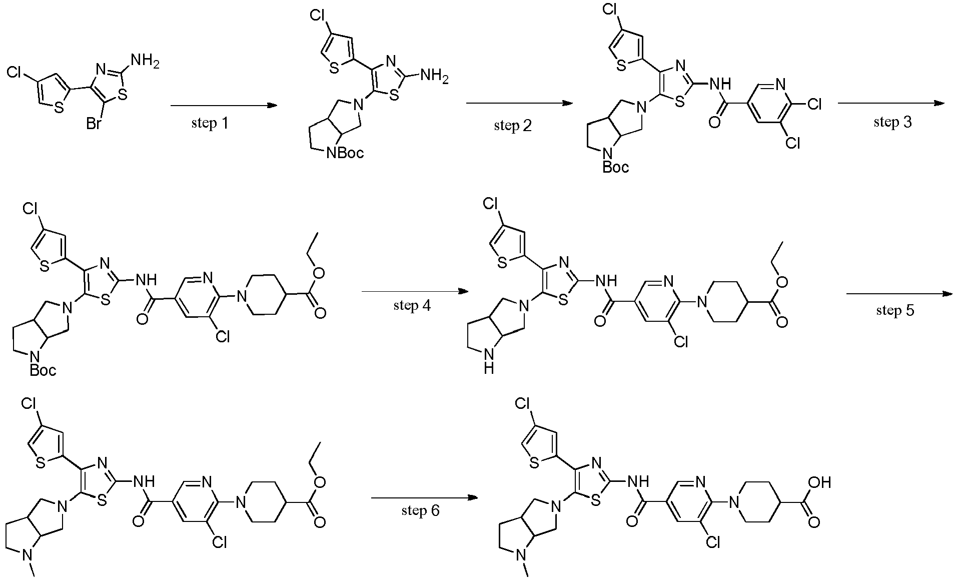

### Step 1: Synthesis of tert-butyl

### 5-[2-amino-4-(4-chlorothiophen-2-yl)-thiazol-5-yl]-hexahydropyrrolo[3,4-b]pyrrole-1(2H )-carboxylate

5-bromo-4-(4-chlorothiophen-2-yl)-2-amino-thiazole (1.4g, 4.7mmol), tert-butyl hexahydropyrrolo[3,4-b]pyrrole-1(*2H*)-carboxylate(1.1g, 4.7mmol) were dissolved in N,N-dimethylformamide (30mL), and anhydrous potassium carbonate (820mg, 5.6mmol) was further added. The mixture was reacted at about 70°C for about 1 hour. The solvent was removed under reduced pressure. The crude product was purified with a silica gel column chromatography to produce the title compound (1.7g).
ESI-MS (m/z): 427.2 [M + H]⁺

### Step 2: Synthesis of tert-butyl

### 5-[4-(4-chlorothiophen-2-yl)-2-(5,6-dichloro-pyridin-3-carbonylamino)-thiazol-5-yl]-hexa hydropyrrolo[3,4-b]pyrrole-1(2H)-carboxylate

The product from Step 1 (1.5g, 3.6mmol), and 5,6-dichloro-pyridin-3-carboxylic acid (1.1g, 5.4mmol) were dissolved in chloroform (30mL), and then diphenyl chloridophosphate (1.4g, 5.4mmol), and N,N-diisopropylethylamine (690mg, 5.4mmol) were successively added. The mixture was reacted at 50°C for about 1.5 hours. The reaction system was cooled down to room temperature, and then was washed with water, an aqueous sodium bicarbonate solution, and a saturated aqueous sodium chloride solution, dried over anhydrous sodium sulfate, and filtered. The filtrate was evaporated under reduced pressure to remove the solvent to produce the title compound, which was directly used in the next step reaction without purification.
ESI-MS (m/z): 600.1[M + H]⁺

### Step 3: Synthesis of tert-butyl

### 5-[2-{5-chloro-6-[4-(ethoxycarbonyl)-piperidin-1-yl]-nicotinamido}-4-(4-chlorothiophen-2-yl)-thiazol-5-yl]-hexahydropyrrolo[3,4-b]pyrrole-1(2H)-carboxylate

The product from Step 2 (1.6g, 2.6mmol), and ethyl 4-piperidine carboxylate (820mg, 5.2mmol) were dissolved in tetrahydrofuran (25mL), and then N,N-diisopropylethylamine (530mg, 5.2mmol) was added. The mixture was heated under reflux and reacted for 18h. The solvent was removed under reduced pressure. The crude product was purified with a silica gel column chromatography to produce the title compound (1.7g).
ESI-MS (m/z): 721.2 [M + H]⁺

### Step 4: Synthesis of ethyl

### 1-(3-chloro-5-{[4-(4-chlorothiophen-2-yl)-5-(hexahydropyrrolo[3,4-b]pyrrol-5(1H)-yl)-thi azol-2-yl]-carbamoyl}-pyridin-2-yl)-piperidine-4-carboxylate

The product from Step 3 (72mg, 0.1mmol) was dissolved in dichloromethane (1.5mL), and thentrifluoroacetic acid (0.5mL) was added dropwisely. The mixture was reacted at room temperature for about 1.5h. The solvent was removed by evaporation under reduced pressure to produce the title compound, which was directly used in the next step reaction without purification.
ESI-MS (m/z): 621.2 [M + H]⁺

### Step 5: Synthesis of ethyl

### 1-(3-chloro-5-{[4-(4-chlorothiophen-2-yl)-5-(1-methyl-hexahydropyrrolo[3,4-b]pyrrol-5(1 H)-yl)-thiazol-2-yl]-carbamoyl}-pyridin-2-yl)-piperidine-4-carboxylate

The product from Step 4 was dissolved in 1,4-dioxane (5mL), and then glacialglacial acetic acid (0.1 mL), paraformaldehyde (15mg, 0.5mmol), and sodium cyanoborohydride (32mg, 0.5mmol) were added. The mixture was reacted at 50°C for about 2 h. The reaction mixture was filtered, and the filtrate was evaporated under reduced pressure to remove the solvent to produce the title compound, which was directly used in the next step reaction without purification.
ESI-MS (m/z): 635.2 [M + H]⁺

### Step 6: Synthesis of

### 1-(3-chloro-5-{[4-(4-chlorothiophen-2-yl)-5-(1-methylhexahydropyrrolo[3,4-b]pyrrol-5(1 H)-yl)-thiazol-2-yl]-carbamoyl}-pyridin-2-yl)-piperidine-4-carboxylic acid

The product from Step 5 was dissolved in a mixed solvent of tetrahydrofuran and water (3mL, V:V=2:1), and then lithium hydroxide monohydrate (25mg, 0.6mmol) was added. The mixture was reacted at room temperature for about 3.5h. The reaction mixture was adjusted with a saturated citric acid solution to the acidity (pH=3), extracted with a mixed solvent of ethyl acetate and tetrahydrofuran (V:V=1:1), the organic phases were combined and washed successively with water and a saturated aqueous sodium chloride solution, dried over anhydrous sodium sulfate, and concentrated to produce a crude title compound. The crude title compound was purified with a high performance liquid chromatography using a trifluoroacetic acid system to produce a trifluoroacetate salt of the title compound (32mg).
¹H NMR (400 MHz, DMSO-d₆) δ 12.66 (s, 1H), 12.31 (s, 1H), 9.82 (s, 1H), 8.84 (d, J = 2.1 Hz, 1H), 8.40 (d, J = 2.1 Hz, 1H), 7.57 (d, J = 1.4 Hz, 1H), 7.55 (d, J = 1.4 Hz, 1H), 4.16-4.10 (m, 1H), 4.00 (s, 1H), 3.96 (s, 1H), 3.66 (s, 1H), 3.61 (d, J = 11.4 Hz, 1H), 3.30-3.16 (m, 4H), 3.03 (t, J = 11.4 Hz, 2H), 2.94 (d, J = 4.2 Hz, 3H), 2.84 (dd, J = 9.4 Hz, 7.2 Hz, 1H), 2.57-2.54 (m, 1H), 2.45 (s, 1H), 1.94 (d, J = 10.1 Hz, 2H), 1.88-1.82 (m, 2H), 1.73-1.63 (m, 2H)
ESI-MS (m/z): 607.2 [M + H]⁺

### Example 2:

### 1-(3-chloro-5-((4-(4-chlorothiophen-2-yl)-5-(1-cyclobutyl-hexahydropyrrolo[3,4-b]-pyrrol -5(1H)-yl)-thiazol-2-yl)-carbamoyl)-pyridin-2-yl)-piperidine-4-carboxylic acid (K2)

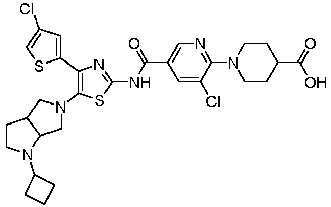

The procedure similar to that in Example 1, except that paraformaldehyde was replaced with cyclobutanone in Step 5 of Example 1, was used to produce a trifluoroacetate salt of the title compound (28mg).
¹H NMR (400 MHz, DMSO-d₆) δ 12.66 (s, 1H), 12.32 (s, 1H), 9.92 (s, 1H), 8.83 (d, J = 2.0hz, 1H), 8.39 (d, J = 2.0hz, 1H), 7.58 (d, J = 1.4 Hz, 1H), 7.50 (d, J = 1.4 Hz, 1H), 4.14 (s, 1H), 3.99 (s, 1H), 3.96 (s, 1H), 3.94-3.92 (m, 1H), 3.63 (s, 1H), 3.53-3.50 (m, 1H), 3.34-3.31 (m, 1H), 3.22-3.14 (m, 3H), 3.03 (t, J = 2.4 Hz, 2H), 2.91 (t, J = 8.5 Hz, 1H), 2.52 (s, 1H), 2.39 (s, 1H), 2.27-2.22 (m, 4H), 1.96-1.92 (m, 3H), 1.78-1.63 (m, 4H) ESI-MS (m/z): 647.2 [M + H]⁺

### Example 3:

### 1-(3-chloro-5-((4-(4-chlorothiophen-2-yl)-5-(1-cyclopentyl-hexahydropyrrolo[3,4-b]pyrro l-5(1H)-yl)-thiazol-2-yl)-carbamoyl)-pyridin-2-yl)-piperidine-4-carboxylic acid (K3)

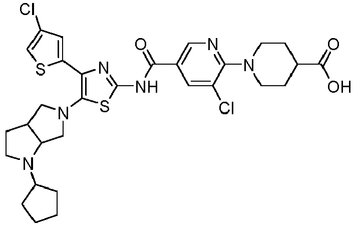

The procedure similar to that in Example 1, except that paraformaldehyde was replaced with cyclopentanone in Step 5 of Example 1, was used to produce a trifluoroacetate salt of the title compound (27mg).
¹H NMR (400 MHz, DMSO-d₆) δ 12.67 (s, 1H), 12.33 (s, 1H), 9.82 (s, 1H), 8.83 (d, J = 2.1 Hz, 1H), 8.39 (d, J = 2.1 Hz, 1H), 7.58 (d, J = 1.4 Hz, 1H), 7.51 (d, J = 1.4 Hz, 1H), 4.33-4.28 (m, 1H), 3.99 (s, 1H), 3.96 (s, 1H), 3.77-3.72 (m, 1H), 3.67-3.61 (m, 1H), 3.56 (d, J = 10.5 Hz, 1H), 3.41-3.37 (m, 1H), 3.29-3.20 (m, 2H), 3.18-3.14 (m, 1H), 3.03 (t, J = 11.5 Hz, 2H), 2.87 (dd, J = 9.6 Hz, 6.8 Hz, 1H), 2.57-2.52 (m, 1H), 2.42-2.33 (m, 1H), 2.07-2.00 (m, 2H), 1.96-1.92 (m, 2H), 1.90-1.85 (m, 1H), 1.75-1.66 (m, 6H), 1.63-1.50 (m, 2H)
ESI-MS (m/z): 661.2 [M + H]⁺

### Example 4:

### 1-[3-chloro-5-((4-(4-chlorothiophen-2-yl)-5-(1-(3-methylcyclopentyl)-hexahydropyrrolo[ 3,4-b]pyrrol-5(1H)-yl)-thiazol-2-yl)-carbamoyl)-pyridin-2-yl)-piperidine-4-carboxylic acid (K4)

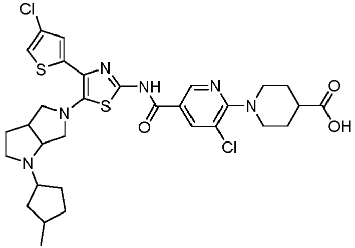

The procedure similar to that in Example 1, except that paraformaldehyde was replaced with 3-methylcyclopentanone in Step 5 of Example 1, and the purification using the trifluoroacetic acid system with the high performance liquid chromatography was replaced with the purification using a hydrochloric acid system with a high performance liquid chromatography in Step 6 of Example 1, was used to produce a hydrochloride salt of the title compound (about 24mg).
¹H NMR (400 MHz, DMSO-d₆) δ 12.64 (s, 1H), 10.41-9.87 (m, 1H), 8.83 (d, J = 2.0hz, 1H), 8.39 (d, J = 2.0hz, 1H), 7.57-7.46 (m, 2H), 4.53-4.29 (m, 1H), 4.00-3.96 (m, 2H), 3.86-3.61 (m, 3H), 3.42-3.34 (m, 2H), 3.30-3.19 (m, 2H), 3.15-3.01 (m, 4H), 2.54-2.51 (m, 1H), 2.41-2.22 (m, 2H), 2.06-1.88 (m, 4H), 1.76-1.67 (m, 2H), 1.54-1.12 (m, 6H)
ESI-MS (m/z): 675.2 [M + H]⁺

### Example 5:

### 1-(3-chloro-5-((4-(4-chlorothiophen-2-yl)-5-(1-cyclohexyl-hexahydropyrrolo[3,4-b]pyrrol -5(1H)-yl)-thiazol-2-yl)-carbamoyl)-pyridin-2-yl)-piperidine-4-carboxylic acid (K5) and isomers thereof (K5-a, K5-b)

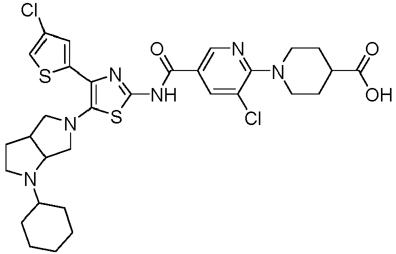

The procedure similar to that in Example 1, except that paraformaldehyde was replaced with cyclohexanone in Step 5 of Example 1, was used to produce a trifluoroacetate salt of the title compound (K5) (about 29mg).
¹H NMR (400 MHz, DMSO-d₆) δ 12.67 (s, 1H), 12.28 (s, 1H), 9.54 (s, 1H), 8.83 (d, J = 2.2 Hz, 1H), 8.39 (d, J = 2.2 Hz, 1H), 7.58 (s, 1H), 7.53 (s, 1H), 4.35 (d, J = 8.1 Hz, 1H), 3.99 (s, 1H), 3.96 (s, 1H), 3.77-3.72 (m, 1H), 3.45-3.39 (m, 1H), 3.18-3.15 (m, 4H), 3.03 (t, J = 12.3 Hz, 2H), 2.84 (dd, J = 10.2 Hz, 6.9 Hz, 1H), 2.56-2.53 (m, 1H), 2.39-2.33 (m, 1H), 2.09 (d, J = 11.2 Hz, 1H), 2.00-1.92 (m, 3H), 1.85-1.80 (m, 2H), 1.72-1.62 (m, 3H), 1.41-1.30 (m, 3H), 1.14-1.08 (m, 1H)
ESI-MS (m/z): 675.2 [M + H]⁺
ethyl 1-(3-chloro-5-((4-(4-chlorothiophen-2-yl)-5-(1-cyclohexyl-hexahydropyrrolo[3,4-b]pyrrol -5(*1H*)-yl)-thiazol-2-yl)-carbamoyl)-pyridin-2-yl)-piperidine-4-carboxylate (790mg, 1.1mmol), the intermediate obtained in the preparation of the title compound (K5) was purified by a chiral HPLC separation to produce an intermediate isomer compound a' (346mg) and an intermediate isomer compound b' (352mg).

The intermediate isomer compound a' (346mg, 0.5mmol) was dissolved in a mixed solvent of tetrahydrofuran and water (15mL, V:V=2:1), and lithium hydroxide monohydrate (130mg, 3.0mmol) was added. The mixture was reacted at room temperature for 3.5h. The reaction mixture was adjusted with 2N hydrochloric acid to acidity (pH=3). The solvent was removed under a reduced pressure, and the residue was purified with a high performance liquid chromatography using a hydrochloric acid system to produce a hydrochloride salt of the target product isomer (K5-a) (about 235mg).
¹H NMR (400 MHz, DMSO-d₆) δ 12.63 (s, 1H), 11.26 (s, 1H), 10.47 (s, 1H), 8.83 (d, J = 2.2 Hz, 1H), 8.39 (d, J = 2.2 Hz, 1H), 7.59 (d, J = 1.5 Hz, 1H), 7.55 (d, J = 1.5 Hz, 1H), 4.35 (d, J = 7.1 Hz, 1H), 3.99 (s, 1H), 3.96(s, 1H), 3.74-3.71 (m, 2H), 3.43-3.39 (m, 1H), 3.22-3.13 (m, 4H), 3.03 (t, J = 12.3 Hz, 2H), 2.94-2.90 (m, 1H), 2.56-2.53 (m, 1H), 2.36-2.30 (m, 1H), 2.09 (s, 1H), 1.96-1.93 (m, 3H), 1.83-1.80 (m, 2H), 1.72-1.61 (m, 3H), 1.57-1.43 (m, 2H), 1.26-1.19 (m, 2H), 1.14-1.06 (m, 1H)
ESI-MS (m/z): 675.2 [M + H]⁺

The intermediate isomer compound b' (352mg, 0.5mmol) was dissolved in a mixed solvent of tetrahydrofuran and water (15mL, V:V=2:1), and lithium hydroxide monohydrate (130mg, 3.0mmol) was added. The mixture was reacted at room temperature for 3.5h. The reaction mixture was adjusted with 2N hydrochloric acid to acidity (pH=3). The solvent was removed under a reduced pressure, and the residue was purified with a high performance liquid chromatography using a hydrochloric acid system to produce a hydrochloride salt of the target product isomer (K5-b) (230mg).
¹H NMR (400 MHz, DMSO-d₆) δ 12.63 (s, 1H), 12.28 (s, 1H), 10.35 (s, 1H), 8.83 (d, J = 2.2 Hz, 1H), 8.39 (d, J = 2.2 Hz, 1H), 7.58-7.56 (m, 2H), 4.35 (d, J = 8.1 Hz, 1H), 3.99 (s, 1H), 3.96 (s, 1H), 3.73-3.72 (m, 2H), 3.43-3.22 (m, 2H), 3.18-3.13 (m, 3H), 3.03 (t, J = 12.3 Hz, 2H), 2.92-2.88 (m, 1H), 2.57-2.52 (m, 1H), 2.34-2.32 (m, 1H), 2.09 (s, 2H), 1.97-1.92 (m, 3H), 1.83-1.80 (d, J = 12.4 Hz, 2H), 1.72-1.60 (m, 3H), 1.55-1.42 (m, 2H), 1.26-1.23(m, 2H), 1.15-1.06 (m, 1H)
ESI-MS (m/z): 675.2 [M + H]⁺

### Example 6:

### 1-[3-chloro-5-((4-(4-chlorothiophen-2-yl)-5-(1-(N-methyl-piperidin-4-yl)-hexahydropyrrolo[3,4-b]pyrrol-5(1H)-yl)-thiazol-2-yl)-carbamoyl)-pyridin-2-yl]-piperidine-4-carboxylic acid (K6)

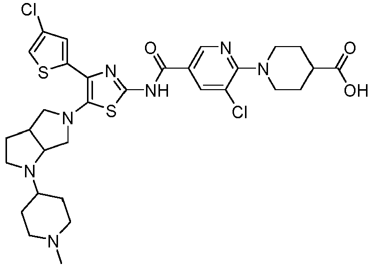

The procedure similar to that in Example 1, except that paraformaldehyde was replaced with N-methyl-4-piperidinone in Step 5 of Example 1, and the purification using the trifluoroacetic acid system with the high performance liquid chromatography was replaced with the purification using a hydrochloric acid system with a high performance liquid chromatography in Step 6 of Example 1, was used to produce a hydrochloride salt of the title compound (about 20mg).
¹H NMR (400 MHz, DMSO-d₆) δ 12.65 (s, 1H), 11.08 (s, 1H), 10.51 (s, 1H), 8.84 (d, J = 1.2 Hz, 1H), 8.39 (d, J = 1.2 Hz, 1H), 7.59-7.56 (m, 2H), 4.38 (s, 1H), 3.98 (d, J = 12.8 Hz, 2H), 3.80 (s, 1H), 3.68-3.53 (m, 3H), 3.25-3.19 (m, 2H), 3.15-2.91 (m, 8H), 2.74 (d, J = 4.0hz, 3H), 2.40-1.93 (m, 8H), 1.73-1.64 (m, 2H)
ESI-MS (m/z): 690.2 [M + H]⁺

### Example 7:

### 1-[3-chloro-5((4-(4-chlorothiophen-2-yl)-5-(1-(4H-tetrahydropyran-4-yl)-hexahydropyrrolo[3,4-b]pyrrol-5(1H)-yl)-thiazol-2-yl)-carbamoyl)-pyridin-2-yl]-piperidine-4-carboxylic acid (K7)

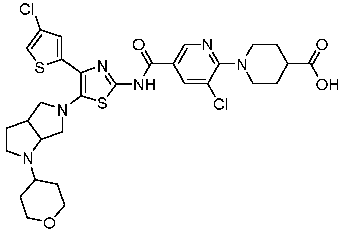

The procedure similar to that in Example 1, except that paraformaldehyde was replaced with 4-tetrahydropyrone in Step 5 of Example 1, and the purification using the trifluoroacetic acid system with the high performance liquid chromatography in Step 6 of Example 1 was replaced with the purification using a hydrochloric acid system with a high performance liquid chromatography, was used to produce a hydrochloride salt of the title compound (22mg).
¹H NMR (400 MHz, DMSO-d₆) δ 12.64 (s, 1H), 10.69 (s, 1H), 9.92 (s, 1H), 8.83 (d, J = 2.0hz, 1H), 8.39 (d, J = 2.0hz, 1H), 7.59 (d, J = 1.2 Hz, 1H), 7.60 (d, J = 1.6 Hz, 1H), 4.38-4.35 (m, 1H), 4.00-3.96 (m, 4H), 3.76-3.60 (m, 3H), 3.41-2.90 (m, 9H), 2.57-2.51 (m, 1H), 2.39-2.33 (m, 1H), 2.04-1.64 (m, 9H)
ESI-MS (m/z): 677.2 [M + H]⁺

### Example 8:

### 1-(3-chloro-5-((4-(4-chlorothiophen-2-yl)-5-(1-cycloheptyl-hexahydropyrrolo[3,4-b]pyrrol-5(1H)-yl)-thiazol-2-yl)-carbamoyl)-pyridin-2-yl)-piperidine-4-carboxylic acid (K8)

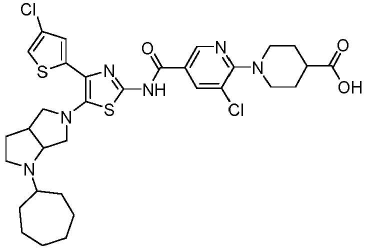

The procedure similar to that in Example 1, except that paraformaldehyde was replaced with cycloheptanone in Step 5 of Example 1, and the purification using the trifluoroacetic acid system with the high performance liquid chromatography in Step 6 of Example 1 was replaced with the purification using a hydrochloric acid system with a high performance liquid chromatography, was used to produce a hydrochloride salt of the title compound (24mg).
¹H NMR (400 MHz, DMSO-d₆) δ 12.63 (s, 1H), 10.37 (s, 1H), 9.92 (s, 1H), 8.34 (d, J = 2.4 Hz, 1H), 8.39 (d, J = 2.0hz, 1H), 7.57-7.55 (m, 2H), 4.37-4.31 (m, 1H), 4.00-3.93 (m, 2H), 3.57-3.53 (m, 1H), 3.45-3.38 (m, 2H), 3.25-2.92 (m, 7H), 2.56-2.51 (m, 1H), 2.34-2.27 (m, 1H), 2.11-2.08 (m, 2H), 1.96-1.93 (m, 3H), 1.75-1.63 (m, 6H), 1.58-1.42 (m, 6H)
ESI-MS (m/z): 689.2 [M + H]⁺

### Example 9:

### 1-[3-chloro-5-((4-(4-chlorothiophen-2-yl)-5-(1-(bicyclo[2.2.1]hept-2-yl)-hexahydropyrrolo[3,4-b]pyrrol-5(1H)-yl)thiazol-2-yl)carbamoyl)pyridin-2-yl]piperidine-4-carboxylic acid (K9)

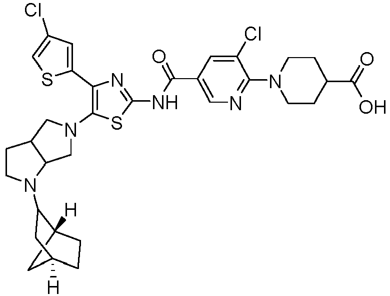

The procedure similar to that in Example 1, except that paraformaldehyde was replaced with 2-bicyclo[2.2.1]heptanone in Step 5 of Example 1, and the purification using the trifluoroacetic acid system with the high performance liquid chromatography in Step 6 of Example 1 was replaced with the purification using a hydrochloric acid system with a high performance liquid chromatography, was used to produce a hydrochloride salt of the title compound (23mg).
¹H NMR (400 MHz, DMSO-d₆) δ 12.64 (s, 1H), 10.58 (s, 1H), 8.83 (d, J = 1.6 Hz, 1H), 8.40 (d, J = 2.0hz, 1H), 7.57-7.56 (m, 2H), 4.28 (s, 1H), 4.00-3.93 (m, 2H), 3.72-3.61 (m, 3H), 3.41-2.92 (m, 7H), 2.56-2.51 (m, 1H), 2.39-2.31 (m, 1H), 2.21-1.33 (m, 12H), 1.04-0.92 (m, 3H)
ESI-MS (m/z): 687.2 [M + H]⁺

### Example 10:

### 1-(3-chloro-5-((4-(4-chlorothiophen-2-yl)-5-(1-(1R,3R,5R,7R)-adamantanyl-hexahydropyrrolo[3,4-b]pyrrol-5(1H)-yl)-thiazol-2-yl)carbamoyl)-pyridin-2-yl)-piperidine-4-carboxylic acid (K10)

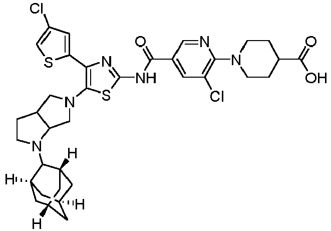

The procedure similar to that in Example 1, except that paraformaldehyde was replaced with adamantanone in Step 5 of Example 1, and the purification using the trifluoroacetic acid system with the high performance liquid chromatography in Step 6 of Example 1 was replaced with the purification using a hydrochloric acid system with a high performance liquid chromatography, was used to produce a hydrochloride salt of the title compound (30mg).
¹H NMR (400 MHz, DMSO-d₆) δ 12.64 (s, 1H), 9.55-9.38 (m, 1H), 8.84-8.83 (m, 1H), 8.39 (d, J = 2.0hz, 1H), 7.57-7.54 (m, 2H), 4.28 (s, 1H), 4.00-3.96 (m, 2H), 3.84-3.80 (m, 1H), 3.44-3.40 (m, 2H), 3.31-3.01 (m, 7H), 2.56-2.51 (m, 1H), 2.33-2.09 (m, 5H), 1.96-1.56 (m, 15H)
ESI-MS (m/z): 727.2 [M + H]⁺

### Example 11:

### 1-(3-chloro-5-((4-(4-chlorothiophen-2-yl)-5-(1-cyclopentyl-octahydropyrrolo[3,4-b]pyridi n-6(1H)-yl)-thiazol-2-yl)-carbamoyl)-pyridin-2-yl)-piperidine-4-carboxylic acid (K11)

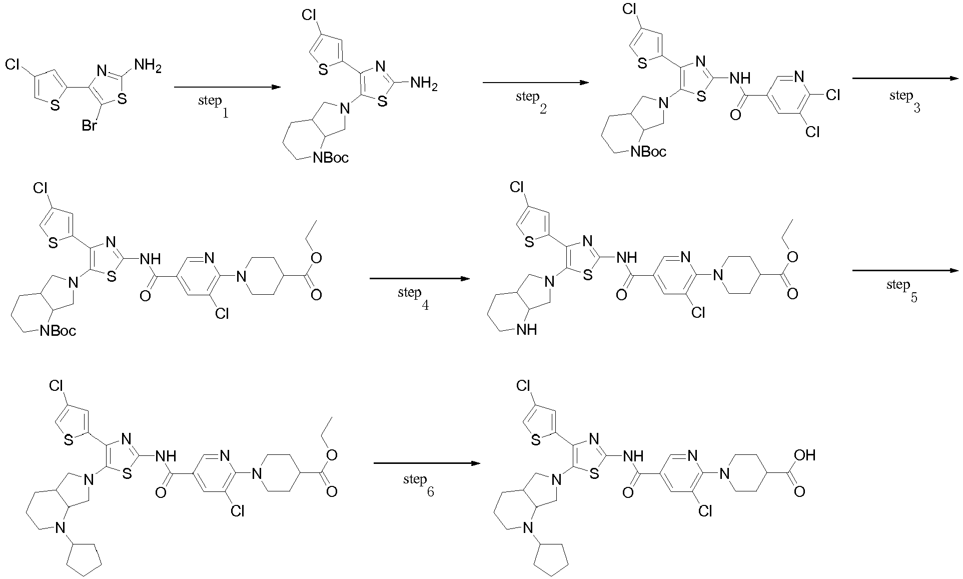

### Step 1: Synthesis of tert-butyl

### 6-(2-amino-4-(4-chlorothiophen-2-yl)thiazol-5-yl)octahydro-1H-pyrrolo[3,4-b]pyridine carboxylate

5-bromo-4-(4-chlorothiophen-2-yl)-2-amino-thiazole (700mg, 2.4mmol), and tert-butyl octahydro-1H-pyrrolo[3,4-b]pyridine carboxylate (557mg, 2.4mmol) were dissolved in N,N-dimethylformamide (15mL). Anhydrous potassium carbonate (410mg, 2.8mmol) was added. The mixture was reacted at 70°C for 1h. The solvent was removed under a reduced pressure. The crude product was purified with a silica gel column chromatography to produce the title compound (819mg).
ESI-MS (m/z): 441.2 [M + H]⁺

### Step 2: Synthesis of

### tert-butyl 6-(4-(4-chlorothiophen-2-yl)-2-(5,6-dichloronicotinamido)thiazol-5-yl)octahydro-1H-pyrr olo[3,4-b]pyridine-1-carboxylate

The product from Step 1 (750mg, 1.8mmol), and 5,6-dichloropyridin-3-carboxylic acid (550mg, 2.7mmol) were dissolved in chloroform (30mL), and then diphenyl chloridophosphate (725mg, 2.7mmol), and N,N-diisopropylethylamine (343mg, 2.7mmol) were successively added. The mixture was reacted at 50°C for 1.5h. The reaction system was cooled to room temperature, and successively washed with water, aqueous sodium bicarbonate solution, and saturated aqueous sodium chloride solution, dried over anhydrous sodium sulfate, and filtered. The solvent was removed by evaporation under reduced pressure to produce the title compound. The product was directly used in the next step without purification.
ESI-MS (m/z): 614.2 [M + H]⁺

### Step 3: Synthesis of

### tert-butyl 6-(2-(5-chloro-6-(4-(ethoxycarbonyl)piperidin-1-yl)-nicotinamido)-4-(4-chlorothiophen-2 -yl)-thiazol-5-yl)-octahydro-1H-pyrrolo[3,4-b]pyridin-carboxylate

The product from Step 2 (800mg, 1.3mmol), and ethyl 4-piperidine carboxylate (410mg, 2.6mmol) were dissolved in dry tetrahydrofuran (25mL), and N,N-diisopropylethylamine (265mg, 2.6mmol) was added. The mixture was heated under flux overnight. The solvent was removed under a reduced pressure. The crude product was purified with a silica gel column chromatography to produce the title compound (850mg).
ESI-MS (m/z): 735.2 [M + H]⁺

### Step 4: Synthesis of ethyl

### 1-(3-chloro-5-((4-(4-chlorothiophen-2-yl)-5-(octahydropyrrolo[3,4-b]pyridin-6(1H)-yl)-thi azol-2-yl)-carbamoyl)pyridin-2-yl)-piperidine-4-carboxylate

The product from Step 3 (73mg, 0.1mmol) was dissolved in dry dichloromethane (1.5mL), and then was added dropwisely trifluoroacetic acid (0.5mL). The mixture was reacted at room temperature for 1.5h. The solvent was removed by evaporation under reduced pressure to produce the title compound. The product was directly used in the next step without purification.
ESI-MS (m/z): 635.2 [M + H]⁺

### Step 5: Synthesis of ethyl

1-(3-chloro-5-((4-(4-chlorothiophen-2-yl)-5-(1-cyclopentyl-octahydropyrrolo[3,4-b]pyridi n-6(*1H*)-yl)-thiazol-2-yl)-carbamoyl)-pyridin-2-yl)-piperidine-4-carboxylate The product from Step 4 (63mg, 0.1mmol) was dissolved in 1,4-dioxane (5mL), and glacial acetic acid (0.1mL), cyclopentanone (43mg, 0.5mmol), and sodium cyanoborohydride (32mg, 0.5mmol) were added. The mixture was reacted at 50°C for 2 h, and then filtered. The filtrate was evaporated under reduced pressure to remove the solvent to produce the title compound, which was directly used in the next step without purification.
ESI-MS (m/z): 703.2 [M + H]⁺

### Step 6: Synthesis of

### 1-(3-chloro-5-((4-(4-chlorothiophen-2-yl)-5-(1-cyclopentyloctahydropyrrolo[3,4-b]pyridi n-6(1H)-yl)thiazol-2-yl)carbamoyl)pyridin-2-yl)piperidine-4-carboxylic acid

The product from Step 5 was dissolved in a mixed solvent of tetrahydrofuran and water (3mL, V:V=2:1), and lithium hydroxide monohydrate (25mg, 0.6mmol) was added. The mixture was reacted at room temperature for 3.5h. The reaction mixture was adjusted with a saturated citric acid solution to the acidity (pH=3), and extracted with a mixed solvent of ethyl acetate and tetrahydrofuran (V:V=1:1, 20mLx3). The organic phases were combined and washed successively with water, saturated aqueous sodium chloride solution, dried over anhydrous sodium sulfate, and filtered. The filtrate was concentrated to produce a crude title compound. The crude title compoundwas purified with a high performance liquid chromatography using a trifluoroacetic acid system to produce a trifluoroacetate salt of the title compound (28mg).
¹H NMR (400 MHz, DMSO-d₆) δ 12.60 (s, 1H), 12.36 (s, 1H), 9.92 (s, 1H), 9.46(s, 1H), 8.84 (d, J = 2.1 Hz, 1H), 8.39 (d, J = 2.1 Hz, 1H), 7.55 (d, J = 1.4 Hz, 1H), 7.31 (d, J = 1.4 Hz, 1H), 4.46-4.40 (m, 1H), 3.99 (s, 1H), 3.96 (s, 1H), 3.82 (t, J = 10.2 Hz, 1H), 3.61-3.48 (m, 1H), 3.44-3.34 (m, 2H), 3.28 (t, J = 9.1 Hz, 1H), 3.19-3.12 (m, 1H), 3.05-2.98 (m, 3H), 2.57-2.51 (m, 1H), 2.12-2.02 (m, 2H), 1.96-1.92 (m, 2H), 1.85-1.54 (m, 12H)
ESI-MS (m/z): 675.2 [M + H]⁺

### Example 12:

### 1-(3-chloro-5-((4-(4-chlorothiophen-2-yl)-5-(1-cyclohexyl-octahydropyrrolo[3,4-b]pyridi n-6(1H)-yl)-thiazol-2-yl)-carbamoyl)-pyridin-2-yl)-piperidine-4-carboxylic acid (K12)

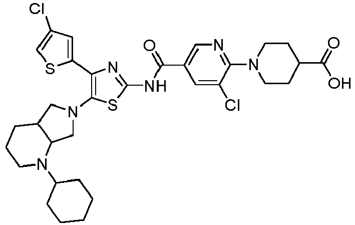

The procedure similar to that in Example 11, except that, cyclopentanone was replaced with cyclohexanone in Step 5 of Example 11, was used to produce a trifluoroacetate salt of the title compound (about 23mg).
¹H NMR (400 MHz, DMSO-d₆) δ 12.59 (s, 1H), 12.30 (s, 1H), 9.77 (s, 1H), 8.84 (d, J = 2.1 Hz, 1H), 8.38 (d, J = 2.1 Hz, 1H), 7.54 (d, J = 1.4 Hz, 1H), 7.34 (d, J = 1.4 Hz, 1H), 4.34 (s, 1H), 3.99 (s, 1H), 3.96 (s, 1H), 3.79-3.69 (m, 1H), 3.49-3.34 (m, 2H), 3.30-3.20 (m, 2H), 3.06-2.96 (m, 4H), 2.57-2.52 (m, 1H), 2.23-2.03 (m, 2H), 1.95-1.92 (m, 3H), 1.83-1.80 (m, 4H), 1.71-1.58 (m, 4H), 1.35-1.09 (m, 6H)
ESI-MS (m/z): 689.2 [M + H]⁺

### Example 13:

### 1-(3-chloro-5-((4-(4-chlorothiophen-2-yl)-5-(1-cyclohexyl-octahydro-1H-pyrrolo[3,2-c]p yridin-5(6H)-yl)-thiazol-2-yl)-carbamoyl)-pyridin-2-yl)-piperidine-4-carboxylic acid (K13)

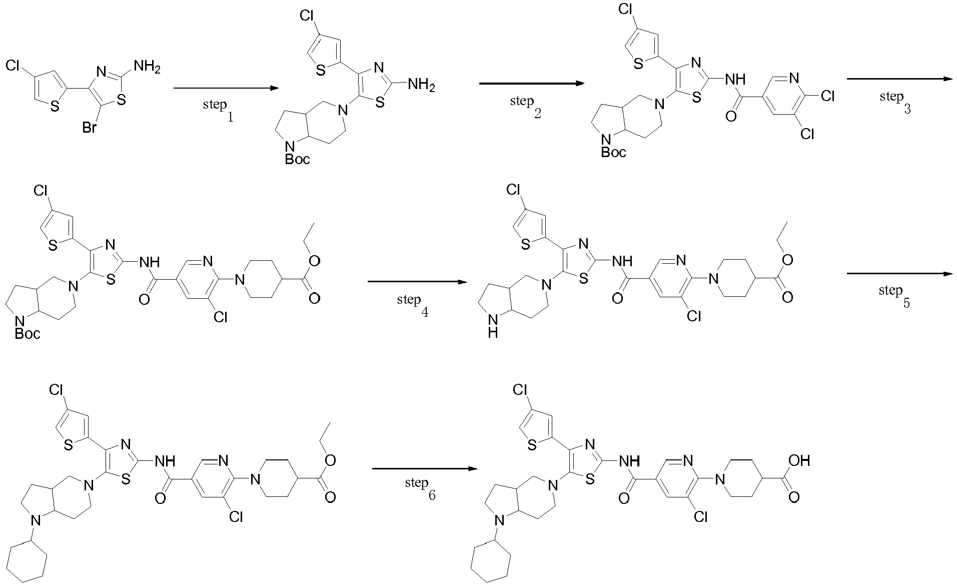

### Step 1: Synthesis of tert-butyl

### 5-(2-amino-4-(4-chlorothiophen-2-yl)thiazol-5-yl)-octahydro-1H-pyrrolo[3,2-c]pyridin-1-carboxylate

tert-butyl octahydro-1H-pyrrolo[3,2-c]pyridin-1-carboxylate (100mg, 0.4mmol), and 5-bromo-4-(4-chloro-thiophen-2-yl)-thiazole-2-amine (157mg, 0.5mmol) were added to N,N-dimethylformamide (4mL), and then triethylamine (447mg, 4.4mmol) was added. The mixture was warmed to 90°C and reacted for 6 h. The reaction mixture was poured into water, and extracted with ethyl acetate. The organic phases were combined and washed successively with water and a saturated aqueous sodium chloride solution, dried over anhydrous sodium sulfate, and filtered. The filtrate was concentrated to produce a crude product. The crude product was purified with a silica gel column chromatography to produce the title compound as a yellow solid (170mg).
ESI-MS (m/z): 441.2 [M + H]⁺

### Step 2: Synthesis of tert-butyl

### 5-(4-(4-chlorothiophen-2-yl)-2-(5,6-dichloronicotinamide)-thiazol-5-yl)-octahydro-1H-py rrolo[3,2-c]pyridin-1-carboxylate

The product from Step 1 (170mg, 0.4mmol), 5,6-dichloronicotinic acid (110mg, 0.6mmol), diphenyl chloridophosphate (207mg, 0.8mmol), and triethylamine (117mg, 1.2mmol) were added to chloroform (4mL). The mixture was warmed to 50°C and reacted for 1h. The reaction mixture was poured into water, and extracted with dichloromethane. The organic phases were combined and washed successively with water, saturated aqueous sodium chloride solution, dried over anhydrous sodium sulfate, and filtered. The filtrate was concentrated to produce a crude product. The crude product was purified with a silica gel column chromatography to produce the title compound (170mg).
ESI-MS (m/z): 614.0 [M + H]⁺

### Step 3: Synthesis of tert-butyl

### 5-(2-(5-chloro-6-(4-(ethoxycarbonyl)-piperidin-1-yl)-nicotinamido)-4-(4-chlorothiophen-2-yl)-thiazol-5-yl)-octahydro-1H-pyrrolo[3,2-c]pyridin-1-carboxylate

At room temperature, the product from Step 2 (170mg, 0.3mmol), ethyl 4-piperidine carboxylate (174mg, 1.1mmol), and triethylamine (84mg, 0.8mmol) were added to tetrahydrofuran (4.0mL). The mixture was warmed to 50°C and reacted for 16 h. The reaction mixture was poured into water, and extracted with ethyl acetate. The organic phases were combined and washed successively with water and a saturated aqueous sodium chloride solution, dried over anhydrous sodium sulfate, and filtered. The filtrate was concentrated to produce a crude product. The crude product was purified with a silica gel column chromatography to produce the title compound (150mg).
ESI-MS (m/z): 735.2 [M + H]⁺

### Step 4: Synthesis of ethyl

### 1-(3-chloro-5-((4-(4-chlorothiophen-2-yl)-5-(octahydro-1H-pyrrol[3,2-c]-pyridin-5(6H)-yl )-thiazol-2-yl)-carbamoyl)-pyridin-2-yl)-piperidine-4-carboxylate

The product from Step 3 (170mg, 0.2mmol) was added to dichloromethane (2.0mL), and trifluoroacetic acid (1mL) was added. The mixture was reacted at room temperature for 1h. The reaction mixture was adjusted with a saturated aquoues sodium bicarbonate solution to weak basicity (pH=9), and extracted with ethyl acetate. The organic phases were combined and washed successively with water and a saturated aqueous sodium chloride solution, dried over anhydrous sodium sulfate, and filtered. The filtrate was concentrated to produce the title compound (147mg). The product was directly used in the next reaction without purification.
ESI-MS (m/z): 635.2 [M + H]⁺

### Step 5: Synthesis of ethyl

### 1-(3-chloro-5-((4-(4-chlorothiophen-2-yl)-5-(1-cyclohexyloctahydro-1H-pyrrolo[3,2-c]py ridin-5(6H)-yl)-thiazol-2-yl)-aminoformamide)-pyridin-2-yl)-piperidine-4-carboxylate

The product from Step 4 (80mg, 0.1mmol), cyclohexanone (62mg, 0.6mmol), and glacial acetic acid (0.5mL) were dissolved in 1,4-dioxane (4mL), and sodium triacetyloxyborohydride (40mg, 0.6mmol) was added. The mixture was warmed to 60°C and reacted for 1h. The reaction mixture was added with a saturated aquoues sodium bicarbonate solution, and then extracted with ethyl acetate. The organic phases were combined and washed successively with water and a saturated aqueous sodium chloride solution, dried over anhydrous sodium sulfate, and filtered. The filtrate was concentrated to produce the title compound (90mg). The product was directly used in the next reaction without purification.
ESI-MS (m/z): 717.2 [M + H]⁺

### Step 6: Synthesis of

### 1-(3-chloro-5-((4-(4-chlorothiophen-2-yl)-5-(1-cyclohexyloctahydro-1H-pyrrolo[3,2-c]py ridin-5(6H)-yl)-thiazol-2-yl)-carbamoyl)-pyridin-2-yl)-piperidine-4-carboxylic acid

The product from Step 5 (80mg, 0.1mmol) was dissolved in a mixed solvent of tetrahydrofuran and water (3mL, V:V=2:1), and lithium hydroxide monohydrate (47mg, 1.1mmol) was added. The mixture was reacted at 40°C for 16 h. The reaction mixture was adjusted with a saturated citric acid solution to the acidity (pH=3), and extracted with a mixed solvent of ethyl acetate and tetrahydrofuran. The organic phases were combined, successively washed with water, a saturated aqueous sodium chloride solution, dried over anhydrous sodium sulfate, and filtered. The filtrate was concentrated to produce a crude title compound. The crude title compound was purified with a high performance liquid chromatography using a trifluoroacetic acid system to produce a trifluoroacetate salt of the title compound (15mg).
¹H NMR (400 MHz, DMSO-d₆): δ 12.64-12.28(m, 2H), 9.85-9.17(m, 1H), 8.83(d, J = 4.0hz, 1H), 8.39-8.38(m, 1H), 7.58-7.55(m, 1H), 7.44-7.43(m, 1H), 4.00-3.96(m, 3H), 3.62-2.33(m, 12H), 2.17-1.62(m, 12H), 1.42-1.14(m, 6H)
ESI-MS (m/z): 689.2 [M + H]⁺

### Example 14:

### 1-(3-chloro-5-((4-(4-chlorothiophen-2-yl)-5-(1-cyclopentyl-octahydro-1H-pyrrolo[3,2-c]p yridin-5(6H)-yl)-thiazol-2-yl)-carbamoyl)-pyridin-2-yl)-piperidine-4-carboxylic acid (K14)

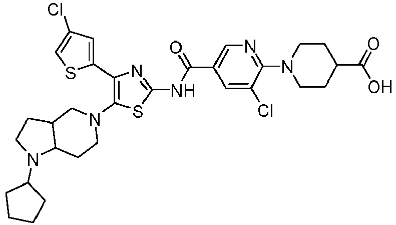

The procedure similar to that in Example 13, except that, cyclohexanone was replaced with cyclopentanone in Step 5 of Example 13 to produce a trifluoroacetate salt of the title compound (15mg).
¹H NMR (400 MHz, DMSO-d₆): δ 12.64-12.01(m, 2H), 9.89-9.40(m, 1H), 8.83(d, J = 2.0hz, 1H), 8.40-8.39(m, 1H), 7.58-7.56(m, 1H), 7.44-7.43(m, 1H), 3.99-3.56(m, 1H), 3.17-3.00(m, 4H), 2.77-2.67(m, 1H), 2.10-1.93(m, 7H), 1.74-1.57(m, 7H)
ESI-MS (m/z): 675.2 [M + H]⁺

### Example 15:

### 3'-chloro-5'-[4-(4-chlorothiophen-2-yl)-5-(2,6-diazaspiro[3.3]heptan-2-yl)-thiazol-2-carb amoyl]-3,4,5,6-tetrahydro-2H-[1,2']bipyridine-4-carboxylic acid (K15)

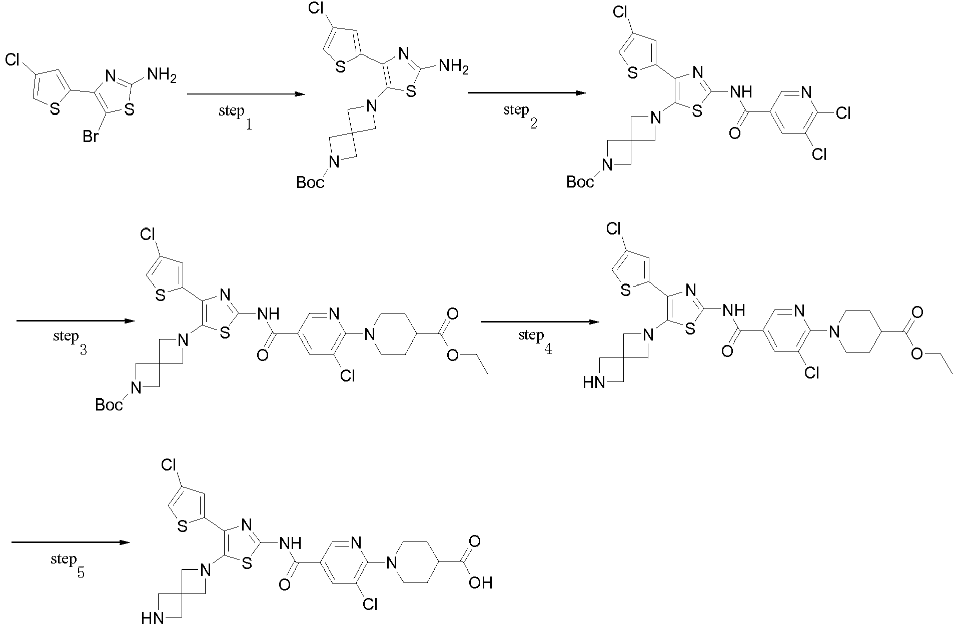

### Step 1: Synthesis of

### tert-butyl 6-(2-amino-4-(4-chloro-thiophen-2-yl)-thiazol-5-yl)-2,6-diazaspiro[3.3]heptane-2-carbo xylate

5-bromo-4-(4-chlorothiophen-2-yl)-2-amino-thiazole (250mg, 0.8mmol), tert-butyl 2,6-diazaspiro[3.3]heptane-2-carboxylate (244mg, 0.8mmol), and anhydrous potassium carbonate (350mg, 2.5mmol) were added to acetonitrile (2.5mL). The mixture was warmed to 80°C and reacted for 16 h. The reaction mixture was poured into ice water, extracted with ethyl acetate. The organic phases were combined and washed with a saturated aqueous sodium chloride solution, dried over anhydrous sodium sulfate, and filtered. The filtrate was evaporated under reduced pressure to remove the solvent to produce a crude product. The crude product was purified with silica gel column chromatography to produce the title compound (160mg).
ESI-MS (m/z): 413.0 [M + H]⁺

### Step 2: Synthesis of tert-butyl

### 6-(4-(4-chlorothiophen-2-yl)-2-(5,6-dichloronicotinamido)thiazol-5-yl)-2,6-diazaspiro[3. 3]heptane-2-carboxylate

The product from Step 1 (160mg, 0.4mmol), 5,6-dichloronicotinic acid (151mg, 0.8mmol), diphenyl chloridophosphate (208mg, 0.8mmol), and triethylamine (117mg, 1.2mmol) were dissolved in chloroform (3.2mL). The mixture was warmed to about 50°C and reacted for 3 h. The reaction mixture was cooled to room temperature, and water and dichloromethane were added. The organic phases were combined, successively washed with a saturated aqueous sodium chloride solution, dried over anhydrous sodium sulfate, and filtered. The filtrate was evaporated under reduced pressure to remove the solvent to produce a crude product. The crude product was purified with silica gel column chromatography to produce the title compound (190mg).
ESI-MS (m/z): 586.0 [M + H]⁺

### Step 3: Synthesis of ethyl

5'-(5-(6-tert-butyloxycarbonyl-2,6-diazaspiro[3.3]heptan-2-yl-4-(4-chloro-thiophen-2-yl)-thiazol-2-carbamoyl)-3'-chloro-3,4,5,6-tetrahydro-*2H*-[1,2']bipyridine-4-carboxylate The product from Step 2 (190mg, 0.3mmol), ethyl 4-piperidine carboxylate (102mg, 0.6mmol), and triethylamine (66mg, 0.6mmol) were dissolved in tetrahydrofuran (3.8mL). The mixture was warmed to 70°C and reacted for 16 h. The reaction mixture was cooled to room temperature. The solvent was removed by evaporation under reduced pressure to produce a crude product. The crude product was purified with silica gel column chromatography to produce the title compound (200mg).
ESI-MS (m/z): 707.2 [M + H]⁺

### Step 4: Synthesis of ethyl

### 3'-chloro-5'-(4-(4-chloro-thiophen-2-yl)-5-(2,6-diazaspiro[3.3]heptan-2-yl)-thiazol-2-car bamoyl)-3,4,5,6-tetrahydro-2H-[1,2']bipyridine-4-carboxylate

The product from Step 3 (200mg, 0.3mmol) was dissolved in dichloromethane (2.0mL), and trifluoroacetic acid (1.0mL) was added. The mixture was reacted at room temperature for 4h. The solvent was removed from the reaction mixture by evaporation under reduced pressure to produce a crude product. The crude product was extracted with ethyl acetate. The organic phases were combined, successively washed with asaturated sodium bicarbonate and a saturated aqueous sodium chloride solution, dried over anhydrous sodium sulfate, and filtered. The filtrate was evaporated under reduced pressure to remove the solvent to produce the title compound (170mg).
ESI-MS (m/z): 607.1 [M + H]⁺

### Step 5: Synthesis of

### 3'-chloro-5'-(4-(4-chloro-thiophen-2-yl)-5-(2,6-diazaspiro[3.3]heptan-2-yl)-thiazol-2-car bamoyl)-3,4,5,6-tetrahydro-2H-[1,2']bipyridine-4-carboxylic acid

The product from Step 4 (70mg, 0.1mmol) was dissolved in a mixed solvent of tetrahydrofuran and water (3mL, V:V=2:1), and lithium hydroxide monohydrate (29mg, 0.7mmol) was added. The mixture was reacted at room temperature for 3 h. The reaction mixture was adjusted with a saturated citric acid solution to the acidity (pH=3), extracted with a mixed solvent of ethyl acetate and tetrahydrofuran. The organic phases were combined, successively washed with water and a saturated aqueous sodium chloride solution, dried over anhydrous sodium sulfate, and filtered. The filtrate was concentrated to produce a crude title compound. The crude title compound was purified with a high performance liquid chromatography using a trifluoroacetic acid system to produce a trifluoroacetate salt of the title compound (39mg).
¹H NMR (400 MHz, DMSO-d₆): δ 12.58 (s, 1H), 12.30 (s, 1H), 8.83 (d, J = 2.1 Hz, 1H), 8.51 (s, 2H), 8.39 (d, J = 2.1 Hz, 1H), 7.49 (d, J = 1.5 Hz, 1H), 7.18 (d, J = 1.6 Hz, 1H), 4.21 (t, J = 6.0 Hz, 4H), 4.05 (s, 4H), 3.97 (s, J = 12.9 Hz, 2H), 3.03 (t, J = 11.6 Hz, 2H), 2.51-2.48 (m, 1H), 1.94 (d, J = 12.0 Hz, 2H), 1.67 (d, J = 11.1 Hz, 2H)
ESI-MS(m/z): 579.1 [M + H]⁺

### Example 16:

### 3'-chloro-5'-[4-(4-chlorothiophen-2-yl)-5-(6-iso-propyl-2,6-diazaspiro[3.3]heptan-2-yl)-t hiazol-2-carbamoyl]-3,4,5,6-tetrahydro-2H-[1,2']bipyridine-4-carboxylic acid (K16)

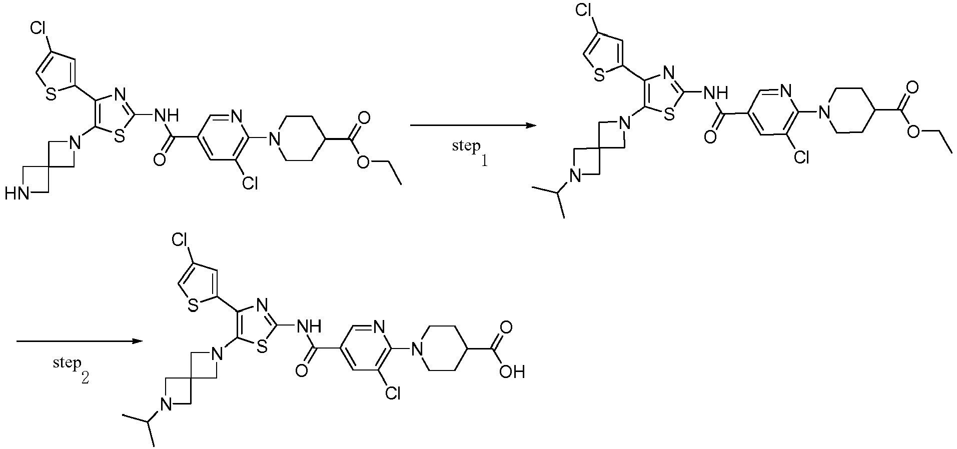

### Step 1: Synthesis of ethyl

### 3'-chloro-5'-(4-(4-chloro-thiophen-2-yl)-5-(6-iso-propyl-2,6-diazaspiro[3.3]heptan-2-yl)-t hiazol-2-carbamoyl)-3,4,5,6-tetrahydro-2H-[1,2']bipyridine-4-carboxylate ethyl

3'-chloro-5'-(4-(4-chloro-thiophen-2-yl)-5-(2,6-diazaspiro[3.3]heptan-2-yl)-thiazol-2-car bamoyl)-3,4,5,6-tetrahydro-2H-[1,2']bipyridine-4-carboxylate (100mg, 0.2mmol) was dissolved in methanol (2.0mL), and acetone (95mg, 1.6mmol), glacial acetic acid (24mg, 0.4mmol), and sodium triacetyloxyborohydride (212mg, 1.0mmol) were added. The mixture was warmed to 50°C and reacted for 16 h. After removing the solvent by evaporation under reduced pressure, the reaction mixture was dissolved with ethyl acetate and then washed with a saturated aqueous sodium chloride solution, dried over anhydrous sodium sulfate, and filtered. The filtrate was evaporated under reduced pressure to remove the solvent to produce the crude title compound (100mg). The product was directly used in the next step without purification.
ESI-MS (m/z): 649.2 [M + H]⁺

### Step 2: Synthesis of

### 3'-chloro-5'-(4-(4-chloro-thiophen-2-yl)-5-(6-iso-propyl-2,6-diazaspiro[3.3]heptan-2-yl)-t hiazol-2-carbamoyl)-3,4,5,6-tetrahydro-2H-[1,2']bipyridine-4-carboxylic acid

The product from Step 1 (100.0mg, 0.2mmol) was dissolved in a mixed solvent of tetrahydrofuran and water (3mL, V:V=2:1), and lithium hydroxidemonohydrate (50mg, 1.2mmol) was added. The mixture was reacted at room temperature for 3 h, adjusted with a saturated citric acid solution to the acidity (pH=3), and extracted with a mixed solvent of ethyl acetate and tetrahydrofuran, The organic phases were combined and washed successively with water, and a saturated aqueous sodium chloride solution, dried over anhydrous sodium sulfate, and filtered. The filtrate was concentrated to produce a crude title compound. The crude title compound was purified with a high performance liquid chromatography using a trifluoroacetic acid system to produce a trifluoroacetate salt of the title compound (52mg).
¹H NMR (400 MHz, DMSO-d₆): δ 12.59 (s, 1H), 12.32 (s, 1H), 9.99 (s, 1H), 8.83 (d, J = 2.1 Hz, 1H), 8.39 (d, J = 2.1 Hz, 1H), 7.51 (d, J = 1.5 Hz, 1H), 7.19 (d, J = 1.6 Hz, 1H), 4.34 (t, J = 3.6 Hz, 4H), 4.12 (s, 2H), 3.97 (d, J = 12.8 Hz, 4H), 3.08-2.98 (m, 2H), 2.55-2.48 (m, 1H), 1.94 (dd, J = 12.0 Hz, 3.8 Hz, 2H), 1.74-1.62 (m, 2H), 1.26-1.16 (m, 1H), 1.12 (d, J = 6.4 Hz, 6H)
ESI-MS(m/z): 621.1 [M + H]⁺

### Example 17:

### 3'-chloro-5'-(4-(4-chlorothiophen-2-yl)-5-(6-cyclohexyl-2,6-diazaspiro[3.3]heptan-2-yl)-t hiazol-2-carbamoyl)-3,4,5,6-tetrahydro-2H-[1,2']bipyridine-4-carboxylic acid (K17)

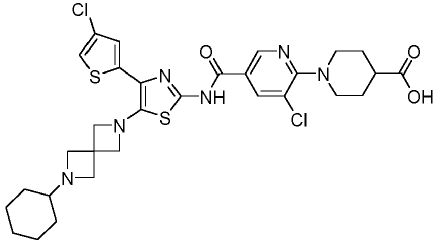

The procedure similar to that in Example 16, except thatacetone was replaced with cyclohexanone in Step 1 of Example 16, was used to produce a trifluoroacetate salt of the title compound (29mg).
¹H NMR (400 MHz, DMSO-d₆): δ 12.59 (s, 1H), 12.31 (s, 1H), 9.88 (s, 1H), 8.83 (d, J = 2.2 Hz, 1H), 8.39 (d, J = 2.1 Hz, 1H), 7.51 (d, J = 1.5 Hz, 1H), 7.19 (d, J = 1.6 Hz, 1H), 4.37 (dd, J = 6.5, 2.4 Hz, 4H), 4.13 (s, 2H), 3.97 (d, J = 10.8 Hz, 4H), 3.12-2.99 (m, 3H), 2.55-2.48 (m, 1H), 1.98-1.85 (m, 4H), 1.78-1.58 (m, 5H), 1.27-1.00 (m, 5H)
ESI-MS(m/z): 661.2 [M + H]⁺

### Example 18:

### 1-(5-((5-(2,5-diazabicyclo[2.2.1]heptan-2-yl)-4-(4-chlorothiophen-2-yl)thiazol-2-yl)carba moyl)-3-chloropyridin-2-yl)piperidine-4-carboxylic acid (K18)

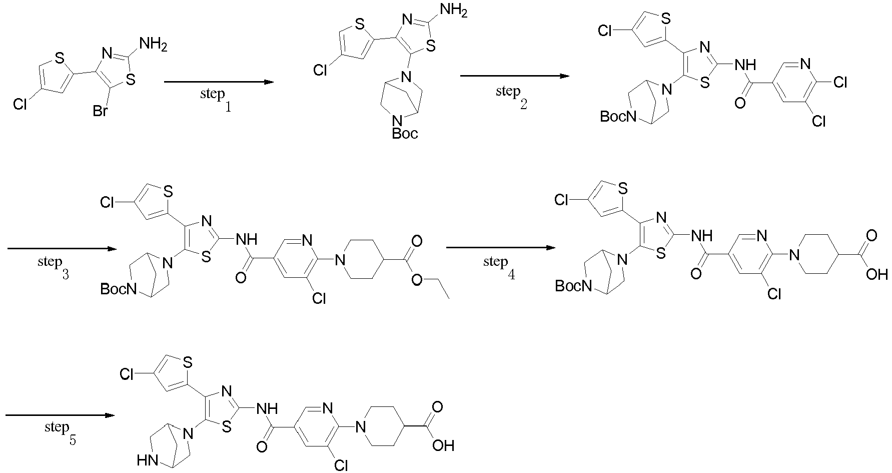

### Step 1: Synthesis of tert-butyl

### 5-(2-amino-4-(4-chlorothiophen-2-yl)-thiazol-5-yl)-2,5-diazabicyclo[2.2.1]heptane-2-car boxylate

5-bromo-4-(4-chlorothiophen-2-yl)-thiazole-2-amine (819mg, 2.77mmol), 2-tert-butyloxycarbonyl-2,5-diazabicyclo[2.2.1]heptane (500mg, 2.52mmol) and potassium carbonate (696mg, 5.04mmol) were added to N,N-dimethylformamide (15mL). The mixture was reacted at 90°C for 1h. The reaction system was added to water, extracted with ethyl acetate. The organic phases were combined, washed with a saturated aqueous sodium chloride solution, dried over anhydrous sodium sulfate, and filtered. The filtrate was evaporated under reduced pressure to remove the solvent to produce a crude product. The crude product was purified with a silica gel column chromatography to produce the title compound (930mg).
ESI-MS (m/z): 413.1 [M + H]⁺

### Step 2: Synthesis of tert-butyl

### 5-(4-(4-chlorothiophen-2-yl)-2-(5,6-dichloropyridin-3-formamide)-thiazol-5-yl)-2,5-diaza bicyclo[2.2.1]heptane-2-carboxylate

The product from Step 1 (840mg, 2.03mmol) and 5,6-dichloronicotinic acid (583mg, 3.05mmol) was dissolved in trichloromethane (15mL), and diphenyl chloridophosphate (817mg, 3.05mmol) and triethylamine (616mg, 6.09mmol) were added. The mixture was reacted at 50°C for 1h. The solvent was removed from the reaction mixture by evaporation under reduced pressure to produce a crude product. The crude product was purified with silica gel column chromatography to produce the title compound (1.1g).
ESI-MS (m/z): 586.1 [M + H]⁺

### Step 3: Synthesis of tert-butyl

### 5-(2-(5-chloro-6-(4-(ethoxycarbonyl)piperidin-1-yl)nicotinamido)-4-(4-chlorothiophen-2-yl)thiazol-5-yl)-2,5-diazabicyclo[2.2.1]heptane-2-carboxylate

The product from Step 2 (1.1g, 1.87mmol) and ethyl 4-piperidine carboxylate (588mg, 3.74mmol) were dissolved in tetrahydrofuran (20mL), and triethylamine (378mg, 3.74mmol) was added. The mixture was reacted at 60°C overnight. The solvent was removed from the reaction mixture by evaporation under reduced pressure to produce a crude product. The crude product was purified with silica gel column chromatography to produce the title compound (1.0g).
ESI-MS (m/z): 707.2 [M + H]⁺

### Step 4: Synthesis of

1-[5-((5-(5-(2-tert-butoxycarbonyl)-2,5-diazabicyclo[2.2.1]heptan-2-yl)-4-(4-chlorothiop hen-2-yl)-thiazol-2-yl)carbamoyl)-3-chloropyridin-2-yl]piperidine-4-carboxylic acid The product from Step 3 (100mg, 0.14mmol) was dissolved in ethanol (1.5mL), and lithium hydroxide monohydrate (40mg, 1.67mmol, dissolved in 0.5mL water) was added. The mixture was reacted at room temperature for 3h, adjusted with a saturated citric acid solution to the acidity (pH=3), and extracted with ethyl acetate. The organic phases were combined, washed with a saturated aqueous sodium chloride solution, dried over anhydrous sodium sulfate, and filtered. The filtrate was evaporated under reduced pressure to remove the solvent to produce the title compound (80mg).
ESI-MS (m/z): 679.2 [M + H]⁺

### Step 5: Synthesis of

### 1-(5-((5-(2,5-diazabicyclo[2.2.1]heptan-2-yl)-4-(4-chlorothiophen-2-yl)-thiazol-2-yl)-car bamoyl)-3-chloropyridin-2-yl)-piperidine-4-carboxylic acid

The product from Step 4 (80mg, 0.12mmol) was dissolved in dichloromethane (1.5mL), and trifluoroacetic acid (3mL) was added. The mixture was reacted at room temperature for 3h. The solvent was removed from the reaction mixture by evaporation under reduced pressure to produce a crude title compound. The crude title compound was purified with a high performance liquid chromatography using a trifluoroacetic acid system to produce a trifluoroacetate salt of the title compound (50mg).
¹H NMR (400 MHz, DMSO-d₆) δ 12.66 (s, 1H), 9.10-9.05 (m, 2H), 8.84 (s, 1H), 8.39 (s, 1H), 7.56 (s, 1H), 7.37 (s, 1H), 4.45 (s, 1H), 4.03-3.97 (m, 3H), 3.37-3.30 (m, 2H), 3.23-3.19 (m, 1H), 3.03 (t, J = 12.0 Hz, 2H), 2.56-2.53 (m, 1H), 2.25 (d, J = 10.8 Hz, 1H), 1.95 (d, J = 11.6 Hz, 3H), 1.73-1.63 (m, 2H), 1.23 (s, 1H)
ESI-MS (m/z): 579.2 [M + H]⁺

### Example 19: Synthesis of

### 1-(3-chloro-5((4-(4-chlorothiophen-2-yl)-5-(5-methyl-2,5-diazabicyclo[2.2.1]heptan-2-yl )thiazol-2-yl)carbamoyl)pyridin-2-yl)piperidine-4-carboxylic acid (K19)

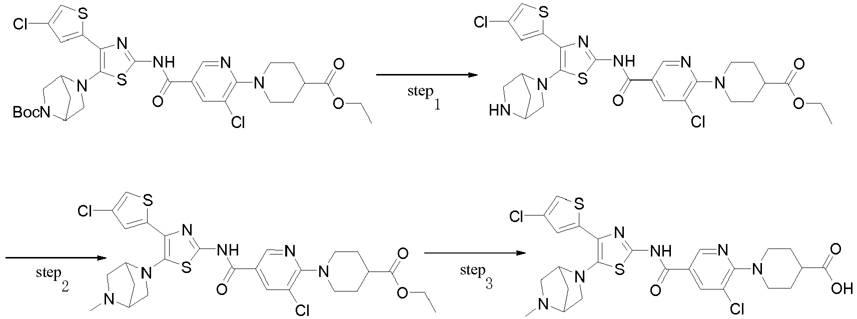

### Step 1: Synthesis of ethyl

### 1-(5-((5-(-2,5-diazabicyclo[2.2.1]heptan-2-yl)-4-(4-chlorothiophen-2-yl)-thiazol-2-yl)-car bamoyl)-3-chloropyridin-2-yl)-piperidine-4-carboxylate

tert-butyl 5-(2-(5-chloro-6-(4-(ethoxycarbonyl)-piperidin-1-yl)-nicotinamido)-4-(4-chlorothiophen-2-yl)-thiazol-5-yl)-2,5-diazabicyclo[2.2.1]heptane-2-carboxylate (1.0g, 1.41mmol) was dissolved in dichloromethane (2mL), and trifluoroacetic acid (6mL) was added. The mixture was reacted at room temperature for 20min. A saturated aquoues sodium carbonate solution was introduced to the reaction mixture. The resulting mixture was extracted with ethyl acetate. The organic phases were combined, washed with a saturated aqueous sodium chloride solution, dried over anhydrous sodium sulfate, and filtered. The filtrate was evaporated under reduced pressure to remove the solvent to produce the title compound (700mg).
ESI-MS (m/z): 607.1 [M + H]⁺

### Step 2: Synthesis of ethyl

### 1-(3-chloro-5-((4-(4-chlorothiophen-2-yl)-5-(5-methyl-2,5-diazabicyclo[2.2.1]heptan-2-yl )thiazol-2-yl)carbamoyl)pyridin-2-yl)piperidine-4-carboxylate

The product from Step 1 (100mg, 0.16mmol) was dissolved in 1,4-dioxane (2mL), and paraformaldehyde (58mg, 0.64mmol), glacial acetic acid (10mg, 0.16mmol)and sodium triacetylborohydride (102mg, 0.48mmol) were added. The mixture was reacted at 60°C for 4h. The solvent was removed from the reaction mixture by evaporation under reduced pressure to produce a crude product. The crude product was purified with a silica gel column chromatography to produce the title compound (95mg).
ESI-MS (m/z): 621.1 [M + H]⁺

### Step 3: Synthesis of

### 1-(3-chloro-(5-(4-(4-chlorothiophen-2-yl)-5-(5-methyl-2,5-diazabicyclo[2.2.1]heptan-2-y l)thiazol-2-yl)carbamoyl)pyridin-2-yl)piperidine-4-carboxylic acid

The product from Step 2 (95mg, 0.15mmol) was dissolved in a mixed solvent of tetrahydrofuran and water (3mL, V:V=2:1), and lithium hydroxide monohydrate (40mg, 1.67mmol) was added. The mixture was reacted at room temperature over night. The reaction mixture was adjusted with a diluted hydrochloric acid to the acidity (pH=4). The solvent was removed by evaporation under reduced pressure to produce a residue. The residuewas purified with a high performance liquid chromatography using a hydrochloric acid system to produce a hydrochloride salt of the title compound (25mg).
¹H NMR (400 MHz, DMSO-d6) δ 12.66 (s, 1H), 10.83 (s, 1H), 8.84 (s, 1H), 8.39 (s, 1H), 7.56 (s, 1H), 7.37 (s, 1H), 4.36 (s, 1H), 4.04-3.96 (m, 3H), 3.85-3.61 (m, 2H), 3.35 (d, J = 10.8 Hz, 1H), 3.06-3.01 (m, 3H), 2.88 (d, J = 4.4 Hz, 3H), 2.43-2.22 (m, 2H), 1.96-1.93 (m, 2H), 1.72-1.64 (m, 2H), 1.23 (s, 1H)
ESI-MS (m/z): 593.2 [M + H]⁺

### Example 20: Synthesis of

### 1-(3-chloro-5-((4-(4-chlorothiophen-2-yl)-5-(5-iso-propyl-2,5-diazabicyclo[2.2.1]heptan-2-yl)-thiazol-2-yl)-carbamoyl)-pyridin-2-yl)-piperidine-4-carboxylic acid (K20)

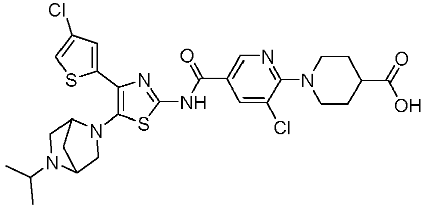

The procedure similar to that in Example 19, except that paraformaldehyde was replaced with acetone in Step 2 of Example 19, was used to produce a hydrochloride salt of the title compound (30mg).
¹H NMR (400 MHz, DMSO-d₆) δ 12.68 (s, 1H), 12.34 (s, 1H), 9.56 (s, 1H), 8.84 (s, 1H), 8.39 (s, 1H), 7.56 (s, 1H), 7.37 (s, 1H), 4.65 (s, 1H), 4.04-3.96 (m, 3H), 3.73-3.66 (m, 2H), 3.48-3.30 (m, 3H), 3.06-3.01 (m, 2H), 2.43-2.22 (m, 2H), 1.96-1.93 (m, 2H), 1.72-1.64 (m, 2H), 1.40-1.23 (m, 7H)
ESI-MS (m/z): 621.2 [M + H]⁺

### Example 21: Synthesis of

### 1-(3-chloro-5-((4-(4-chlorothiophen-2-yl)-5-(5-cyclobutyl-2,5-diazabicyclo[2.2.1]heptan-2-yl)-thiazol-2-yl)-carbamoyl)-pyridin-2-yl)-piperidine-4-carboxylic acid (K21)

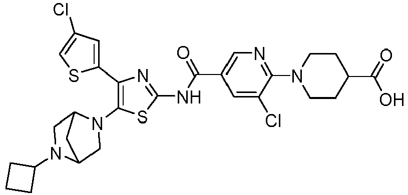

The procedure similar to that in Example 19, except that paraformaldehyde was replaced with cyclobutanone in Step 2 of Example 19, was used to produce a hydrochloride salt of the title compound (25mg).
¹H NMR (400 MHz, DMSO-d₆) δ 12.66 (s, 1H), 10.83 (s, 1H), 8.84 (d, J = 2.0 Hz, 1H), 8.40 (d, J = 2.0 Hz, 1H), 7.60 (d, J = 1.2 Hz, 1H), 7.40 (d, J = 1.6 Hz, 1H), 4.29 (s, 1H), 4.04-3.63 (m, 6H), 3.37-3.34 (m, 1H), 3.10-3.00 (m, 3H), 2.57-2.53 (m, 1H), 2.37-2.18 (m, 6H), 1.96-1.63 (m, 6H)
ESI-MS (m/z): 633.2 [M + H]⁺

### Example 22: Synthesis of

### 1-(3-chloro-5((4-(4-chlorothiophen-2-yl)-5-(5-cyclopentyl-2,5-diazabicyclo[2.2.1]heptan -2-yl)-thiazol-2-yl)-carbamoyl)-pyridin-2-yl)-piperidine-4-carboxylic acid (K22)

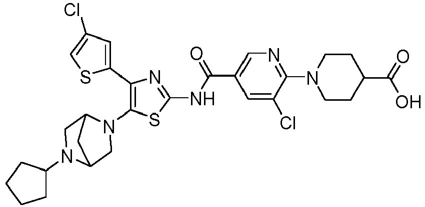

The procedure similar to that in Example 19, except that paraformaldehyde was replaced with cyclopentanone in Step 2 of Example 19, was used to produce a hydrochloride salt of the title compound (35mg).
¹H NMR (400 MHz, DMSO-d₆) δ 12.68 (s, 1H), 10.41 (s, 1H), 8.84 (d, J = 2.0 Hz, 1H), 8.40 (d, J = 1.6 Hz, 1H), 7.56 (d, J = 1.6 Hz, 1H), 7.40 (d, J = 1.6 Hz, 1H), 4.47-4.46 (m, 1H), 4.04-3.97 (m, 3H), 3.82-3.62 (m, 3H), 3.26-3.24 (m, 2H), 3.06-3.01 (m, 2H), 2.57-2.53 (m, 1H), 2.37-2.18 (m, 6H), 1.96-1.63 (m, 8H)
ESI-MS (m/z): 647.2 [M + H]⁺

### Example 23:

### 1-(3-chloro-5-((4-(4-chlorothiophen-2-yl)-5-(5-cyclohexyl-2,5-diazabicyclo[2.2.1]heptan -2-yl)-thiazol-2-yl)-carbamoyl)-pyridin-2-yl)-piperidine-4-carboxylic acid (K23)

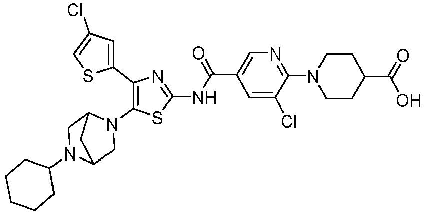

The procedure similar to that in Example 19, except that paraformaldehyde was replaced with cyclohexanone in Step 2 of Example 19, was used to produce a hydrochloride salt of the title compound (31mg).
¹H NMR (400 MHz, DMSO-d₆) δ 12.68 (s, 1H), 12.33 (s, 1H), 9.52 (s, 1H), 8.84 (s, 1H), 8.39 (s, 1H), 7.56 (s, 1H), 7.37 (s, 1H), 4.65 (s, 1H), 4.04-3.96 (m, 3H), 3.70-3.60 (m, 1H), 3.48-3.36 (m, 3H), 3.30-3.15 (m, 1H), 3.06-3.01 (m, 2H), 2.57-2.53 (m, 1H), 2.36-2.22 (m, 2H), 2.08-2.04 (m, 1H), 1.96-1.78 (m, 4H), 1.72-1.64 (m, 3H), 1.36-1.12 (m, 6H)
ESI-MS (m/z): 661.2 [M + H]⁺

### Example 24:

### 3'-chloro-5'-(4-(4-chlorothiophen-2-yl)-5-(2,5-diazabicyclo[2.2.2]octan-2-yl)-thiazol-2-ca rbamoyl)-3,4,5,6-tetrahydro-2H-[1,2']bipyridine-4-carboxylic acid (K24)

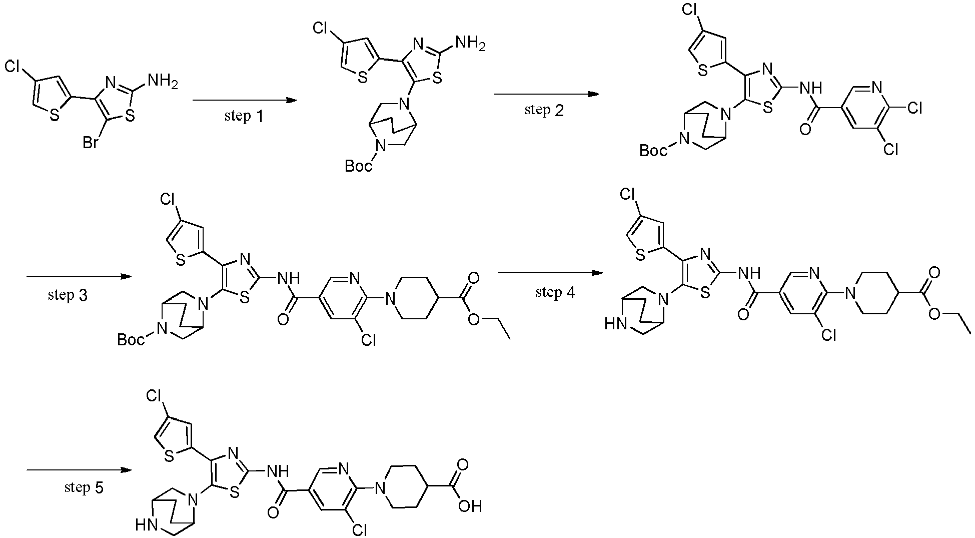

### Step 1: Synthesis of tert-butyl

### 5-(2-amino-4-(4-chloro-thiophen-2-yl)-thiazol-5-yl)-2,5-diazabicyclo[2.2.2]octane-2-car boxylate

5-bromo-4-(4-chlorothiophen-2-yl)-2-amino-thiazole (630mg, 2.2mmol), tert-butyl 2, 5-diazabicyclo[2.2.2]octane-2-carboxylate (450mg, 2.2mmol), and anhydrous potassium carbonate (589mg, 4.4mmol) were added to N,N-dimethylformamide (13.0mL). The mixture was reacted at 80°C for 1h. The reaction mixture was poured into ice water, and extracted with ethyl acetate. The organic phases were combined, washed with a saturated aqueous sodium chloride solution, dried over anhydrous sodium sulfate, and filtered. The filtrate was evaporated under reduced pressure to remove the solvent to produce a crude product. The crude product was purified with a silica gel column chromatography to produce the title compound (750mg).
ESI-MS (m/z): 427.1 [M + H]⁺

### Step 2: Synthesis of tert-butyl

### 5-(4-(4-chloro-thiophen-2-yl)-2-((5,6-dichloro-pyridin-3-carbonyl)-amino)-thiazol-5-yl)-2 ,5-diazabicyclo[2.2.2]octane-2-carboxylate

The product from Step 1 (750mg, 1.8mmol), 5,6-dichloronicotinic acid (685mg, 3.6mmol), diphenyl chloridophosphate (965mg, 3.6mmol), triethylamine (545mg, 5.4mmol) were dissolved in chloroform (15mL). The mixture was reacted at 50°C for 3 h. The reaction mixture was poured into 100ml water, and extracted with dichloromethane. The organic phases were combined, washed with a saturated aqueous sodium chloride solution, dried over anhydrous sodium sulfate, and filtered. The filtrate was evaporated under reduced pressure to remove the solvent to produce a crude product. The crude product was purified with silica gel column chromatography to produce the title compound (1.0g).
ESI-MS (m/z): 600.0 [M + H]⁺

### Step 3: Synthesis of ethyl

5'-(5-(5-tert-butyloxycarbonyl-2,5-diazabicyclo[2.2.2]octan-2-yl)-4-(4-chloro-thiophen-2 -yl)-thiazol-2-carbamoyl)-3'-chloro-3,4,5,6-tetrahydro-2*H*-[1,2']bipyridine-4-carboxylate The product from Step 2 (1.0g, 1.7mmol), ethyl 4-piperidine carboxylate (522mg, 3.4mmol), and triethylamine (343mg, 3.4mmol) were dissolved in tetrahydrofuran (20.0mL). The mixture was reacted at 70°C for 16 h. The solvent was removed from the reaction mixture by evaporation under reduced pressure to produce a crude product. The crude product was purified with silica gel column chromatography to produce the title compound (1.1g).
ESI-MS (m/z): 721.2 [M + H]⁺

### Step 4: Synthesis of ethyl

### 5'-(5-(2,5-diazabicyclo[2.2.2]octan-2-yl)-4-(4-chloro-thiophen-2-yl)-thiazol-2-carbamoyl )-3'-chloro-3,4,5,6-tetrahydro-2H-[1,2']bipyridine-4-carboxylate

The product from Step 3 (800mg, 1.5mmol) was dissolved in dichloromethane (12.0mL), and trifluoroacetic acid (4.0mL) was added. The mixture was reacted at room temperature for 3 h, adjusted with saturated sodium bicarbonate to weak basicity (pH=8), and extracted with ethyl acetate. The organic phases were combined, washed with a saturated aqueous sodium chloride solution, dried over anhydrous sodium sulfate, and filtered. The filtrate was evaporated under reduced pressure to remove the solvent to produce the title compound (670mg).
ESI-MS (m/z): 621.1 [M + H]⁺

### Step 5: Synthesis of

### 3'-chloro-5'-(4-(4-chlorothiophen-2-yl)-5-(2,5-diazabicyclo[2.2.2]octan-2-yl)-thiazol-2-ca rbamoyl)-3,4,5,6-tetrahydro-2H-[1,2']bipyridine-4-carboxylic acid

The product from Step 4 (100mg, 0.2mmol) was dissolved in a mixed solvent of tetrahydrofuran and water (3mL, V:V=2:1), and lithium hydroxide monohydrate (50mg, 1.2mmol) was added. The mixture was reacted at room temperature for 3 h. The reaction mixture was adjusted with a saturated citric acid solution to the acidity (pH=3), and extracted with a mixed solvent of ethyl acetate and tetrahydrofuran. The organic phases were combined and washed successively with water and a saturated aqueous sodium chloride solution, dried over anhydrous sodium sulfate, and filtered. The filtrate was concentrated to produce a crude title compound. The crude title compound was purified with HPLC using a trifluoroacetic acid system to produce a trifluoroacetate salt of the title compound (50mg).
¹H NMR (400 MHz, DMSO-d₆): δ 12.68 (s, 1H), 12.32 (s, 1H), 9.08 (s, 1H), 8.96 (s, 1H), 8.84 (d, J = 2.1 Hz, 1H), 8.40 (d, J = 2.2 Hz, 2H), 7.57 (d, J = 1.6 Hz, 1H), 7.46 (d, J = 1.6 Hz, 1H), 4.01-3.96 (m, 2H), 3.76 (s, 1H), 3.64-3.53 (m, 2H), 3.31-3.24 (m, 1H), 3.03 (s, 1H), 3.03-2.92 (m, 1H), 2.55-2.48 (m, 1H), 2.31 (d, J = 12.8 Hz, 1H), 2.08 (d, J = 11.8 Hz, 1H), 2.09-1.92 (m, 3H), 1.86-1.75 (m, 1H), 1.75-1.60 (m, 2H)
ESI-MS (m/z) : 593.2 [M + H]⁺

### Example 25:

### 3'-chloro-5'-(4-(4-chlorothiophen-2-yl)-5-(5-methyl-2,5-diazabicyclo[2.2.2]octan-2-yl)-th iazol-2-carbamoyl)-3,4,5,6-tetrahydro-2H-[1,2']bipyridine-4-carboxylic acid (K25)

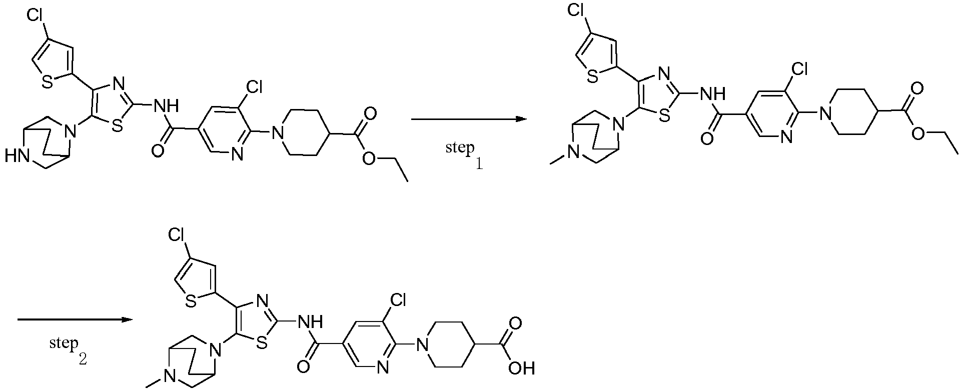

### Step 1: Synthesis of ethyl

### 3'-chloro-5'-(4-(4-chlorothiophen-2-yl)-5-(5-methyl-2,5-diazabicyclo[2.2.2]octan-2-yl)-th iazol-2-carbamoyl)-3,4,5,6-tetrahydro-2H-[1,2']bipyridine-4-carboxylate ethyl

3'-chloro-5'-(4-(4-chlorothiophen-2-yl)-5-(2,5-diazabicyclo[2.2.2]octan-2-yl)-thiazol-2-ca rbamoyl)-3,4,5,6-tetrahydro-2H-[1,2']bipyridine-4-carboxylate (100mg, 0.2mmol) was dissolved in anhydrous methanol (2.0mL), and paraformaldehyde (60mg, 0.2mmol), glacial acetic acid (24mg, 0.4mmol), sodium triacetyloxyborohydride (212mg, 1.0mmol) were added. The mixture was reacted at 50°C for 16 h, evaporated under reduced pressure to remove the solvate, dissolved in ethyl acetate washed with a saturated aqueous sodium chloride solution, and dried over anhydrous sodium sulfate. The solvent was removed by evaporation under reduced pressure to produce the crude title compound (100mg). The product was directly used in the next reaction without further purification.
ESI-MS (m/z): 635.1 [M + H]⁺

### Step 2: Synthesis of

### 3'-chloro-5'-(4-(4-chlorothiophen-2-yl)-5-(5-methyl-2,5-diazabicyclo[2.2.2]octan-2-yl)-th iazol-2-carbamoyl)-3,4,5,6-tetrahydro-2H-[1,2']bipyridine-4-carboxylic acid

The product from Step 1 (100mg, 0.2mmol) was dissolved in a mixed solvent of tetrahydrofuran and water (3mL, V:V=2:1), and lithium hydroxide monohydrate (50mg, 1.2mmol) was added. The mixture was reacted at room temperature for 3 h. The reaction mixture was adjusted with a saturated citric acid solution to the acidity (pH=3), and extracted with a mixed solvent of ethyl acetate and tetrahydrofuran. The organic phases were combined and washed successively with water and a saturated aqueous sodium chloride solution, dried over anhydrous sodium sulfate, and filtered. The filtrate was concentrated to produce a crude title compound. The crude title compound was purified with HPLC using a hydrochloric acid system to produce a hydrochloride salt of the title compound (55mg).
¹H NMR (400 MHz, DMSO-d₆): δ 12.67 (d, J = 11.2 Hz, 1H), 11.22 (d, J = 29.1 Hz, 1H), 8.84 (d, J = 2.1 Hz, 1H), 8.40 (t, J = 1.7 Hz, 1H), 7.61-7.48 (m, 1H), 7.41 (d, J = 1.6 Hz, 1H), 3.98 (d, J = 13.1 Hz, 3H), 3.80 (d, J = 12.3 Hz, 1H), 3.68 (s, 2H), 3.35 (dd, J = 23.3 Hz, 11.3 Hz, 3H), 3.03 (t, J = 12.0 Hz, 2H), 2.92 (dd, J = 17.1,4.9 Hz, 3H), 2.56-2.50 (m, 1H), 2.28 (d, J = 12.2 Hz, 2H), 2.08-1.91 (m, 3H), 1.90-1.74 (m, 1H), 1.74-1.62 (m, 2H) ESI-MS (m/z): 607.1 [M + H]⁺

### Example 26:

### 3'-chloro-5'-(4-(4-chlorothiophen-2-yl)-5-(5-iso-propyl-2,5-diazabicyclo[2.2.2]octan-2-yl )-thiazol-2-carbamoyl)-3,4,5,6-tetrahydro-2H-[1,2']bipyridine-4-carboxylic acid (K26)

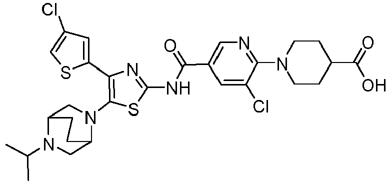

The procedure similar to that in Example 25, except that paraformaldehyde was replaced with acetone in Step 1 of Example 25, was used to produce a hydrochloride salt of the title compound (31mg).
¹H NMR (400 MHz, DMSO-d₆): δ 12.68 (d, J = 5.9 Hz, 1H), 10.47 (s, 1H), 8.84 (d, J = 2.1 Hz, 1H), 8.41 (t, J = 2.3 Hz, 2H), 7.57 (d, J = 1.5 Hz, 1H), 7.45 (dd, J = 28.0, 1.6 Hz, 1H), 4.01-3.96 (m, 2H), 3.92-3.59 (m, 4H), 3.39-3.24 (m, 3H), 3.10-2.99 (m, 2H), 2.58-2.52 (m, 1H), 2.45-2.18 (m, 2H), 2.07-1.76 (m, 4H), 1.74-1.61 (m, 2H), 1.46-1.30 (m, 6H)
ESI-MS (m/z):635.2 [M + H]⁺

### Example 27:

### 3'-chloro-5'-(4-(4-chlorothiophen-2-yl)-5-(5-cyclobutyl-2,5-diazabicyclo[2.2.2]octan-2-yl )-thiazol-2-carbamoyl)-3,4,5,6-tetrahydro-2H-[1,2']bipyridine-4-carboxylic acid (K27)

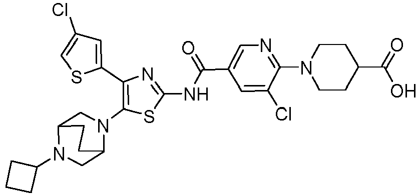

The procedure similar to that in Example 25, except that paraformaldehyde was replaced with cyclobutanone in Step 1 of Example 25, the purification using a hydrochloric acid system with a high performance liquid chromatography was replaced with the purification using a trifluoroacetic acid system with a high performance liquid chromatography in Step 2 of Example 25, was used to produce a trifluoroacetate salt of the title compound (41mg).
¹H NMR (400 MHz, DMSO-d₆): δ 12.68 (s, 1H), 12. 33 (s, 1H), 10.27 (s, 1H), 8.84 (d, J = 2.0hz, 1H), 8.40 (s, 1H), 7.56 (s, 1H), 7.44 (d, J = 15.2 Hz, 1H), 4.16 (d, J = 50.7 Hz, 1H), 3.98 (d, J = 12.9 Hz, 2H), 3.89-3.61 (m, 3H), 3.30 (s, 2H), 3.03 (t, J = 2.4 Hz, 3H), 2.55-2.49 (m, 1H), 2.35-2.14 (m, 6H), 1.94 (d, J = 13.6 Hz, 3H), 1.73-1.36 (m, 5H)
ESI-MS (m/z): 647.1 [M + H]⁺

### Example 28:

### 3'-chloro-5'-(4-(4-chlorothiophen-2-yl)-5-(5-cyclopentyl-2,5-diazabicyclo[2.2.2]octan-2-yl)-thiazol-2-carbamoyl)-3,4,5,6-tetrahydro-2H-[1,2']bipyridine-4-carboxylic acid (K28)

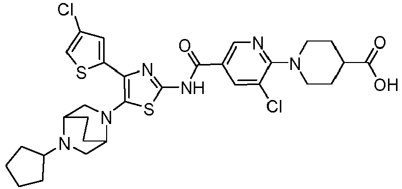

The procedure similar to that in Example 25, except that paraformaldehyde was replaced with cyclopentanone in Step 2 of Example 25, the purification using a hydrochloric acid system with a high performance liquid chromatography was replaced with the purification using a trifluoroacetic acid system with a high performance liquid chromatography in Step 3 of Example 25, was used to produce a trifluoroacetate salt of the title compound (46mg).
¹H NMR (400 MHz, DMSO-d₆): δ 12.68 (s, 1H), 12.33 (s, 1H), 9.91 (s, 1H), 8.84 (d, J = 2.1 Hz, 1H), 8.40 (t, J = 2.1 Hz, 1H), 7.57 (s, 1H), 7.45 (d, J = 12.0 Hz, 1H), 3.98 (d, J = 2.0 Hz, 3H), 3.85-3.62 (m, 3H), 3.36-3.25 (m, 2H), 3.03 (t, J = 2.5 Hz, 2H), 2.55-2.49 (m, 1H), 2.35-2.11 (m, 5H), 1.96-1.83 (m, 3H), 1.76-1.52 (m, 9H)
ESI-MS (m/z): 661.2 [M + H]⁺

### Example 29:

### 3'-chloro-5'-(4-(4-chlorothiophen-2-yl)-5-(5-cyclohexyl-2,5-diazabicyclo[2.2.2]octan-2-yl )-thiazol-2-carbamoyl)-3,4,5,6-tetrahydro-2H-[1,2']bipyridine-4-carboxylic acid (K29)

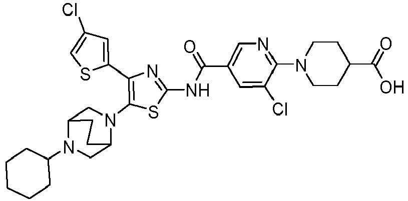

The procedure similar to that in Example 25, except that paraformaldehyde was replaced with cyclohexanone in Step 1 of Example 25, was used to produce a hydrochloride salt of the title compound (60mg).
¹H NMR (400 MHz, DMSO-d₆): δ 12.66 (d, J = 9.9 Hz, 1H), 10.78 (s, 1H), 8.84 (d, J = 2.2 Hz, 1H), 8.40 (d, J = 2.2 Hz, 2H), 7.56 (dd, J = 7.7, 1.6 Hz, 1H), 7.45 (dd, J = 28.5, 1.6 Hz, 1H), 4.03-3.89 (m, 4H), 3.71 (d, J = 12.8 Hz, 1H), 3.42-3.22 (m, 4H), 3.08-2.98 (m, 2H), 2.58-2.52 (m, 1H), 2.36-2.04 (m, 4H), 1.99-1.91 (m, 3H), 1.85 (s, 3H), 1.73-1.61 (m, 4H), 1.53-1.13 (m, 5H)
ESI-MS (m/z): 675.2 [M + H]⁺

### Example 30: Synthesis of

### 3'-chloro-5'-(4-(4-chlorothiophen-2-yl)-5-(5-cyclohexyl-octahydropyrrolo[3,4-c]pyridin-2 -yl)-thiazol-2-carbamoyl)-3,4,5,6-tetrahydro-2H-[1,2']bipyridine-4-carboxylic acid (K30)

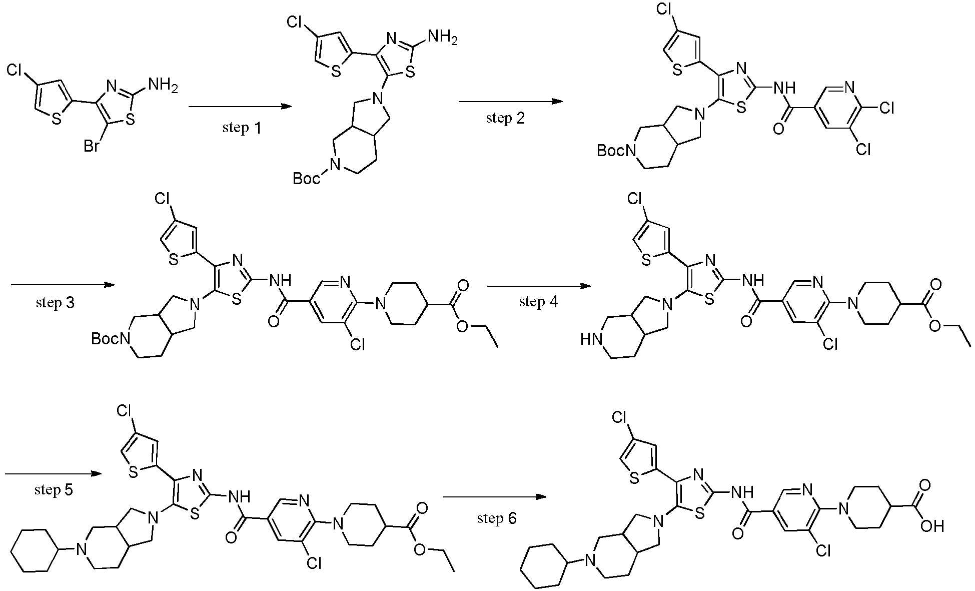

### Step 1: Synthesis of tert-butyl

### 2-(2-amino-4-(4-chloro-thiophen-2-yl)-thiazol-5-yl)-octahydropyrrolo[3,4-c]pyridin-5-car boxylate

5-bromo-4-(4-chlorothiophen-2-yl)-2-amino-thiazole (260mg, 0.9mmol), 5-tert-butyloxycarbonyl-octahydropyrrolo[3,4-C]pyridine (200mg, 0.9mmol), and anhydrous potassium carbonate (250mg, 1.8mmol) were dissolved in N,N-dimethylformamide (5.2mL). The mixture was reacted at 80°C for 1h. The reaction mixture was poured into ice water (20mL), and extracted with ethyl acetate. The organic phases were combined and washed successively with water and a saturated aqueous sodium chloride solution, dried over anhydrous sodium sulfate, and filtered. The filtrate was concentrated to produce a crude product. The crude product was purified with a silica gel column chromatography to produce the title compound (250mg).
ESI-MS (m/z): 441.1 [M + H]⁺

### Step 2: Synthesis of tert-butyl

### 2-(4-(4-chlorothiophen-2-yl)-2-(5,6-dichloronicotinamido)thiazol-5-yl)hexahydro-1H-pyr rolo[3,4-c]pyridine-5(6H)-carboxylate

The product from Step 1 (150mg, 0.3mmol), 5,6-dichloronicotinic acid (98mg, 0.45mmol), diphenyl chloridophosphate (134mg, 0.45mmol), and triethylamine (90mg, 0.9mmol) were dissolved in chloroform (3.0mL). The mixture was reacted at 50°C for 3 h. The reaction mixture was poured into water, and extracted with dichloromethane. The organic phases were combined and washed successively with water and a saturated aqueous sodium chloride solution, dried over anhydrous sodium sulfate, and filtered. The filtrate was concentrated to produce a crude product. The crude product was purified with a silica gel column chromatography to produce the title compound (200mg).
ESI-MS (m/z): 614.1 [M + H]⁺

### Step 3: Synthesis of ethyl

### 5'-(5-(5-tert-butyloxycarbonyl-octahydropyrrolo[3,4-c]pyridin-2-yl)-4-(4-chloro-thiophen-2-yl)-thiazol-2-carbamoyl)-3'-chloro-3,4,5,6-tetrahydro-2H-[1,2']bipyridine-4-carboxylat e

The product from Step 2 (200.0mg, 0.3mmol), ethyl 4-piperidine carboxylate (102.0mg, 0.6mmol), and triethylamine (66mg, 0.6mmol) were dissolved in tetrahydrofuran (4.0mL). The mixture was reacted at 70°C for 16 h. The solvent was removed by evaporation under reduced pressure to produce a crude product. The crude product was purified with silica gel column chromatography to produce the title compound (210mg).
ESI-MS (m/z): 735.2 [M + H]⁺

### Step 4: Synthesis of ethyl

### 3'-chloro-5'-(4-(4-chloro-thiophen-2-yl)-5-(octahydropyrrolo[3,4-c]pyridin-2-yl)-thiazol-2 -carbamoyl)-3,4,5,6-tetrahydro-2H-[1,2']bipyridine-4-carboxylate

The product from Step 3 (210mg, 1.5mmol) was dissolved in dichloromethane (2.1mL), and trifluoroacetic acid (1.0mL) was added dropwisely. The mixture was reacted at room temperature for 3 h. The reaction mixture was concentrated, adjusted with a saturated sodium bicarbonate to weak basicity (pH=8), and extracted with ethyl acetate. The organic phases were combined, washed with a saturated aqueous sodium chloride solution, dried over anhydrous sodium sulfate, and filtered. The filtrate was evaporated under reduced pressure to remove the solvent to produce the title compound (150mg).
ESI-MS (m/z): 635.1 [M + H]⁺

### Step 5: Synthesis of ethyl

### 3'-chloro-5'-(4-(4-chloro-thiophen-2-yl)-5-(5-cyclohexyl-octahydropyrrolo[3,4-c]pyridin-2-yl)-thiazol-2-carbamoyl)-3,4,5,6-tetrahydro-2H-[1,2']bipyridine-4-carboxylate

To a 10mL one-neck flask were added the product from Step 4 (70mg, 0.1mmol), 1,4-dioxane (1.4mL), cyclohexanone (108mg, 1.0mmol), glacial acetic acid (12mg, 0.2mmol), and sodium cyanoborohydride (33mg, 0.5mmol). The mixture was reacted at 60°C for 2 h. The reaction mixture was dissolved in ethyl acetate after removing the solvent under a reduced pressure, washed with a saturated aqueous sodium chloride solution, dried over anhydrous sodium sulfate, and filtered. The filtrate was evaporated under reduced pressure to remove the solvent to produce the title compound (70mg). The product was directly used in the next reaction without further purification.
ESI-MS (m/z): 717.2 [M + H]⁺

### Step 6: Synthesis of

### 3'-chloro-5'-(4-(4-chlorothiophen-2-yl)-5-(5-cyclohexyl-octahydropyrrolo[3,4-c]pyridin-2 -yl)-thiazol-2-carbamoyl)-3,4,5,6-tetrahydro-2H-[1,2']bipyridine-4-carboxylic acid

The product from Step 5 (70mg, 0.1mmol) was dissolved in a mixed solvent of tetrahydrofuran and water (3mL, V:V=2:1), and lithium hydroxide monohydrate (25mg, 0.6mmol) was added. The mixture was reacted at room temperature for 3 h. The reaction mixture was adjusted with a saturated citric acid solution to the acidity (pH=3), and extracted with a mixed solvent of ethyl acetate and tetrahydrofuran. The organic phases were combined and washed successively with water and a saturated aqueous sodium chloride solution, dried over anhydrous sodium sulfate, and filtered. The filtrate was concentrated to produce a crude title compound. The crude title compound was purified with HPLC using a trifluoroacetic acid system to produce a trifluoroacetate salt of the title compound (25mg).
¹H NMR (400 MHz, DMSO-d₆): δ 12.58 (s, 1H), 12.30 (s, 1H), 9.18(s, 0.5H), 8.83 (t, J = 1.8 Hz, 1H), 8.75 (s, 0.5H), 8.39 (t, J = 1.9 Hz, 1H), 7.53 (dd, J = 9.2, 1.5 Hz, 1H), 7.25 (t, J = 1.9 Hz, 1H), 3.97 (d, J = 13.1 Hz, 3H), 3.53-3.41 (m, 3H), 3.37-3.29 (m, 2H), 3.20 (d, J = 13.8 Hz, 1H), 3.12 (t, J = 9.4 Hz, 1H), 3.03 (t, J = 12.1 Hz, 2H), 2.85-2.71 (m, 2H), 2.66-2.55 (m, 1H), 2.50-2.45 (m, 1H), 2.07-1.75 (m, 8H), 1.71-1.62 (m, 3H), 1.52-1.42 (m, 2H), 1.35-1.27 (m, 3H), 1.21-1.14 (m, 1H)
ESI-MS (m/z): 689.2 [M + H]⁺

### Example 31:

### 3'-chloro-5'-(4-(4-chlorothiophen-2-yl)-5-(5-cyclopentyl-octahydropyrrol[3,4-c]pyridin-2-yl)-thiazol-2-carbamoyl)-3,4,5,6-tetrahydro-2H-[1,2']bipyridine-4-carboxylic acid (K31)

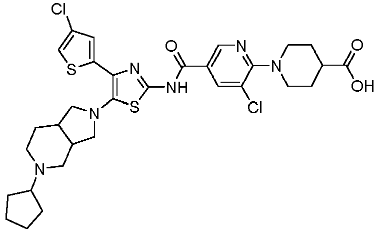

The procedure similar to that in Example 30, except that cyclohexanone was replaced with cyclopentanone in Step 5 of Example 30, was used to produce a trifluoroacetate salt of the title compound (28mg).
¹H NMR (400 MHz, DMSO-d₆): δ 12.57 (s, 1H), 9.43 (s, 0.5H), 9.11 (s, 0.5H), 8.83 (d, J = 1.9 Hz, 1H), 8.38 (d, J = 1.8 Hz, 1H), 7.51 (d, J = 2.1 Hz, 1H), 7.24 (dd, J = 9.0 Hz, 1.3 Hz, 1H), 3.97 (d, J = 13.2 Hz, 2H), 3.74-3.50 (m, 4H), 3.46-3.37 (m, 2H), 3.32-3.22 (m, 1H), 3.18-3.10 (m, 1H), 3.03-2.95 (m, 2H), 2.86-2.78 (m, 2H), 2.66-2.55 (m, 1H), 2.47-2.42 (m, 1H), 2.15-1.92 (m, 3H), 1.82-1.71 (m, 3H), 1.70-1.61 (m, 6H), 1.51-1.41 (m, 2H)
ESI-MS (m/z): 675.2 [M + H]⁺

### Example 32:

### 3'-chloro-5'-(4-(4-chlorothiophen-2-yl)-5-(5-cyclohexyl-hexahydropyrrolo[3,4-b]pyrrol-1-yl)-thiazol-2-carbamoyl)-3,4,5,6-tetrahydro-2H-[1,2']bipyridine-4-carboxylic acid (K32)

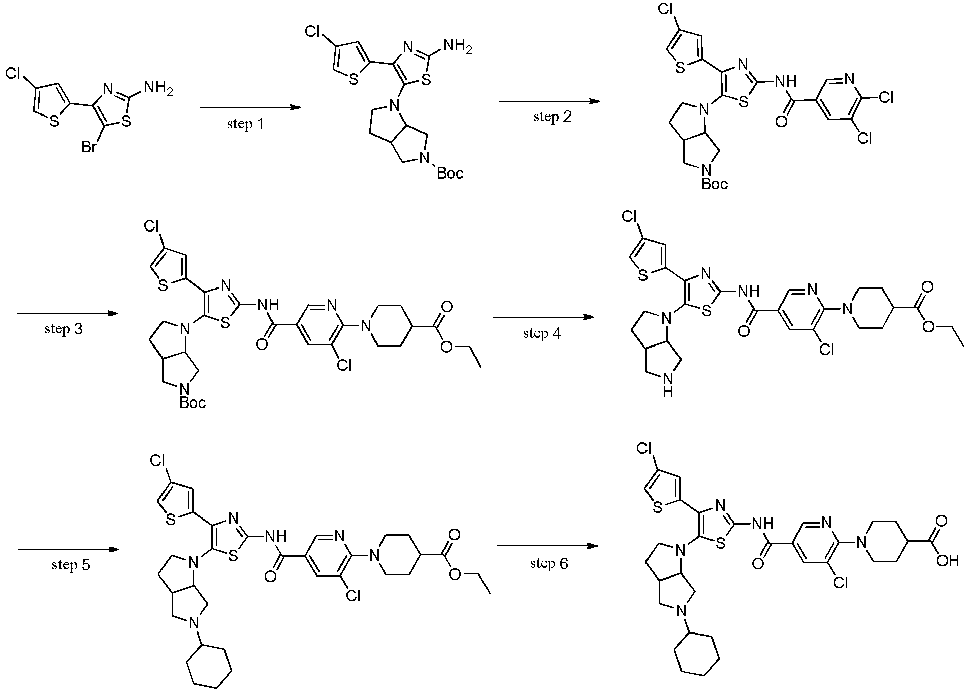

### Step 1: Synthesis of tert-butyl

### 1-(2-amino-4-(4-chloro-thiophen-2-yl)-thiazol-5-yl)-hexahydropyrrolo[3,4-b]pyrrole-5-ca rboxylate

5-bromo-4-(4-chlorothiophen-2-yl)-2-amino-thiazole (200mg, 0.7mmol), 5-tert-butyloxycarbonyl-hexahydropyrrolo[3,4-b]pyrrol (144mg, 0.7mmol), and anhydrous potassium carbonate (190mg, 1.4mmol) was suspended in N,N-dimethylformamide (4.0mL). The mixture was reacted at 80°C for 1h. The reaction mixture was poured into 20mL ice water, extracted with ethyl acetate. The organic phases were combined, washed with a saturated aqueous sodium chloride solution, dried over anhydrous sodium sulfate, and filtered. The filtrate was evaporated under reduced pressure to remove the solvent to produce a crude product. The crude product was purified with a silica gel column chromatography to produce the title compound (180mg).
ESI-MS (m/z): 427.1 [M + H]⁺

### Step 2: Synthesis of

### tert-butyl-1-(4-(4-chlorothiophen-2-yl)-2-(5,6-dichloronicotinamido)thiazol-5-yl)hexahyd ropyrrolo[3,4-b]pyrrole-5(1H)-carboxylate

The product from Step 1 (180mg, 0.4mmol), 5,6-dichloronicotinic acid (122mg, 0.6mmol), diphenyl chloridophosphate (169mg, 0.6mmol), and triethylamine (81mg, 0.8mmol) were dissolved in chloroform (4.0mL). The mixture was reacted at 50°C for 3 h. The reaction mixture was poured into 100ml water, and extracted with dichloromethane. The organic phases were combined, washed with a saturated aqueous sodium chloride solution, dried over anhydrous sodium sulfate, and filtered. The filtrate was evaporated under reduced pressure to remove the solvent to produce a crude product. The crude product was purified with silica gel column chromatography to produce the title compound (190mg).
ESI-MS (m/z): 600.0 [M + H]⁺

### Step 3: Synthesis of ethyl

### 5'-(5-(5-tert-butyloxycarbonyl-hexahydropyrrolo[3,4-b]pyrrol-1-yl)-4-(4-chloro-thiophen-2-yl)-thiazol-2-carbamoyl)-3'-chloro-3,4,5,6-tetrahydro-2H-[1,2']bipyridine-4-carboxylat e

The product from Step 2 (190mg, 0.3mmol), and ethyl 4-piperidine carboxylate (100mg, 0.6mmol), triethylamine (71mg, 0.7mmol) were dissolved in tetrahydrofuran (4.0mL). The mixture was reacted at 70°C for 16 h. The solvent was removed from the reaction mixture by evaporation under reduced pressure to produce a crude product. The crude product was purified with silica gel column chromatography to produce the title compound (190mg).
ESI-MS (m/z): 721.2[M + H]⁺

### Step 4: Synthesis of ethyl

### 3'-chloro-5'-(4-(4-chloro-thiophen-2-yl)-5-(hexahydropyrrolo[3,4-b]pyrrol-1-yl)-thiazol-2-carbamoyl)-3,4,5,6-tetrahydro-2H-[1,2']bipyridine-4-carboxylate

The product from Step 3 (190mg, 0.3mmol) was dissolved in dichloromethane (2.0mL), and trifluoroacetic acid (1.0mL) was added dropwisely. The mixture was reacted at room temperature for 3 h. The reaction mixture was concentrated, adjusted with a saturated sodium bicarbonate to weak basicity (pH=8), and extracted with ethyl acetate. The organic phases were combined, washed with a saturated aqueous sodium chloride solution, dried over anhydrous sodium sulfate, and filtered. The filtrate was evaporated under reduced pressure to remove the solvent to produce the title compound (150mg).
ESI-MS (m/z): 621.1 [M + H]⁺

### Step 5: Synthesis of ethyl

### 3'-chloro-5'-(4-(4-chloro-thiophen-2-yl)-5-(5-cyclohexyl-hexahydropyrrolo[3,4-b]pyrrol-1 -yl)-thiazol-2-carbamoyl)-3,4,5,6-tetrahydro-2H-[1,2']bipyridine-4-carboxylate

The product from Step 4 (150mg, 0.2mmol) was dissolved in anhydrous methanol (5.0mL), and cyclohexanone (235mg, 2.0mmol), glacial acetic acid (30mg, 0.5mmol), and sodium triacetyloxyborohydride (212mg, 1.0mmol) were added. The mixture was reacted at 50°C for 16 h. The reaction mixture was concentrated, dissolved in ethyl acetate (20mL), washed with a saturated aqueous sodium chloride solution, dried over anhydrous sodium sulfate, and filtered. The filtrate was evaporated under reduced pressure to remove the solvent to produce a crude product (160mg). The product was directly used in the next reaction without further purification.
ESI-MS (m/z): 703.2 [M + H]⁺

### Step 6: Synthesis of

### 3'-chloro-5'-(4-(4-chlorothiophen-2-yl)-5-(5-cyclohexyl-hexahydropyrrolo[3,4-b]pyrrol-1-yl)-thiazol-2-carbamoyl)-3,4,5,6-tetrahydro-2H-[1,2']bipyridine-4-carboxylic acid

The product from Step 5 (160mg, 0.2mmol) was dissolved in a mixed solvent of tetrahydrofuran and water (3mL, V:V=2:1), and lithium hydroxide monohydrate (60mg, 1.4mmol) was added. The mixture was reacted at room temperature for 3 h. The reaction mixture was adjusted with a saturated citric acid solution to the acidity (pH=3), and extracted with a mixed solvent of ethyl acetate and tetrahydrofuran. The organic phases were combined and washed successively with water and a saturated aqueous sodium chloride solution, dried over anhydrous sodium sulfate, and filtered. The filtrate was concentrated to produce a crude title compound. The crude title compound was purified with HPLC using a trifluoroacetic acid system to produce a trifluoroacetate salt of the title compound (30mg).
¹H NMR (400 MHz, DMSO-d₆): δ 12.65 (d, J = 7.8 Hz, 1H), 12.29 (s, 1H), 9.78 (s, 0.5H), 9.28 (s, 0.5H), 8.84 (d, J = 2.1 Hz, 1H), 8.39 (d, J = 2.1 Hz, 1H), 7.57 (dd, J = 4.1, 1.5 Hz, 1H), 7.54 (d, J = 1.6 Hz, 0.5H), 7.38 (d, J = 1.6 Hz, 0.5H), 4.07-3.97 (m, 3H), 3.84-3.74 (m, 2H), 3.65-3.55 (m, 3H), 3.46-3.37 (m, 2H), 3.29-3.18 (m, 2H), 2.76-2.68 (m, 1H), 2.52-2.46 (m, 1H), 2.35-2.24 (m, 1H), 2.03-1.88 (m, 5H), 1.80-1.52 (m, 4H), 1.45-1.14 (m, 6H)
ESI-MS (m/z): 675.2 [M + H]⁺

### Example 33:

### 1-(3-chloro-5-((4-(4-chloropyridin-2-yl)-5-(2-cyclohexyl-hexahydropyrrolo[3,4-c]pyrrol-5 -yl)-thiazol-2-yl)-carbamoyl)-pyridin-2-yl)-pyridin-4-carboxylic acid (K33)

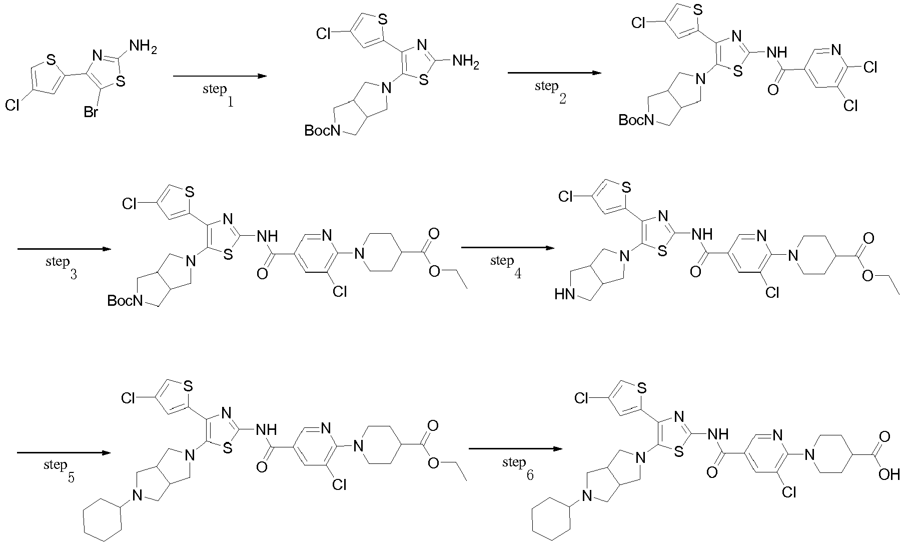

### Step 1: Synthesis of tert-butyl

### 5-(2-amino-4-(4-chlorothiophen-2-yl)-thiazol-5-yl)hexahydropyrrolo[3,4-c]pyrrole-2(1H) -carboxylate

5-bromo-4-(4-chlorothiophen-2-yl)-thiazole-2-amine (200mg, 0.68mmol), tert-butyl hexahydropyrrolo[3,4-c]pyrrol-2-carboxylate (144mg, 0.68mmol) and potassium carbonate (188mg, 1.36mmol) were suspended in N,N-dimethylformamide (2mL). The mixture was reacted at 90°C for 2 h. The reaction mixture was poured into water, and extracted with ethyl acetate. The organic phases were combined, washed with a saturated aqueous sodium chloride solution, dried over anhydrous sodium sulfate, and filtered. The filtrate was evaporated under reduced pressure to remove the solvent to produce a crude product. The crude product was purified with a silica gel column chromatography to produce the title compound (150mg).
ESI-MS (m/z): 427.1 [M + H]⁺

### Step 2: Synthesis of tert-butyl

### 5-(4-(4-chlorothiophen-2-yl)-2-(5,6-dichloropyridin-3-formamide)-thiazol-5-yl)hexahydr opyrrolo[3,4-c]pyrrole-2(1H)-carboxylate

The product from Step 1 (150mg, 0.35mmol) and 5,6-dichloronicotinic acid (101mg, 0.53mmol) were dissolved in trichloromethane (3mL), and thendiphenyl chloridophosphate (141mg, 0.53mmol) and N,N-diisopropylethylamine (89mg, 0.7mmol) were added dropwisely. The mixture was reacted at 60°C for 1h. The solvent was removed from the reaction mixture by evaporation under reduced pressure to produce a crude product. The crude product was purified with silica gel column chromatography to produce the title compound (140mg).
ESI-MS (m/z): 600.1 [M + H]⁺

### Step 3: Synthesis of tert-butyl

### 5-(2-(5-chloro-6-(4-ethoxycarbonyl-piperidin-1-yl)nicotinamide)-4-(4-chlorothiophen-2-yl)thiazol-5-yl)hexahydropyrrolo[3,4-c]pyrrole-2(1H)-carboxylate

The product from Step 2 (140mg, 0.23mmol) and ethyl 4-piperidine carboxylate (72mg, 0.46mmol) were dissolved in tetrahydrofuran (2mL), and then triethylamine (47mg, 0.46mmol) was added dropwisely. The mixture was reacted at 60°C overnight. The solvent was removed from the reaction mixture by evaporation under reduced pressure to produce a crude product. The product was purfied with silica gel column chromatography to produce the title compound (135mg).
ESI-MS (m/z): 721.2 [M + H]⁺

### Step 4: Synthesis of ethyl

### 1-(3-chloro-5-((4-(4-chlorothiophen-2-yl)-5-(hexahydropyrrolo[3,4-C]pyrrol-2(1H)-yl)-thi azol-2-yl)-carbamoyl)-pyridin-2-yl)-piperidine-4-carboxylate

The product from Step 3 (135mg, 0.19mmol) was dissolved in dichloromethane (2mL), and trifluoroacetic acid (6mL) was added. The mixture was reacted at room temperature for 20min. A saturated sodium carbonate solution was introduced to the reaction mixture. The resulting mixture was extracted with ethyl acetate. The organic phases were combined, washed with a saturated aqueous sodium chloride solution, dried over anhydrous sodium sulfate, and filtered. The filtrate was evaporated under reduced pressure to remove the solvent to produce the title compound (120mg).
ESI-MS (m/z): 621.2 [M + H]⁺

### Step 5: Synthesis of ethyl

### 1-(3-chloro-5-((4-(4-chlorothiophen-2-yl)-5-(5-cyclohexyl-hexahydropyrrolo[3,4-c]pyrrol -2(1H)-yl)-thiazol-2-yl)carbamoyl)-pyridin-2-yl)-piperidine-4-carboxylate

The product from Step 4 (100mg, 0.19mmol) was dissolved in 1,4-dioxane (2mL), and cyclohexanone (75mg, 0.76mmol) and glacial acetic acid (12mg, 0.19mmol) were added. The mixture was reacted at 60°C for 1h. Then sodium triacetylborohydride (102mg, 0.48mmol) was added, and the resulting mixture was reacted at 60°C for 3 h. The solvent was removed from the reaction mixture by evaporation under reduced pressure to produce a crude product. The crude product was purified with silica gel column chromatography to produce the title compound (90mg).
ESI-MS (m/z): 703.2 [M + H]⁺

### Step 6: Synthesis of

### 1-(3-chloro-5-((4-(4-chlorothiophen-2-yl)-5-(5-cyclohexylhexahydropyrrolo[3,4-C]pyrrol -2(1H)-yl)thiazol-2-yl)-carbamoyl)-pyridin-2-yl)-piperidine-4-carboxylic acid

The product from Step 5 (90mg, 0.15mmol) was dissolved in a mixed solvent of tetrahydrofuran and water (3mL, V:V=2:1), and lithium hydroxide monohydrate (40mg, 1.67mmol) was added. The mixture was reacted at room temperature over night. The reaction mixture was adjusted with 1N hydrochloric acid to acidity (pH=4). The solvent was removed from the reaction mixture by evaporation under reduced pressure to produce a crude title compound. The crude title compound was purified with HPLC using a hydrochloric acid system to produce a hydrochloride salt of the title compound (40mg).
¹H NMR (400 MHz, DMSO-d6) δ 12.68 (s, 1H), 10.41 (s, 1H), 8.84 (d, J = 2.0 Hz, 1H), 8.40 (d, J = 1.6 Hz, 1H), 7.56 (d, J = 1.6 Hz, 1H), 7.40 (d, J = 1.6 Hz, 1H), 4.00-3.93 (m, 3H), 3.64-3.61 (m, 1H), 3.41 -2.91 (m, 11H), 2.57-2.51 (m, 1H), 2.12-1.62 (m, 9H), 1.46-1.10 (m, 5H)
ESI-MS (m/z): 675.2 [M + H]⁺

### Example 34:

### 3'-chloro-5'-(5-(1-cyclohexyl-hexahydropyrrolo[3,4-b]pyrrol-5-yl)-4-(3-trifluoromethylph enyl)-thiazol-2-carbamoyl)-3,4,5,6-tetrahydro-2H-[1,2']bipyridine-4-carboxylic acid (K34)

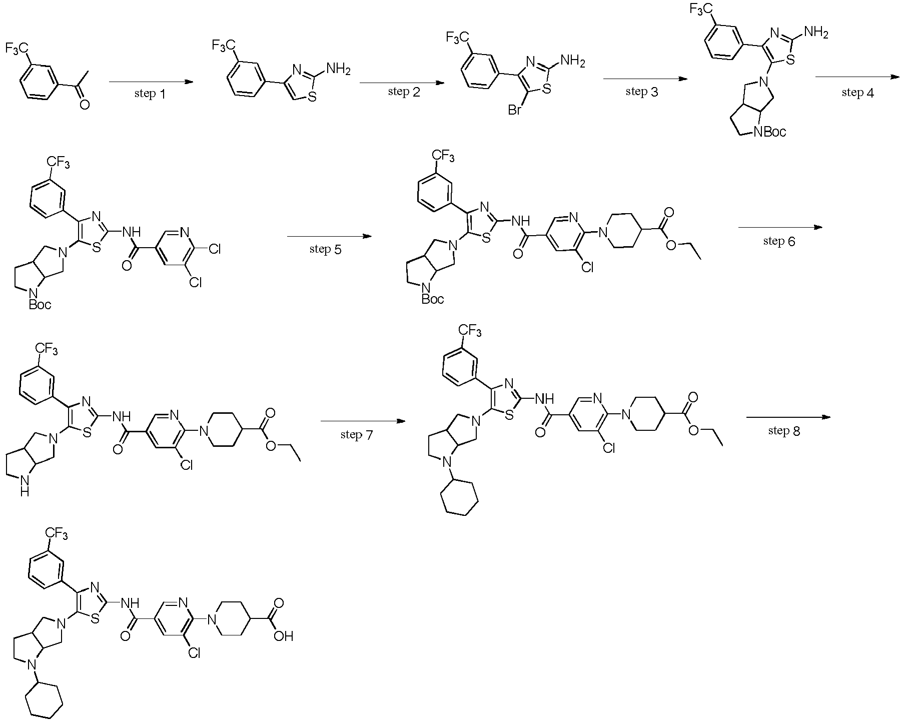

### Step 1: Synthesis of 4-(3-trifluoromethyl-phenyl)-thiazole-2-amine

Trifluoroacetophenone (1.0g, 5.3mmol), thiourea(0.8g, 10.6mmol), triethylamine (2.1g, 21.2mmol), and carbon tetrabromide (7.0g, 21.2mmol) were dissolved in anhydrous acetonitrile (20.0mL). The mixture was reacted at room temperature for 3 h. The reaction mixture was poured into water, and extracted with ethyl acetate. The organic phases were combined, washed with a saturated aqueous sodium chloride solution, dried over anhydrous sodium sulfate, and filtered. The filtrate was evaporated under reduced pressure to remove the solvent to produce a crude product. The crude product was purified with silica gel column chromatography to produce the title compound (0.85g).
ESI-MS (m/z): 245.2 [M + H]⁺

### Step 2: Synthesis of 5-bromo-4-(3-trifluoromethyl-phenyl)-thiazole-2-amine

The product from Step 1 (0.85g, 3.5mmol) was dissolved in N,N-dimethylformamide (10.0mL) to produce a stock solution. N-bromosuccimide (0.65g, 3.5mmol) was dissolved in N,N-dimethylformamide (5mL), and the resulting solution was slowly added dropwisely to the above stock solution. The mixture was reacted at room temperature for 1h. The reaction mixture was poured into ice water (100mL), and filtered by suction. The filter cake was washed with water, and dried to produce the title compound (0.75g).
ESI-MS (m/z): 323.0 [M + H]⁺

### Step 3: Synthesis of tert-butyl

### 5-(2-amino-4-(3-trifluoromethylphenyl)-thiazol-5-yl)-hexahydropyrrolo[3,4-b]pyrrole-1-c arboxylate

The product from Step 2 (150mg, 0.5mmol), tert-butyl hexahydropyrrolo[3,4-b]pyrrole-1-carboxylate (100mg, 0.5mmol), and anhydrous potassium carbonate (128mg, 0.9mmol) were suspended in N,N-dimethylformamide (3.0mL). The mixture was reacted at 80°C for 1h. The reaction mixture was poured into 20ml ice water, and extracted with ethyl acetate. The organic phases were combined, washed with a saturated aqueous sodium chloride solution, dried over anhydrous sodium sulfate, and filtered. The filtrate was evaporated under reduced pressure to remove the solvent to produce a crude product. The crude product was purified with a silica gel column chromatography to produce the title compound (110mg).
ESI-MS (m/z): 455.2 [M + H]⁺

### Step 4: Synthesis of tert-butyl

5-[2-[(5,6-dichloro-pyridin-3-carbonyl)-amino]-4-(3-trifluoromethylphenyl)-thiazol-5-yl]-h exahydropyrrolo[3,4-b]pyrrole-1-carboxylate The product from Step 3 (110mg, 0.2mmol), 5,6-dichloronicotinic acid (92mg, 0.4mmol), diphenyl chloridophosphate (96mg, 0.3mmol), and triethylamine (73mg, 0.7mmol) were dissolved in chloroform (2.5mL). The mixture was reacted at 50°C for 3 h. The reaction mixture was poured into 20ml water, and extracted with dichloromethane. The organic phases were combined, washed with a saturated aqueous sodium chloride solution, dried over anhydrous sodium sulfate, and filtered. The filtrate was evaporated under reduced pressure to remove the solvent to produce a crude product. The crude product was purified with a fast silica gel column chromatography to produce the title compound (120mg).
ESI-MS (m/z): 628.1 [M + H]⁺

### Step 5: Synthesis of ethyl

### 5'-(5-(1-tert-butyloxycarbonyl-hexahydropyrrolo[3,4-b]pyrrol-5-yl)-4-(3-trifluoromethylph enyl)-thiazol-2-carbamoyl)-3'-chloro-3,4,5,6-tetrahydro-2H-[1,2']bipyridine-4-carboxylat e

The product from Step 4 (120mg, 0.2mmol), ethyl 4-piperidine carboxylate (60mg, 0.4mmol), and triethylamine (50mg, 0.4mmol) were dissolved in tetrahydrofuran (3.0mL). The mixture was reacted at 70°C for 16 h. The solvent was removed from the reaction mixture by evaporation under reduced pressure to produce a crude product. The crude product was purified with silica gel column chromatography to produce the title compound (130mg).
ESI-MS (m/z): 749.2 [M + H]⁺

### Step 6: Synthesis of ethyl

### 3'-chloro-5'-(5-(hexahydropyrrolo[3,4-b]pyrrol-5-yl)-4-(3-trifluoromethylphenyl)-thiazol-2 -carbamoyl)-3,4,5,6-tetrahydro-2H-[1,2']bipyridine-4-carboxylate

The product from Step 5 (130mg, 0.2mmol) was dissolved in dichloromethane (2.0mL), and trifluoroacetic acid (1.0mL) was added dropwisely. The mixture was reacted at room temperature for 3 h. The reaction mixture was concentrated, adjusted with a saturated sodium bicarbonate solution to weak basicity (pH=8), and extracted with ethyl acetate. The organic phases were combined, washed with a saturated aqueous sodium chloride solution, dried over anhydrous sodium sulfate, and filtered. The filtrate was evaporated under reduced pressure to remove the solvent to produce the title compound (100mg).
ESI-MS (m/z): 649.2 [M + H]⁺

### Step 7: Synthesis of ethyl

### 3'-chloro-5'-(5-(1-cyclohexyl-hexahydropyrrolo[3,4-b]pyrrol-5-yl)-4-(3-trifluoromethylph enyl)-thiazol-2-carbamoyl)-3,4,5,6-tetrahydro-2H-[1,2']bipyridine-4-carboxylate

The product from Step 6 (100mg, 0.2mmol) was dissolved in anhydrous methanol (3.0mL), and cyclohexanone (196mg, 2.0mmol), glacial acetic acid (18mg, 0.3mmol), and sodium triacetyloxyborohydride (212mg, 1.0mmol) were added. The mixture was reacted at 50°C for 16 h. The reaction mixture was concentrated, dissolved in ethyl acetate, washed with a saturated aqueous sodium chloride solution, dried over anhydrous sodium sulfate, and filtered. The filtrate was evaporated under reduced pressure to remove the solvent to produce the title compound (110mg). The product was directly used in the next step without purification.
ESI-MS (m/z): 731.3[M + H]⁺

### Step 8: Synthesis of

### 3'-chloro-5'-(5-(1-cyclohexyl-hexahydropyrrolo[3,4-b]pyrrol-5-yl)-4-(3-trifluoromethylph enyl)-thiazol-2-carbamoyl)-3,4,5,6-tetrahydro-2H-[1,2']bipyridine-4-carboxylic acid

The product from Step 7(110mg, 0.2mmol) was dissolved in a mixed solvent of tetrahydrofuran and water (3mL, V:V=2:1), and lithium hydroxide monohydrate (50mg, 1.2mmol) was added. The mixture was reacted at room temperature for 3 h. The reaction mixture was adjusted with a saturated citric acid solution to the acidity (pH=3), extracted with a mixed solvent of ethyl acetate and tetrahydrofuran. The organic phases were combined and washed successively with water and a saturated aqueous sodium chloride solution, dried over anhydrous sodium sulfate, and filtered. The filtrate was concentrated to produce a crude title compound. The crude title compound was purified with HPLC using a trifluoroacetic acid system to produce a trifluoroacetate salt of the title compound (30mg).
¹H NMR (400 MHz, DMSO-d₆): δ 12.61 (s, 1H), 12.29 (s, 1H), 9.55 (s, 1H), 8.85 (d, J = 2.2 Hz, 1H), 8.39 (d, J = 2.1 Hz, 1H), 8.34 (dd, J = 6.7, 1.8 Hz, 1H), 8.29 (s, 1H), 7.75 - 7.67 (m, 2H), 4.38-4.32 (m, 1H), 3.98 (d, J = 13.1 Hz, 2H), 3.71-3.57 (m, 3H), 3.45-3.38 (m, 1H), 3.30-3.18 (m, 5H), 2.77-2.68 (m, 1H), 2.55-2.48 (m, 1H), 2.37-2.26 (m, 1H), 2.18-2.03 (m, 2H), 1.95-1.86 (m, 2H), 1.80-1.71 (m, 3H), 1.68-1.53 (m, 3H), 1.44-1.12 (m, 5H)
ESI-MS (m/z): 703.2 [M + H]⁺

### Example 35:

### (E)-3-(2,6-dichloro-4-(5-(1-cyclohexyl-hexahydropyrrolo[3,4-b]pyrrol-5-yl)-4-(3-trifluoro methylphenyl)-thiazol-2-carbamoyl)-phenyl)-2-methyl-acrylic acid (K35)

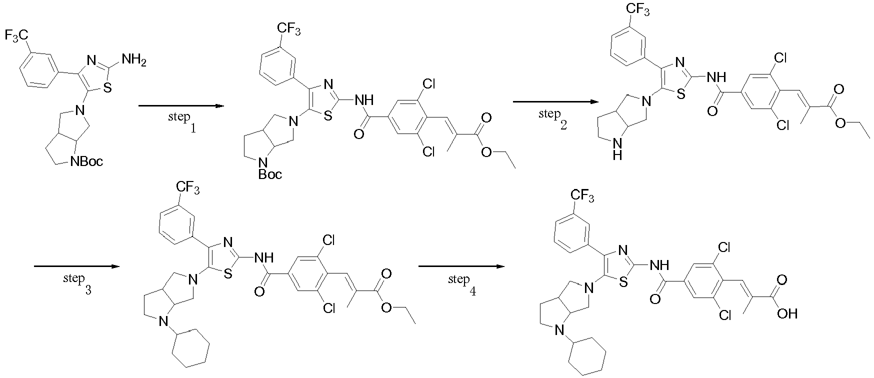

### Step 1: Synthesis of tert-butyl

### (E)-5-(2-(3,5-dichloro-4-(2-ethoxycarbonyl-propenyl)-benzoylamino)-4-(3-trifluorometh yl-phenyl)-thiazol-5-yl)-hexahydropyrrolo[3,4-b]pyrrole-1-carboxylate

tert-butyl 5-(2-amino-4-(3-trifluoromethylphenyl)-thiazol-5-yl)-hexahydropyrrolo[3,4-b]pyrrole-1-c arboxylate (110mg, 0.2mmol), 3,5-dichloro-4-(2-ethoxycarbonylpropenyl)-benzoic acid (145mg, 0.5mmol), diphenyl chloridophosphate (129mg, 0.5mmol), and triethylamine (73mg, 0.7mmol) were dissolved in chloroform (3.0mL). The mixture was reacted at 50°C for 3h. The reaction mixture was poured into 20ml water, and extracted with dichloromethane. The organic phases were combined, washed with a saturated aqueous sodium chloride solution, dried over anhydrous sodium sulfate, and filtered. The filtrate was evaporated under reduced pressure to remove the solvent to produce a crude product. The crude product was purified with silica gel column chromatography to produce the title compound (140mg).
ESI-MS (m/z): 739.2 [M + H]⁺

### Step 2: Synthesis of

### (E)-3-(2,6-dichloro-4-(5-(hexahydropyrrolo[3,4-b]pyrrol-5-yl)-4-(3-trifluoromethylphenyl) -thiazol-2-carbamoyl)-phenyl)-2-methyl-acrylic acid ethyl ester

The product from Step 1 (140mg, 0.2mmol) was dissolved in dichloromethane (2.0mL), and trifluoroacetic acid (1.0mL) was added dropwisely. The mixture was reacted at room temperature for 3 h. The reaction mixture was concentrated, adjusted with a saturated sodium bicarbonate to weak basicity (pH=8), and extracted with ethyl acetate. The organic phases were combined and washed with a saturated aqueous sodium chloride solution, dried over anhydrous sodium sulfate, and filtered. The filtrate was evaporated under reduced pressure to remove the solvent to produce the title compound (100mg).
ESI-MS (m/z): 639.1 [M + H]⁺

### Step 3: Synthesis of

### (E)-3-(2,6-dichloro-4-(5-(1-cyclohexyl-hexahydropyrrolo[3,4-b]pyrrol-5-yl)-4-(3-trifluoro methylphenyl)-thiazol-2-carbamoyl)-phenyl)-2-methyl-acrylic acid ethyl ester

The product from Step 2 (100mg, 0.2mmol) was dissolved in anhydrous methanol (2.0mL), and cyclohexanone (196mg, 2.0mmol), glacial acetic acid (18mg, 0.3mmol), and sodium triacetyloxyborohydride (212mg, 1.0mmol) were added. The mixture was reacted at 50°C for 16h. The reaction mixture was concentrated, dissolved in ethyl acetate (20mL), washed with a saturated aqueous sodium chloride solution, dried over anhydrous sodium sulfate, and filtered. The filtrate was evaporated under reduced pressure to remove the solvent to produce the title compound (110mg). The product was directly used in the next reaction without further purification.
ESI-MS (m/z): 721.2[M + H]⁺

### Step 4: Synthesis of

### (E)-3-(2,6-dichloro-4-(5-(1-cyclohexyl-hexahydropyrrolo[3,4-b]pyrrol-5-yl)-4-(3-trifluoro methylphenyl)-thiazol-2-carbamoyl)-phenyl)-2-methyl-acrylic acid

The product from Step 3 (110mg, 0.2mmol) was dissolved in a mixed solvent of tetrahydrofuran and water (3mL, V:V=2:1), and lithium hydroxide monohydrate (50mg, 1.2mmol) was added. The mixture was reacted at room temperature for 3 h. The reaction mixture was adjusted with a saturated citric acid solution to the acidity (pH=3), and extracted with a mixed solvent of ethyl acetate and tetrahydrofuran. The organic phases were combined and washed successively with water and a saturated aqueous sodium chloride solution, dried over anhydrous sodium sulfate, and filtered. The filtrate was concentrated to produce a crude title compound. The crude title compound was purified with HPLC using a trifluoroacetic acid system to produce a trifluoroacetate salt of the title compound (30mg).
¹H NMR (400 MHz, DMSO-d₆): δ 12.83 (s, 1H), 10.26 (s, 1H), 8.44 (d, J = 7.0 Hz, 1H), 8.27 (d, J = 8.9 Hz, 3H), 7.70 (d, J = 7.3 Hz, 2H), 7.40 (d, J = 1.5 Hz, 1H), 4.33 (d, J = 8.0 Hz, 1H), 3.69 (d, J = 10.6 Hz, 2H), 3.21-3.00 (m, 5H), 2.79 (dd, J = 9.6, 6.3 Hz, 1H), 2.35-2.25 (m, 1H), 2.15-2.05 (m, 2H), 1.91-1.78 (m, 3H), 1.68 (d, J = 1.4 Hz, 3H), 1.65-1.58 (m, 2H), 1.51-1.45 (m, 2H), 1.30-1.20 (m, 2H), 1.16-1.08 (m, 1H)
ESI-MS (m/z): 693.2 [M + H]⁺

### Example 36:

### (E)-3-(2,6-dichloro-4-((4-(4-chlorothiophen-2-yl)-5-(1-cyclohexyl-hexahydropyrrolo[3,4-b]pyrrol-5(1H)-yl)-thiazol-2-yl)-carbamoyl)-phenyl)-2-methylacrylic acid (K36)

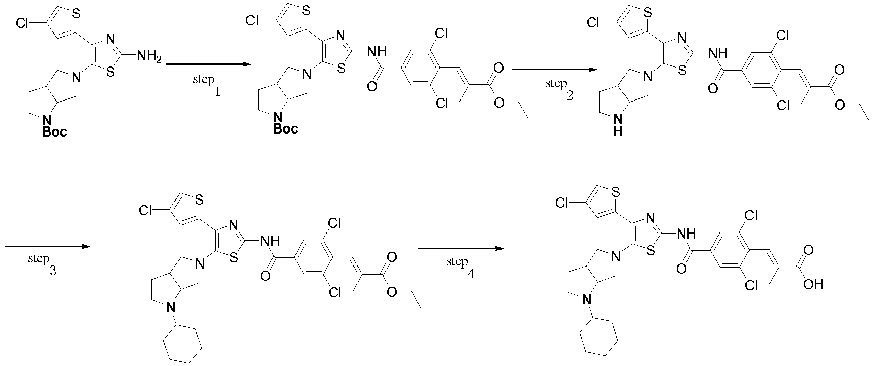

### Step 1: Synthesis of tert-butyl

### (E)-5-(4-(4-chlorothiophen-2-yl)-2-(3,5-dichloro-4-(3-ethoxy-2-methyl-3-oxo-propenyl)-benzoylamino)-thiazol-5-yl)-hexahydropyrrolo[3,4-b]pyrrole-1(2H)-carboxylate tert-butyl

5-(2-amino-4-(4-chlorothiophen-2-yl)-thiazol-5-yl)hexahydropyrrolo[3,4-b]pyrrole-1(2*H*) -carboxylate (639mg, 1.5mmol), (E)-3,5-dichloro-4-(3-ethoxy-2-methyl-3-oxo-1-propenyl)-benzoic acid (679mg, 2.2mmol), diphenyl chloridophosphate (603mg, 2.2mmol), and triethylamine (454mg, 4.5mmol) were added to chloroform (10mL). The mixture was warmed to 50°C and reacted for 2 h. The reaction mixture was poured into water, and extracted with dichloromethane. The organic phases were combined, successively washed with water and a saturated aqueous sodium chloride solution, dried over anhydrous sodium sulfate, and filtered. The filtrate was concentrated to produce a crude product. The crude product was purified with a silica gel column chromatography to produce the title compound (1.0g).
ESI-MS (m/z): 711.1 [M + H]⁺.

### Step 2: Synthesis of

### (E)-3-(2,6-dichloro-4-((5-(4-chlorothiophen-2-yl)-4-(hexahydropyrrolo[3,4-b]pyrrol-5(1H )-yl)-thiazol-2-yl)-carbamoyl)-phenyl)-2-methylacrylic acid ethyl ester

The product from Step 1(1.0g, 1.4mmol) was added to dichloromethane (2.0mL), and after being completely dissolved, trifluoroacetic acid (5mL) was added. The mixture was reacted at room temperature for 6 h. After removing the solvate by evaporation, the reaction mixture was adjusted with a saturated aquoues sodium bicarbonate solutionto weak basicity (pH=8), and extracted with dichloromethane. The organic phases were combined and washed successively with water and a saturated aqueous sodium chloride solution, dried over anhydrous sodium sulfate, and filtered. The filtrate was concentrated to produce the title compound (800mg). The crude product was directly used in the next reaction without further purification.
ESI-MS (m/z): 611.1 [M + H]⁺

### Step 3: Synthesis of

### (E)-3-(2,6-dichloro-4-((4-(4-chlorothiophen-2-yl)-5-(1-cyclohexyl-hexahydropyrrolo[3,4-b]pyrrol-5(1H)-yl)-thiazol-2-yl)-carbamoyl)-phenyl)-2-methylacrylic acid ethyl ester

The product from Step 2 (50mg, 0.08mmol), cyclohexanone (40mg, 0.4mmol), and glacial acetic acid (0.1mL) were dissolved in 1,4-dioxane (4mL), and sodium cyanoborohydride (26mg, 0.4mmol) was added. The mixture was warmed to 60°C and reacted for 1h. The reaction mixture was poured into water. After adding a saturated aqueous sodium bicarbonate solution, the resulting mixture was extracted with ethyl acetate. The organic phases were combined, washed successively with water and a saturated aqueous sodium chloride solution, dried over anhydrous sodium sulfate, and filtered. The filtrate was concentrated to produce the title compound (80mg), which was directly used in the next reaction.
ESI-MS (m/z): 693.1 [M + H]⁺

### Step 4: Synthesis of

### (E)-3-(2,6-dichloro-4-((4-(4-chlorothiophen-2-yl)-5-(1-cyclohexyl-hexahydropyrrolo[3,4-b]pyrrol-5(1H)-yl)-thiazol-2-yl)-carbamoyl)-phenyl)-2-methylacrylic acid

The product from Step 3 (80mg, 0.1mmol) was dissolved in a mixed solvent of tetrahydrofuran and water (3mL, V:V=2:1), and lithium hydroxide monohydrate (38mg, 0.9mmol) was added. The mixture was reacted at 40°C for 16h. The reaction mixture was adjusted with a saturated citric acid solution to the acidity (pH=3), and extracted with a mixed solvent of ethyl acetate and tetrahydrofuran. The organic phases were combined and washed successively with water and a saturated aqueous sodium chloride solution, dried over anhydrous sodium sulfate, and filtered. The filtrate was concentrated to produce a crude title compound. The crude title compound was purified with HPLC using a trifluoroacetic acid system to produce a trifluoroacetate salt of the title compound (20mg).
¹H NMR (400 MHz, DMSO-d₆): δ 12.91(s, 2H), 9.52(brs, 1H), 8.25(s, 2H), 7.60(d, J = 1.6 Hz, 1H), 7.54(d, J = 1.6 Hz, 1H), 7.40(d, J = 1.6 Hz, 1H), 4.36(d, J = 8.0 Hz, 1H), 3.78-2.51 (m, 13H), 2.50-1.09(m, 9H)
ESI-MS (m/z): 665.2[M + H]⁺

### Example 37:

### (E)-3-(2,6-dichloro-4-((4-(4-chlorothiophen-2-yl)-5-(1-cyclobutyl-hexahydropyrrolo[3,4-b]pyrrol-5(1H)-yl)-thiazol-2-yl)-carbamoyl)-phenyl)-2-methylacrylic acid (K37)

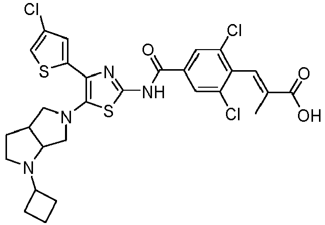

The procedure similar to that in Example 36, except that cyclohexanone was replaced with cyclobutanone in Step 3 of Example 36, was used to produce a trifluoroacetate salt of the title compound (20mg).
¹H NMR (400 MHz, DMSO-d₆): δ 12.91(s, 2H), 9.95(brs, 1H), 8.25(s, 2H), 7.60(d, J = 1.6 Hz, 1H), 7.51(d, J = 1.6 Hz, 1H), 7.40(d, J = 1.6 Hz, 1H), 4.14-2.51(m, 10H), 2.49-1.72(m, 7H)1.68(d, J = 1.6 Hz, 3H)
ESI-MS (m/z) 637.2[M + H]⁺

### Example 38:

### (E)-3-(4-((5-(1-(3,3-difluorocyclobutyl)-hexahydropyrrolo[3,4-b]pyrrol-5(1H)-yl)-4-(4-chl orothiophen-2-yl)-thiazol-2-yl)-carbamoyl)-2,6-dichlorophenyl)-2-methylacrylic acid (K38)

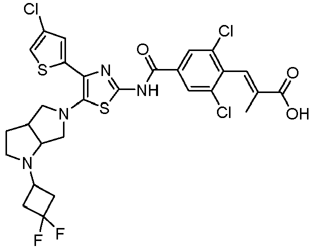

The procedure similar to that in Example 36, except that cyclohexanone was replaced with 3,3-difluorocyclobutanone in Step 3 of Example 36, was used to produce a trifluoroacetate salt of the title compound (10mg).
¹H NMR (400 MHz, DMSO-d₆) δ 12.99 (s, 1H), 12.88 (s, 1H), 11.57 (s, 1H), 8.25 (s, 2H), 7.58 (s, 1H), 7.55 (s, 1H), 7.39 (d, J = 1.6 Hz, 1H), 4.24 (s, 1H), 3.97 (s, 1H), 3.75-3.71 (m, 2H), 3.24-3.15 (m, 6H), 3.04-2.96 (m, 3H), 2.42-2.33 (m, 1H), 1.96 (s, 1H), 1.68 (d, J = 1.2 Hz, 3H)
ESI-MS (m/z): 673.0 [M + H]⁺

### Example 39:

### (E)-3-(4-((5-(1-(3-fluorocyclobutyl)-hexahydropyrrolo[3,4-b]pyrrol-5(1H)-yl)-4-(4-chlorot hiophen-2-yl)-thiazol-2-yl)-carbamoyl)-2,6-dichlorophenyl)-2-methylacrylic acid (K39)

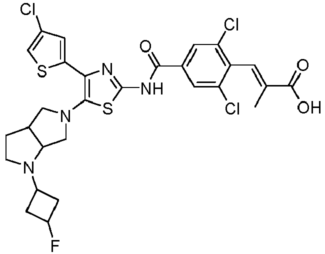

In the synthesis of Example 39, the procedure similar to that in Example 36, except that cyclohexanone was replaced with 3-fluorocyclobutanone in Step 3 of Example 36, and the purification using a trifluoroacetic acid system with a high performance liquid chromatography was replaced with the purification using a hydrochloric acid system with a high performance liquid chromatography in Step 4 of Example 36, was used to produce a hydrochloride salt of the title compound (11mg).
¹H NMR (400 MHz, DMSO-d₆) δ 13.01 (s, 1H), 12.88 (s, 1H), 10.14 (s, 1H), 8.25 (s, 2H), 7.58-7.56 (m, 2H), 7.39 (d, J = 1.2 Hz, 1H), 4.24-4.20 (s, 1H), 3.74-3.63 (m, 3H), 3.27-3.13 (m, 4H), 2.94-2.90 (m, 1H), 2.42-2.33 (m, 1H), 1.97-1.71 (m, 3H), 1.68 (d, J = 1.2 Hz, 3H), 1.14-1.08 (m, 3H)
ESI-MS (m/z): 655.2 [M + H]⁺

### Example 40: Synthesis of

### (E)-3-(2,6-dichloro-4-((4-(4-chlorothiophen-2-yl)-5-(1-cyclopentyl-hexahydropyrrolo[3,4 -b]pyrrol-5(1H)-yl)-thiazol-2-yl)-carbamoyl)-phenyl)-2-methylacrylic acid (K40)

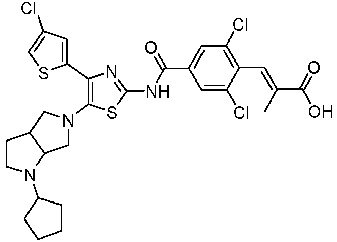

The procedure similar to that in Example 36, except that cyclohexanone was replaced with cyclopentanone in Step 3 of Example 36, was used to produce a trifluoroacetate salt of the title compound (20mg).
¹H NMR (400 MHz, DMSO-d₆) δ 12.90 (s, 2H), 9.73 (brs, 1H), 8.24 (s, 2H), 7.59 (d, J = 1.6 Hz, 1H), 7.51 (d, J = 1.6 Hz, 1H), 7.39 (d, J = 1.6 Hz, 1H), 4.31-2.87 (m, 12H), 2.66-1.56 (m, 10H)
ESI-MS (m/z) 651.2[M + H]⁺

### Example 41:

### (E)-3-(4-((5-(1-(3-methylcyclopentyl)-hexahydropyrrolo[3,4-b]pyrrol-5(1H)-yl)-4-(4-chlor othiophen-2-yl)-thiazol-2-yl)-carbamoyl)-2,6-dichlorophenyl)-2-methylacrylic acid (K41)

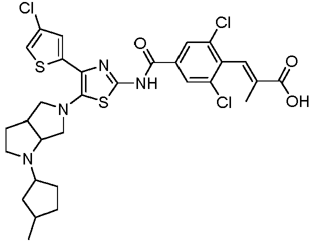

The procedure similar to that in Example 36, except that cyclohexanone was replaced with 3-methylcyclopentanone in Step 3 of Example 36, and the purification using a trifluoroacetic acid system with a high performance liquid chromatography was replaced with the purification using a hydrochloric acid system with a high performance liquid chromatography in Step 4 of Example 36, was used to produce a hydrochloride salt of the title compound (21mg).
¹H NMR (400 MHz, DMSO-d₆) δ 13.00 (s, 1H), 12.89 (s, 1H), 10.15 (s, 1H), 8.25 (s, 2H), 7.59 (s, 1H), 7.54 (s, 1H), 7.40 (s, 1H), 4.28 (s, 1H), 3.79-3.63 (m, 2H), 3.43-3.38 (m, 1H), 3.26-3.15 (m, 3H), 2.97-2.90 (m, 2H), 2.39-2.33 (m, 1H), 2.22-1.76 (m, 6H), 1.68 (s, 3H), 1.38-1.15 (m, 2H), 1.04-0.97 (m, 3H)
ESI-MS (m/z): 665.2 [M + H]⁺

### Example 42:

### (E)-3-(4-((5-(1-(N-methyl-piperidin-4-yl)-hexahydropyrrolo[3,4-b]pyrrol-5(1H)-yl)-4-(4-c hlorothiophen-2-yl)-thiazol-2-yl)-carbamoyl)-2,6-dichlorophenyl)-2-methylacrylic acid (K42)

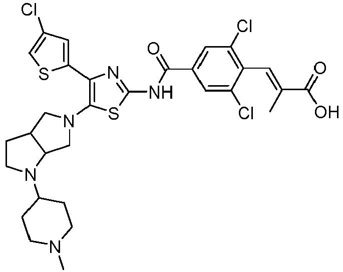

The procedure similar to that in Example 36, except that cyclohexanone was replaced with N-methyl-4-piperidinone in Step 3 of Example 36, and the purification using a trifluoroacetic acid system with a high performance liquid chromatography was replaced with the purification using a hydrochloric acid system with a high performance liquid chromatography in Step 4 of Example 36, was used to produce a hydrochloride salt of the title compound (30mg).
¹H NMR (400MHz, DMSO-d₆) δ 12.90(s, 1H), 11.11(s, 1H), 10.55(s, 1H), 8.25(s, 2H), 7.61-7.57(m, 2H), 7.39(d, J=1.2Hz, 1H), 4.39(s, 1H), 3.80-3.66(m, 2H), 3.55-3.36(m, 3H), 3.25-3.12(m, 4H), 3.00-2.91(m, 3H), 2.75-2.68(m, 3H), 2.37-1.92(m, 5H), 1.68(d, J=1.2Hz, 3H)
ESI-MS(m/z):680.2[M+H]⁺

### Example 43:

### (E)-3-(4-((5-(1-(4H-tetrahydropyran-4-yl)-hexahydropyrrolo[3,4-b]pyrrol-5(1H)-yl)-4-(4-chlorothiophen-2-yl)-thiazol-2-yl)-carbamoyl)-2,6-dichlorophenyl)-2-methylacrylic acid (K43)

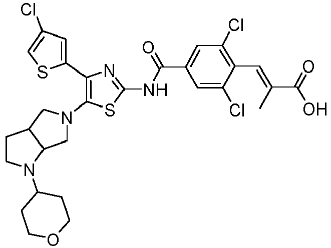

The procedure similar to that in Example 36, except that, cyclohexanone was replaced with 4-tetrahydropyrone in Step 3 of Example 36, and the purification using a trifluoroacetic acid system with a high performance liquid chromatography was replaced with the purification using a hydrochloric acid system with a high performance liquid chromatography in Step 4 of Example 36, was used to produce a hydrochloride salt of the title compound (25mg).
¹H NMR (400MHz, DMSO-d₆) δ 12.99(s, 1H), 12.89(s, 1H), 10.33(s, 1H), 8.25(s, 2H), 7.61-7.57(m, 2H), 7.39(d, J=1.2Hz, 1H), 4.40-4.35(m, 1H), 3.97(d, J=9.6Hz, 2H), 3.77-3.74(m, 1H), 3.65-3.61(m, 1H), 3.45-3.41(m, 2H), 3.35-3.16(m, 5H), 2.93-2.89(m, 1H), 2.42-2.33(m, 1H), 2.05-1.89(m, 3H), 1.82-1.73(m, 2H), 1.68(d, J=1.2Hz, 3H)
ESI-MS(m/z):667.2[M+H]⁺

### Example 44:

### (E)-3-(4-((5-(1-cycloheptyl-hexahydropyrrolo[3,4-b]pyrrol-5(1H)-yl)-4-(4-chlorothiophen -2-yl)-thiazol-2-yl)-carbamoyl)-2,6-dichlorophenyl)-2-methylacrylic acid (K44)

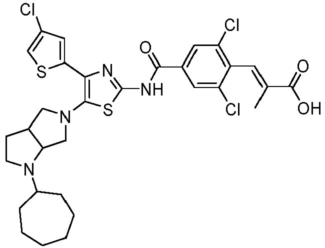

The procedure similar to that in Example 36, except that cyclohexanone was replaced with cycloheptanone in Step 3 of Example 36, the purification using a trifluoroacetic acid system with a high performance liquid chromatography was replaced with the purification using a hydrochloric acid system with a high performance liquid chromatography in Step 4 of Example 36, was used to produce a hydrochloride salt of the title compound (33mg).
¹H NMR (400MHz, DMSO-d₆) δ 12.89(s, 1H), 9.78(s, 1H), 8.25(s, 2H), 7.59-7.55(m, 2H), 7.40(d, J=1.2Hz, 1H), 4.37-4.31(m, 1H), 3.71-3.55(m, 2H), 3.45-3.34(m, 2H), 3.25-3.16(m, 3H), 2.94-2.90(m, 2H), 2.36-2.31(m, 1H), 2.13-2.07(m, 2H), 2.02-1.89(m, 1H), 1.75-1.65(m, 6H), 1.54-1.43(m, 6H)
ESI-MS(m/z):679.2[M+H]⁺

### Example 45:

### (E)-3-(4-((5-(1-(bicyclo[2.2.1]hept-2-yl)-hexahydropyrrolo[3,4-b]pyrrol-5(1H)-yl)-4-(4-ch lorothiophen-2-yl)-thiazol-2-yl)-carbamoyl)-2,6-dichlorophenyl)-2-methylacrylic acid (K45)

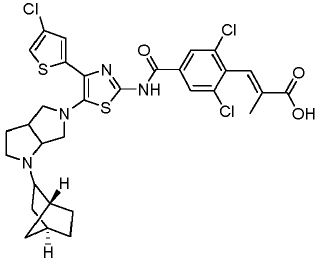

The procedure similar to that in Example 36, except that cyclohexanone was replaced with 2-bicyclo[2.2.1]heptanone in Step 3 of Example 36, the purification using a trifluoroacetic acid system with a high performance liquid chromatography was replaced with the purification using a hydrochloric acid system with a high performance liquid chromatography in Step 4 of Example 36, was used to produce a hydrochloride salt of the title compound (27mg).
¹H NMR (400MHz, DMSO-d₆) δ 12.88(s, 1H), 10.53-9.83(m, 1H), 8.25(s, 2H), 7.59-7.39(m, 3H), 4.54-4.29(m, 1H), 3.83-3.49(m, 4H), 3.37-3.10(m, 4H), 2.67-2.54(m, 1H), 2.42-2.22(m, 2H), 2.06-1.87(m, 2H), 1.76-1.66(m, 4H), 1.52-1.11(m, 6H) ESI-MS(m/z):677.2[M+H]⁺

### Example 46:

### (E)-3-(4-((5-(1-((1R,3R,5R,7R)-adamantan-2-yl)hexahydropyrrolo[3,4-b]pyrrol-5(1H)-yl )-4-(4-chlorothiophen-2-yl)thiazol-2-yl)carbamoyl)-2,6-dichlorophenyl)-2-methylacrylic acid (K46)

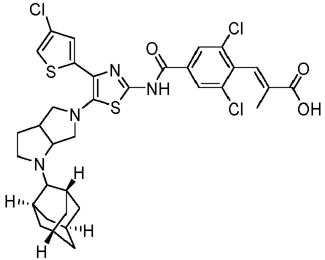

The procedure similar to that in Example 36, except that cyclohexanone was replaced with adamantanone in Step 3 of Example 36, was used to produce a trifluoroacetate salt of the title compound (30mg).
¹H NMR (400MHz, DMSO-d₆) δ 12.83(s, 1H), 12.41(s, 1H), 9.71(s, 1H), 8.24(s, 1H), 7.69-7.39(m, 3H), 4.56-4.23(s, 1H), 3.82-3.70(m, 1H), 3.56-3.38(m, 2H), 3.25-3.20(m, 2H), 3.13-2.81(m, 3H), 2.67-2.61(m, 1H), 2.37-2.18(m, 4H), 1.87-1.72(m, 7H), 1.68-1.67(m, 5H), 1.57-1.23(m, 2H)
ESI-MS(m/z):717.2[M+H]⁺

### Example 47:

### (E)-3-(2,6-dichloro-4-(4-(4-chlorothiophen-2-yl)-5-(5-cyclohexyl-2,5-diazabicyclo[2.2.2] octan-2-yl)-thiazol-2-carbamoyl)-phenyl)-2-methyl-acrylic acid (K47)

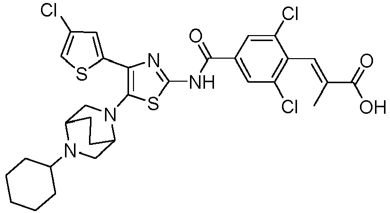

The procedure similar to that in Example 36, except that tert-butyl 5-(2-amino-4-(4-chlorothiophen-2-yl)-thiazol-5-yl)hexahydropyrrolo[3,4-b]pyrrole-1(*2H*) -carboxylate was replaced with tert-butyl
5-(2-amino-4-(4-chloro-thiophen-2-yl)-thiazol-5-yl)-2,5-diazabicyclo[2.2.2]octane-2-car boxylate (458mg, 1.1mmol) in Step 1 of Example 36, was used to produce a trifluoroacetate salt of the title compound (340mg).
¹H NMR (400MHz, DMSO-d₆): δ 12.91(d, J=5.6Hz, 1H), 10.26(s, 1H), 8.25(d, J=0.9Hz, 2H), 7.58(dd, J=4.4, 1.6Hz, 1H), 7.49(d, J=1.6Hz, 0.5H), 7.43(d, J=1.6Hz, 0.5H), 7.39(t, J=1.4Hz, 1H), 4.00(d, J=34.4Hz, 1H), 3.85-3.72(m, 2H), 3.64-3.56(m, 1H), 3.48-3.40(m, 2H), 3.25(d, J=12.6Hz, 1H), 2.36-2.18(m, 4H), 2.07-1.91(m, 1H), 1.85-1.79(m, 3H), 1.70-1.64(m, 4H), 1.55-1.48(m, 1H), 1.40-1.29(m, 3H), 1.24-1.14(m, 1H)
ESI-MS(m/z):665.2[M+H]⁺

### Example 48:

### (E)-3-(2,6-dichloro-4-((4-(4-chlorothiophen-2-yl)-5-(5-cyclohexylhexahydro-1H-pyrrolo[ 3,2-c]pyridin-2(3H)-yl)thiazol-2-yl)carbamoyl)phenyl)-2-methylacrylic acid (K48)

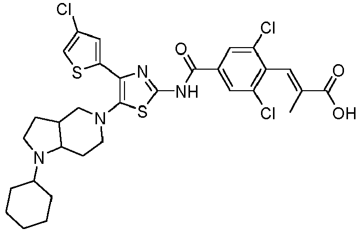

The procedure similar to that in Example 36, except that tert-butyl 5-(2-amino-4-(4-chlorothiophen-2-yl)-thiazol-5-yl)hexahydropyrrolo[3,4-b]pyrrole-1(2*H*) -carboxylate was replaced with tert-butyl
5-(2-amine-4-(4-chlorothiophen-2-yl)thiazol-5-yl)octahydro-1H-pyrrolo[3,2-c]piperidine-1-carboxylate (250mg, 0.6mmol) in Step 1 of Example 36, was used to produce a trifluoroacetate salt of the title compound (5mg).
¹H NMR (400MHz, DMSO-d₆) δ 12.87(s, 3H), 9.71-9.07(m, 3H), 8.23(s, 2H), 7.59-7.38(m, 3H), 3.98-1.06(m, 26H)
ESI-MS(m/z)679.1[M+H]⁺

### Example 49:

### 3'-chloro-5'-(5-(1-cyclohexyl-hexahydropyrrolo[3,4-b]pyrrol-5-yl)-4-(2-fluoro-3-trifluoromethylphenyl)-thiazol-2-carbamoyl)-3,4,5,6-tetrahydro-2H-[1,2']bipyridine-4-carboxylic acid (K49)

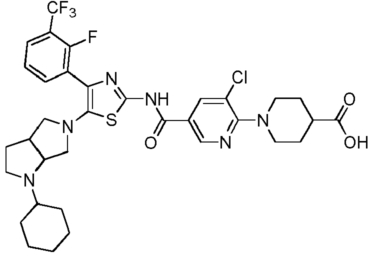

The procedure similar to that in Example 34, except that trifluoroacetophenone was replaced with 2-fluoro-3-trifluoromethylacetophenone (2.0g, 9.7mmol) in Step 1 of

Example 34, and the purification using a trifluoroacetic acid system with a high performance liquid chromatography was replaced with the purification using a hydrochloric acid system with a high performance liquid chromatography in Step 8 of Example 34, was used to produce a hydrochloride salt of the title compound (15mg).
¹H NMR (400MHz, DMSO-d₆): δ 12.61(s, 1H), 12.29(s, 1H), 9.55(s, 1H), 8.85(d, J=2.2Hz, 1H), 8.39(d, J=2.1Hz, 1H), 8.29(s, 1H), 7.75-7.67(m, 2H), 4.38-4.32(m, 1H), 3.98(d, J=13.1Hz, 2H), 3.71-3.57(m, 3H), 3.45-3.38(m, 1H), 3.30-3.18(m, 5H), 2.77-2.68(m, 1H), 2.55-2.48(m, 1H), 2.37-2.26(m, 1H), 2.18-2.03(m, 2H), 1.95-1.86(m, 2H), 1.80-1.71(m, 3H), 1.68-1.53(m, 3H), 1.44-1.12(m, 5H).
ESI-MS(m/z):721.2[M+H]⁺

### Example 50:

### 3-(2,6-dichloro-4-(5-(1-cyclohexyl-hexahydropyrrolo[3,4-b]pyrrol-5-yl)-4-(2-fluoro-3-trifluoromethylphenyl)-thiazol-2-carbamoyl)-phenyl)-2-methyl-acrylic acid (K50)

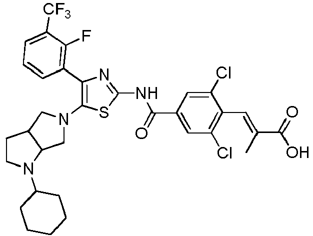

The procedure similar to that in Example 35, except that tert-butyl 5-(2-amino-4-(3-trifluoromethylphenyl)-thiazol-5-yl)-hexahydropyrrolo[3,4-b]pyrrole-1-c arboxylate was replaced with tert-butyl
5-(2-amino-4-(2-fluoro-3-trifluoromethylphenyl)-thiazol-5-yl)-hexahydropyrrolo[3,4-b]pyrrol-1-carboxylate (180mg, 0.4mmol) in Step 1 of Example 35, and the purification using a trifluoroacetic acid system with a high performance liquid chromatography was replaced with the purification using a hydrochloric acid system with a high performance liquid chromatography in Step 4 of Example 35, was used to produce a hydrochloride salt of the title compound (56mg).
¹H NMR (400MHz, DMSO-d₆): δ 12.83(s, 1H), 10.26(s, 1H), 8.27(d, J=8.9Hz, 3H), 7.70(d, J=7.3Hz, 1H), 7.40(d, J=1.5Hz, 1H), 7.30(s, 1H), 4.33(d, J=8.0Hz, 1H), 3.69(d, J=10.6Hz, 2H), 3.21-3.00(m, 5H), 2.79(dd, J=9.6, 6.3Hz, 1H), 2.35-2.25(m, 1H), 2.15-2.05(m, 2H), 1.91-1.78(m, 3H), 1.68(d, J=1.4Hz, 3H), 1.65-1.58(m, 2H), 1.51-1.45(m, 2H), 1.30-1.20(m, 2H), 1.16-1.08(m, 1H).
ESI-MS(m/z):711.2[M+H]⁺

### Example 51:

### (E)-3-(4-((5-((3aR,6aR)-1-((1R,3R,5R,7R)-adamantan-2-yl)hexahydropyrrolo[3,4-b]pyrrol-5(1H)-yl)-4-(4-chlorothiophen-2-yl)thiazol-2-yl)carbamoyl)-2,6-dichlorophenyl)-2-methylacrylic acid (K51)

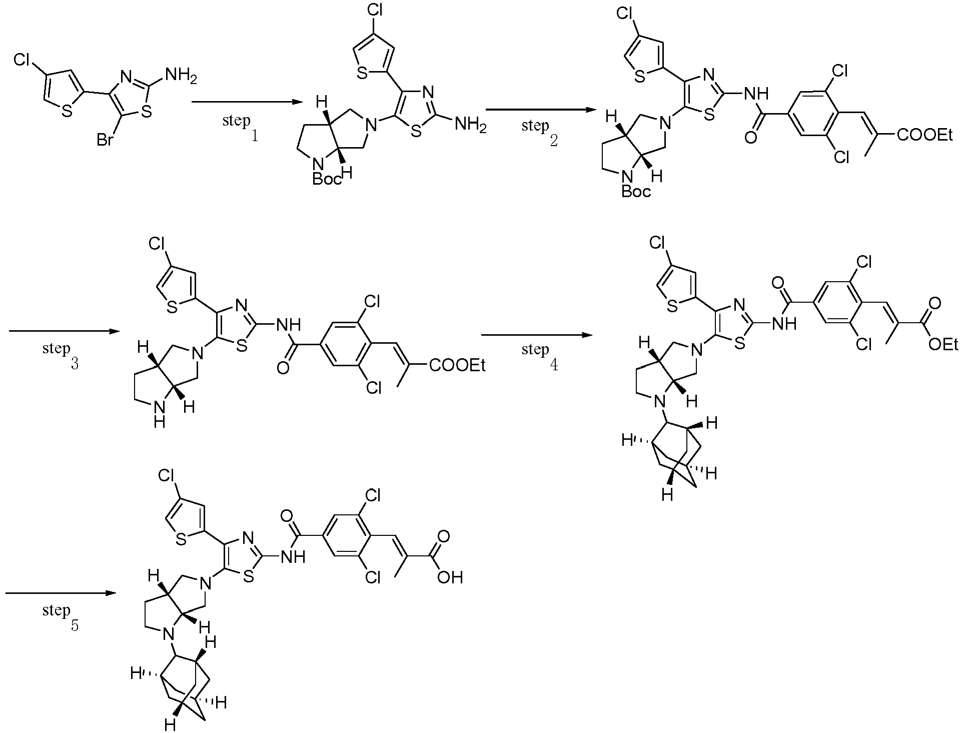

### Step 1: Synthesis of tert-butyl

### (3aR,6aR)-5-(2-amino-4-(4-chlorothiophen-2-yl)-thiazol-5-yl)-hexahydropyrrolo[3,4-b]p yrrole-1(2H)-carboxylate

5-bromo-4-(4-chlorothiophen-2-yl)-2-amino-thiazole (1.4g, 4.7mmol), and tert-butyl (3aR,6aR)-hexahydropyrrolo[3,4-b]pyrrole-1(2*H*)-carboxylate (1.1g, 4.7mmol) were dissolved in N,N-dimethylformamide (30mL), and anhydrous potassium carbonate (820mg, 5.6mmol) was added. The mixture was reacted at 70°C for 1h. The solvent was removed under a reduced pressure. The residue was purified with a silica gel column chromatography to produce the title compound (1.7g).
ESI-MS(m/z):427.2[M+H]⁺

### Step 2: Synthesis of tert-butyl

### (E)-(3aR,6aR)-5-(4-(4-chlorothiophen-2-yl)-2-(3,5-dichloro-4-(3-ethoxy-2-methyl-3-oxo -1-propenyl)-benzoylamino)thiazol-5-yl)hexahydropyrrolo[3,4-b]pyrrole-1(2H)-carboxylate

The product from Step 1 (830mg, 1.9mmol), 3,5-dichloro-4-(3-ethoxy-2-methyl-3-oxo-1-propenyl)-benzoic acid (880mg, 2.9mmol) were dissolved in chloroform (30mL), and then diphenyl chloridophosphate (782mg, 2.9mmol), and N,N-diisopropylethylamine (370mg, 2.9mmol) were successively added. The mixture was reacted at 50°C for 4h. The reaction system was cooled down to room temperature, successively washed with water, an aqueous sodium bicarbonate solution, and a saturated aqueous sodium chloride solution, dried over anhydrous sodium sulfate, and filtered. The solvent was removed under a reduced pressure. The residue was purified with a silica gel column chromatography to produce the title compound (578mg).
ESI-MS(m/z):711.2[M+H]⁺

### Step 3: Synthesis of

### (E)-(3aR,6aR)-3-(2,6-dichloro-4-((4-(4-chlorothiophen-2-yl)-5-(hexahydropyrrolo[3,4-b] pyrrol-5(1H)-yl)-thiazol-2-yl)-carbamoyl)-phenyl)-2-methylacrylic acid ethyl ester

The product from Step 2 (71mg, 0.1mmol) was dissolved in anhydrous dichloromethane (1.5mL), and then trifluoroacetic acid (0.5mL) was added dropwisely. The mixture was reacted at room temperature for 1.5h. The solvent was removed by evaporation under reduced pressure to produce the title compound, which was directly used in the next reaction without purification.
ESI-MS(m/z):611.2[M+H]⁺

### Step 4: Synthesis of

### (E)-3-(4-((5-((3aR,6aR)-1-((1R,3R,5R,7R)-adamantan-2-yl)hexahydropyrrolo[3,4-b]pyr rol-5(1H)-yl)-4-(4-chlorothiophen-2-yl)-thiazol-2-yl)-carbamoyl)-2,6-dichlorophenyl)-2-methylacrylic acid ethyl ester

The product from Step 3 was dissolved in 1,4-dioxane (5mL), and glacial acetic acid (0.1mL), adamantanone (75mg, 0.5mmol), and sodium cyanoborohydride (32mg, 0.5mmol) were added. The mixture was reacted at 50°C for 2h, and then filtered. The solvent was removed by evaporation under reduced pressure to produce the title compound, which was directly used in the next reaction without purification.
ESI-MS(m/z):745.2[M+H]⁺

### Step 5: Synthesis of

### (E)-3-(4-((5-((3aR,6aR)-1-((1R,3R,5R,7R)-adamantan-2-yl)hexahydropyrrolo[3,4-b]pyr rol-5(1H)-yl)-4-(4-chlorothiophen-2-yl)-thiazol-2-yl)-carbamoyl)-2,6-dichlorophenyl)-2-methylacrylic acid

The product from Step 4 was dissolved in a mixed solvent of tetrahydrofuran and water (3mL, V:V=2:1), and lithium hydroxide monohydrate (25mg, 0.6mmol) was added. The mixture was reacted at room temperature for 3.5h, and adjusted with hydrochloric acid to pH=2. The solvent was removed under a reduced pressure, and purified by using a hydrochloric acid system with a high performance liquid chromatography to produce a hydrochloride salt of the title compound (300mg).
¹H NMR (400MHz, DMSO-d₆) δ 12.88(s, 2H), 8.23(s, 1H), 7.68(s, 1H), 7.49(s, 1H), 7.39(s, 1H), 3.24(d, J=8.64Hz, 2H), 3.08-3.03(m, 2H), 2.94(t, J=6.36Hz, 1H), 2.88-2.81(m, 1H), 2.63-2.59(m, 1H), 2.31-2.28(m, 2H), 2.19(d, J=11.36Hz, 2H), 2.04(s, 1H), 1.96(s, 1H), 1.77-1.66(m, 13H), 1.44(d, J=11.20Hz, 1H), 1.30(d, J=11.04Hz, 1H)
ESI-MS(m/z):717.2[M+H]⁺

### Example 52:

### (E)-3-(4-((5-((3aS,6aS)-1-((1S,3S,5S,7S)-adamantan-2-yl)hexahydropyrrolo[3,4-b]pyrr ol-5(1H)-yl)-4-(4-chlorothiophen-2-yl)thiazol-2-yl)carbamoyl)-2,6-dichlorophenyl)-2-met hylacrylic acid (K52)

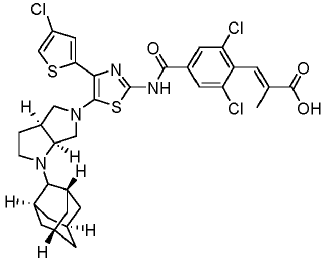

The procedure similar to that in Example 51, except that tert-butyl (3aR,6aR)-hexahydropyrrolo[3,4-b]pyrrole-1(2*H*)-carboxylate was replaced with tert-butyl (3aS,6aS)-hexahydropyrrolo[3,4-b]pyrrole-1(*2H*)-carboxylate, was used to produce a hydrochloride salt of the title compound (300mg) .
¹H NMR (400MHz, DMSO-d₆) δ 12.88(s, 2H), 8.23(s, 1H), 7.68(s, 1H), 7.49(s, 1H), 7.39(s, 1H), 3.24(d, J=8.64Hz, 2H), 3.08-3.03(m, 2H), 2.94(t, J=6.36Hz, 1H), 2.88-2.81(m, 1H), 2.63-2.59(m, 1H), 2.31-2.28(m, 2H), 2.19(d, J=11.36Hz, 2H), 2.04(s, 1H), 1.96(s, 1H), 1.77-1.66(m, 13H), 1.44(d, J=11.20Hz, 1H), 1.30(d, J=11.04Hz, 1H)
ESI-MS(m/z):717.2[M+H]⁺

### Biological Assay

### In vitro activity test

Thrombopoietin is a glycoprotein associated with platelet production, and plays a key role in regulating megakaryocyte production and platelet production by bone marrow megakaryocytes.

The TPO mimetic compound was synthesized in vitro, and the compound acted on the TPO receptor (TPOR) on the cell to stimulate cell proliferation and differentiation. The OD490 value was detected by the MTS method, and the more the number of cells, the larger the OD value, thereby detecting the response of the compound to cell proliferation and differentiation. The agonist concentration at which the increased signal arrived at the highest level was Emax, and the concentration of the compound at which the increased signal arrived at 50% of the signal of Emax was EC₅₀. The activity of the compound was determinable by EC₅₀, and the smaller the EC₅₀, the higher the activity of the compound.

Mouse primary B cells BAF3 stably expressing human TPOR were cultured in 1640 medium containing 10% FBS. On the day of the assay, the cells were counted and seeded in a 96-well plate at 1×10⁴ cells/50µL. 50 µL of different concentrations of the tested compound were added to the wells to a final concentration of 10000nM, 3000nM, 300nM, 30nM, 3nM, 0.3nM, 0.03nM, 0.003nM, 0.0003nM, and the final concentration of DMSO was 1%. After the cells were cultured with the compound for 24h at 37°C in a 5% CO₂ incubator, 10µL of detection reagent (Promega, Cell Titer 96® Aqueous One Solution) was added. After mixing, the resulting mixture was incubated for 3h in the incubator. The OD 490 was detected using a multifunctional automatic microplate reader, and the EC₅₀ was fitted using GraphPad Prism 5 software.

**Table 1: Effect of the compound on the proliferation of BAF3/TPOR cells**

| No. | EC₅₀ (nM) |
|---|---|
| Lusutrombopag | 1.93 |
| Example 2 | 0.21 |
| Example 5 | 0.08 |
| Example 8 | 1.11 |
| Example 9 | 0.21 |
| Example 10 | 0.10 |
| Example 29 | 0.89 |
| Example 35 | 0.57 |
| Example 36 | 0.10 |
| Example 37 | 0.34 |
| Example 38 | 0.43 |
| Example 40 | 0.07 |
| Example 44 | 0.13 |
| Example 45 | 0.59 |
| Example 46 | 0.08 |
| Example 48 | 0.60 |
| Example 51 | 0.05 |
| Example 52 | 0.13 |

The data in Table 1 demonstrated that the above compounds of the present invention had lower EC₅₀ values compared to Lusutrombopag, showing better BAF3/TPOR cell proliferation and better thrombopoietin receptor agonistic activity.

Other compounds of the invention had BAF3/TPOR cell proliferation effects similar to those described above.

### hERG test

In cardiomyocytes, the potassium channel encoded by hERG (human Ether-a-go-go Related Gene) mediated the delayed rectifier potassium current (IKr). The IKr inhibition was the most important mechanism for drug-induced QT prolongation and induction of arrhythmia. In the hERG test, the criterion was that if the compound had an IC₅₀ of >10µM, the compound was judged to have no inhibitory effect on hERG.

The electrophysiological manual patch clamp was used to detect the effect of the tested compound on the hERG potassium channel. The test results were shown in Table 2 below:

**Table 2**

| Compound | IC₅₀ (µM) |
|---|---|
| Lusutrombopag | 2.37 |
| Example 51 | >30 |
| Example 52 | 7.90 |

The test results in Table 2 indicated that lusutrombopag had significant cardiac hERG potassium channel inhibition and had the potential risk of inducing arrhythmia. The above compounds of the present invention, especially the compound of Example 51, had no hERG inhibition and possessed higher safety.

### Industrial applicability

The compound of the present invention exhibits a good effect on drug safety when applied as medicament for thrombopoietin receptor mediated diseases, and exhibits good bioactivity and metabolic advantage in the body on pharmacodynamics or pharmacokinetics in vivo or in vitro.

## Claims

1. A compound represented by formula (I), or an isomer or racemate thereof, a salt thereof, an ester thereof, a solvate thereof, a chemically protected form thereof, a prodrug or metabolite thereof, a crystal form thereof, and a mixture thereof: 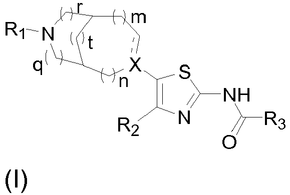
wherein m, n, q, and r are individually and separately selected from an integer of 0-4;
t is selected from an integer of 0-3;
X is N or C;
-̅-̅-̅ represents a single or double bond; when X is N, -̅-̅-̅ is a single bond;
R₁ is selected from hydrogen, optionally substituted C₁-C₁₂alkyl, optionally substituted C₂-C₁₂alkenyl, optionally substituted C₂-C₁₂alkynyl, optionally substituted C₃-C₁₂cycloalkyl, optionally substituted C₅-C₁₂cycloalkenyl, optionally substituted C₅-C₁₂polycycliccycloalkyl, optionally substituted C₆-C₁₂polycycliccycloalkenyl, optionally substituted C₄-C₁₂fused cycloalkyl, optionally substituted C₆-C₁₂fused cycloalkenyl, optionally substituted C₆-C₁₀aryl, optionally substituted C₃-C₁₀heterocyclyl, wherein the above "optionally substituted" in the definition of R₁ refers to being unsubstituted or substituted by one or more identical or different groups selected from : C₁-C₆alkyl, C₁-C₆alkyl substituted by one or more halogens, cyano, halogen, C₁-C₆alkoxy or C₁-C₆alkoxy substituted by one or more halogens;
R₂ is selected from C₆-C₁₀aryl optionally substituted by R₄, 5- or 6-membered heteroaryl optionally substituted by R₄ and containing 1-3 identical or different heteroatoms selected from N, O and S, 8- to 10-membered heteroaryl optionally substituted by R₄ and containing 1-4 identical or different heteroatoms selected from N, O and S;
R₄ is selected from hydrogen, substituted or unsubstituted C₁-C₁₂alkyl, substituted or unsubstituted C₃-C₁₂cycloalkyl, halogen, cyano, nitro, substituted or unsubstituted C₁-C₁₂alkoxy, substituted or unsubstituted C₂-C₁₂alkoxyalkyl, carboxyl, carboxyl-substituted C₂-C₆alkenyl, ester group, ester group-substituted C₂-C₁₂alkenyl, R₅R₆N-, (C₁-C₁₂alkyl) C(=O)N(R₅)-, R₅R₆NC(=O)-, R₅SO, R₅SO₂, R₅R₆NSO₂, wherein R₅ and R₆ are each independently selected from hydrogen, C₁-C₁₂alkyl and C₃-C₁₂cycloalkyl;
R₃ is selected from an aryl or heteroaryl represented by formula (II), or, R₃ is selected from a heteroaryl represented by formula (III): 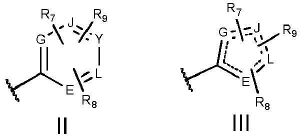 wherein J, L, G, E and Y are each independently selected from N, O, S, CH or C, wherein R₇, R₈ and R₉ are each independently selected from hydrogen, halogen, OH, cyano, nitro, carboxyl, ester group, substituted or unsubstituted C₃-C₁₀cycloalkyl, substituted or unsubstituted C₁-C₄alkyl, substituted or unsubstituted C₂-C₄alkenyl, C₁-C₄alkoxy, R₅R₆N, substituted or unsubstituted 4-8 membered saturated heterocyclyl containing at least one atom selected from N, O, S and S(O)ₑ, e is 1 or 2;
said "substituted" refers to being substituted by one or more identical or different groups selected from : C₁-C₆alkyl, cyano, halogen, carboxyl, ester, phosphoric acid group, phosphate ester group.

2. The compound according to Claim 1, or an isomer or racemate thereof, a salt thereof, an ester thereof, a solvate thereof, a chemically protected form thereof, a prodrug or metabolite thereof, a crystal form thereof, and a mixture thereof, wherein said compound has a structure represented by formula (I'): 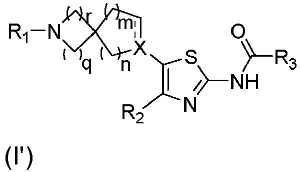 wherein m, n, q, and r are individually and separately selected from an integer of 0-4; R₁, R₂ and R₃ are defined as in claim 1.

3. The compound according to Claim 1 or 2, or an isomer or racemate thereof, a salt thereof, an ester thereof, a solvate thereof, a chemically protected form thereof, a prodrug or metabolite thereof, a crystal form thereof, and a mixture thereof, wherein X is N, and -̅-̅-̅ is a single bond.

4. The compound according to Claim 1 or 3, or an isomer or racemate thereof, a salt thereof, an ester thereof, a solvate thereof, a chemically protected form thereof, a prodrug or metabolite thereof, a crystal form thereof, and a mixture thereof, wherein said group 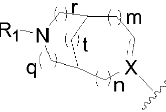 is as shown in formula (IV): 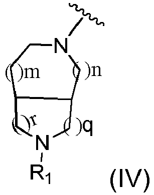 wherein m, n, q, and r are individually and separately selected from an integer of 0-4; R₁ are defined as in claim 1.

5. The compound according to Claim 1 or 3 or an isomer or racemate thereof, a salt thereof, an ester thereof, a solvate thereof, a chemically protected form thereof, a prodrug or metabolite thereof, a crystal form thereof, and a mixture thereof, wherein the group 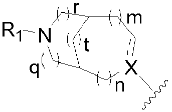 is as shown in formula (V): 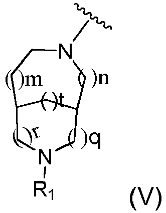 wherein m, n, q, and r are individually and separately selected from an integer of 0-4; t is an integer of 1-3; R₁ are defined as in claim 1.

6. The compound according to any of Claims 1-5, or an isomer or racemate thereof, a salt thereof, an ester thereof, a solvate thereof, a chemically protected form thereof, a prodrug or metabolite thereof, a crystal form thereof, and a mixture thereof,
wherein R₁ is selected from
substituted or unsubstituted C₃-C₁₀alkyl, substituted or unsubstituted C₃-C₁₀alkenyl, substituted or unsubstituted C₃-C₁₀cycloalkyl, substituted or unsubstituted C₅-C₁₀cycloalkenyl, substituted or unsubstituted C₅-C₁₂polycycliccycloalkyl, substituted or unsubstituted C₆-C₁₂polycycliccycloalkenyl, substituted or unsubstituted C₄-C₁₂fused cycloalkyl, substituted or unsubstituted C₆-C₁₂fused cycloalkenyl, substituted or unsubstituted 4-8 membered saturated heterocyclyl containing at least one heteroatom selected from R₇N, O and S, substituted or unsubstituted 5-membered or 6-membered or 8-membered to 10-membered heteroaryl containing 1-4 identical or different heteroatoms selected from N, O and S.

7. The compound according to any of Claims 1-5, or an isomer or racemate thereof, a salt thereof, an ester thereof, a solvate thereof, a chemically protected form thereof, a prodrug or metabolite thereof, a crystal form thereof, and a mixture thereof,
wherein R₁ is selected from
substituted or unsubstituted C₁-C₁₀alkyl, substituted or unsubstituted C₃-C₁₀alkenyl, substituted or unsubstituted C₃-C₁₀cycloalkyl, substituted or unsubstituted C₅-C₁₀cycloalkenyl, substituted or unsubstituted C₅-C₁₂polycycliccycloalkyl, substituted or unsubstituted C₆-C₁₂polycycliccycloalkenyl, substituted or unsubstituted C₄-C₁₂fused cycloalkyl, substituted or unsubstituted C₆-C₁₂fused cycloalkenyl, substituted or unsubstituted 4-8 membered saturated heterocyclyl containing at least one group selected from R₁₀N, O and S, substituted or unsubstituted 5-membered or 6-membered or 8-membered to 10-membered heteroaryl containing 1-4 identical or different heteroatoms selected from N, O and S, R₁₀ can be selected from hydrogen, C₁-C₁₂alkyl or C₃-C₁₂cycloalkyl.

8. The compound according to any of Claims 1-7 or an isomer or racemate thereof, a salt thereof, an ester thereof, a solvate thereof, a chemically protected form thereof, a prodrug or metabolite thereof, a crystal form thereof, and a mixture thereof,
wherein R₁ is selected from methyl, ethyl, propyl, iso-propyl, butyl, iso-butyl, tert-butyl, cyclopropyl, cyclobutyl, cyclopentyl, cyclohexyl, 2, 2, 2-trifluoroethyl,

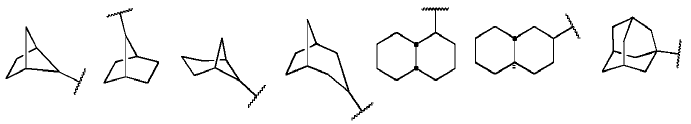

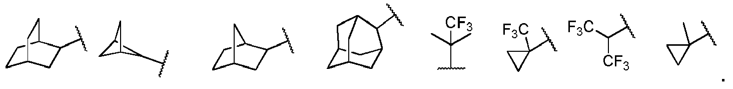

9. The compound according to any of Claims 1-7, or an isomer or racemate thereof, a salt thereof, an ester thereof, a solvate thereof, a chemically protected form thereof, a prodrug or metabolite thereof, a crystal form thereof, and a mixture thereof,
wherein R₁ is selected from cycloheptyl, piperidinyl, methylpiperidinyl, tetrahydropyranyl, methylcyclopentyl, pyrrolyl,

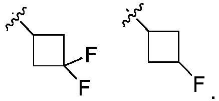

10. The compound according to any of Claims 1-9, or an isomer or racemate thereof, a salt thereof, an ester thereof, a solvate or hydrate thereof, a chemically protected form thereof, a prodrug or metabolite thereof, a crystal form thereof, and a mixture thereof wherein R₁ is selected from methyl, ethyl, cyclopropyl, iso-propyl, butyl, iso-butyl, tert-butyl, cyclopropyl, cyclobutyl, cyclopentyl, cyclohexyl or

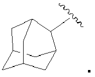

11. The compound according to any of Claims 1-10 or an isomer or racemate thereof, a salt thereof, an ester thereof, a solvate thereof, a chemically protected form thereof, a prodrug or metabolite thereof, a crystal form thereof, and a mixture thereof, wherein, R₄ is selected from hydrogen, halogen, cyano, nitro, C₁-C₄alkyl, C₁-C₄alkoxy, C₃-C₁₂cycloalkyl, C₁-C₄alkyl substituted by one or more halogens, C₁-C₄alkoxy substituted by one or more halogens, C₂-C₁₂alkoxyalkyl, alkoxyalkyl substituted by C₃-C₁₂cycloalkyl.

12. The compound according to any of Claims 1-11 or an isomer or racemate thereof, a salt thereof, an ester thereof, a solvate thereof, a chemically protected form thereof, a prodrug or metabolite thereof, a crystal form thereof, and a mixture thereof, wherein, R₂ is selected from the following groups optionally substituted by R₄: phenyl, naphthyl, furyl, thienyl, pyrrolyl, oxazolyl, thiazolyl, pyridinyl, pyrimidinyl, pyridazinyl, pyrazinyl, indolyl, quinolinyl or purinyl;
R₄ is selected from hydrogen, Cl, F, methyl or CF₃.

13. The compound according to any of Claims 1-12 or an isomer or racemate thereof, a salt thereof, an ester thereof, a solvate thereof, a chemically protected form thereof, a prodrug or metabolite thereof, a crystal form thereof, and a mixture thereof, wherein, R₂ is selected from

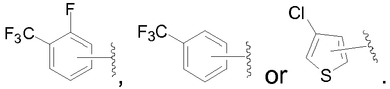

14. The compound according to any of Claims 1-13 or an isomer or racemate thereof, a salt thereof, an ester thereof, a solvate thereof, a chemically protected form thereof, a prodrug or metabolite thereof, a crystal form thereof, and a mixture thereof, wherein, R₂ is selected from

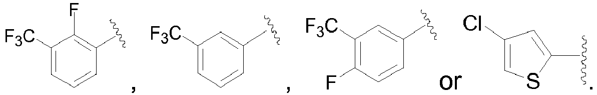

15. The compound according to any of Claims 1-14 or an isomer or racemate thereof, a salt thereof, an ester thereof, a solvate thereof, a chemically protected form thereof, a prodrug or metabolite thereof, a crystal form thereof, and a mixture thereof,
wherein R₃ is selected from formula VI, formula VII or formula VIII 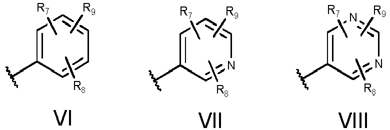 wherein R₇, R₈ and R₉ are each independently selected from hydrogen, halogen, OH, cyano, nitro, carboxyl, ester group, C₃-C₆cycloalkyl, C₁-C₄alkyl substituted by more than one halogen, substituted or unsubstituted C₂-C₄alkenyl, C₁-C₄alkoxy substituted by more than one halogen, R₅R₆N, substituted or unsubstituted 4-8 membered saturated heterocyclyl containing at least one atom selected from N, O, S and S(O)ₑ, e is 1 or 2.

16. The compound according to any of Claims 1-15, or an isomer or racemate thereof, a salt thereof, an ester thereof, a solvate thereof, a chemically protected form thereof, a prodrug or metabolite thereof, a crystal form thereof, and a mixture thereof, wherein, R₃ is selected from 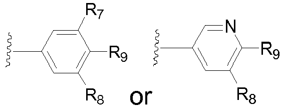 wherein R₇, R₈ are each independently selected from methyl, ethyl, CF₃, Cl, Br, F, cyclopropyl; R₉ is selected from carboxyl, ester group, OH, NH₂, halogen, C₁-C₁₀alkyl containing a substituent of carboxyl or ester group, C₂-C₄alkenyl containing a substituent of carboxyl or ester group, C₁-C₁₀alkoxy containing a substituent of carboxyl or ester group, C₁-C₁₀alkylamino containing a substituent of carboxyl or ester group, C₁-C₁₀alkylthio containing a substituent of carboxyl or ester group, C₄-C₁₀heterocyclyl containing a substituent of carboxyl or ester group.

17. The compound according to any of Claims 1-16 or an isomer or racemate thereof, a salt thereof, an ester thereof, a solvate thereof, a chemically protected form thereof, a prodrug or metabolite thereof, a crystal form thereof, and a mixture thereof, wherein, R₃ is selected from

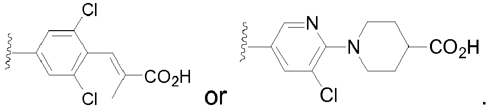

18. A compound, which is selected from:
1-(3-chloro-5-{[4-(4-chlorothiophen-2-yl)-5-(1-methyl-hexahydropyrrolo[3,4-b]pyrrol-5(*1H*)-yl)thiazol-2-yl]carbamoyl}pyridin-2-yl)piperidine-4-carboxylic acid;
1-(3-chloro-5-{[4-(4-chlorothiophen-2-yl)-5-(1-cyclobutyl-hexahydropyrrolo[3,4-b]-pyrrol-5(*1H*)-yl)-thiazol-2-yl]-carbamoyl}-pyridin-2-yl)-piperidine-4-carboxylic acid;
1-(3-chloro-5-{[4-(4-chlorothiophen-2-yl)-5-(1-cyclopentyl-hexahydropyrrolo[3,4-b]pyrrol-5(*1H*)-yl)-thiazol-2-yl]-carbamoyl}-pyridin-2-yl)-piperidine-4-carboxylic acid;
1-[3-chloro-5-({4-(4-chlorothiophen-2-yl)-5-[1-(3-methylcyclopentyl)-hexahydropyrrolo[3,4-b]pyrrol-5(*1H*)-yl]-thiazol-2-yl}-carbamoyl)-pyridin-2-yl]-piperidine-4-carboxylic acid;
1-(3-chloro-5-{[4-(4-chlorothiophen-2-yl)-5-(1-cyclohexyl-hexahydropyrrolo[3,4-b]pyrrol-5(*1H*)-yl)-thiazol-2-yl]-carbamoyl}-pyridin-2-yl)-piperidine-4-carboxylic acid and a stereoisomer thereof;
1-[3-chloro-5-({4-(4-chlorothiophen-2-yl)-5-[1-(N-methyl-piperidin-4-yl)-hexahydropyrrolo[3,4-b]pyrrol-5(*1H*)-yl]-thiazol-2-yl}-carbamoyl)-pyridin-2-yl]-piperidine-4-carboxylic acid;
1-[3-chloro-5-({4-(4-chlorothiophen-2-yl)-5-[1-(4H-tetrahydropyran-4-yl)-hexahydropyrrolo[3,4-b]pyrrol-5(*1H*)-yl]-thiazol-2-yl}-carbamoyl)-pyridin-2-yl]-piperidine-4-carboxylic acid;
1-(3-chloro-5-{[4-(4-chlorothiophen-2-yl)-5-(1-cycloheptyl-hexahydropyrrolo[3,4-b]pyrrol-5(*1H*)-yl)-thiazol-2-yl]-carbamoyl}-pyridin-2-yl)-piperidine-4-carboxylic acid;
1-[3-chloro-5-({4-(4-chlorothiophen-2-yl)-5-[1-(bicyclo[2.2.1]hept-2-yl)-hexahydropyrrolo[3,4-b]pyrrol-5(*1H*)-yl]thiazol-2-yl}carbamoyl)pyridin-2-yl]piperidine-4-carboxylic acid;
1-(3-chloro-5-{[4-(4-chlorothiophen-2-yl)-5-(1-(1R,3R,5R,7R)-adamantanyl-hexahydropyrrolo[3,4-b]pyrrol-5(*1H*)-yl)-thiazol-2-yl]-carbamoyl}-pyridin-2-yl)-piperidine-4-carboxylic acid;
1-(3-chloro-5-{[4-(4-chlorothiophen-2-yl)-5-(1-cyclopentyl-octahydropyrrolo[3,4-b]pyridin-6(*1H*)-yl)-thiazol-2-yl]-carbamoyl}-pyridin-2-yl)-piperidine-4-carboxylic acid;
1-(3-chloro-5-{[4-(4-chlorothiophen-2-yl)-5-(1-cyclohexyl-octahydropyrrolo[3,4-b]pyridin-6(*1H*)-yl)-thiazol-2-yl]-carbamoyl}-pyridin-2-yl)-piperidine-4-carboxylic acid;
1-(3-chloro-5-{[4-(4-chlorothiophen-2-yl)-5-(1-cyclohexyl-octahydro-*1H*-pyrrolo[3,2-c]pyridin-5(6*H*)-yl)-thiazol-2-yl]-carbamoyl}-pyridin-2-yl)-piperidine-4-carboxylic acid;
1-(3-chloro-5-{[4-(4-chlorothiophen-2-yl)-5-(1-cyclopentyl-octahydro-1*H*-pyrrolo[3,2-c]pyridin-5(*6H*)-yl)-thiazol-2-yl]-carbamoyl}-pyridin-2-yl)-piperidine-4-carboxylic acid;
3'-chloro-5'-[4-(4-chlorothiophen-2-yl)-5-(2,6-diazaspiro[3.3]heptan-2-yl)-thiazol-2-carbamoyl]-3,4,5,6-tetrahydro-2*H*-[1,2']bipyridine-4-carboxylic acid;
3'-chloro-5'-[4-(4-chlorothiophen-2-yl)-5-(6-iso-propyl-2,6-diazaspiro[3.3]heptan-2-yl)-thiazol-2-carbamoyl]-3,4,5,6-tetrahydro-2*H*-[1,2']bipyridine-4-carboxylic acid;
3'-chloro-5'-[4-(4-chlorothiophen-2-yl)-5-(6-cyclohexyl-2,6-diazaspiro[3.3]heptan-2-yl)-thiazol-2-carbamoyl]-3,4,5,6-tetrahydro-2*H*-[1,2']bipyridine-4-carboxylic acid;
1-(5-{[5-(2,5-diazabicyclo[2.2.1]heptan-2-yl)-4-(4-chlorothiophen-2-yl)thiazol-2-yl]carbamoyl}-3-chloropyridin-2-yl)piperidine-4-carboxylic acid;
1-(3-chloro-5{[4-(4-chlorothiophen-2-yl)-5-(5-methyl-2,5-diazabicyclo[2.2.1]heptan-2-yl)thiazol-2-yl]carbamoyl}pyridin-2-yl)piperidine-4-carboxylic acid;
1-(3-chloro-5-{[4-(4-chlorothiophen-2-yl)-5-(5-iso-propyl-2,5-diazabicyclo[2.2.1]heptan-2-yl)-thiazol-2-yl]-carbamoyl}-pyridin-2-yl)-piperidine-4-carboxylic acid;
1-(3-chloro-5-{[4-(4-chlorothiophen-2-yl)-5-(5-cyclobutyl-2,5-diazabicyclo[2.2.1]heptan-2-yl)-thiazol-2-yl]-carbamoyl}-pyridin-2-yl)-piperidine-4-carboxylic acid;
1-(3-chloro-5{[4-(4-chlorothiophen-2-yl)-5-(5-cyclopentyl-2,5-diazabicyclo[2.2.1]heptan-2-yl)-thiazol-2-yl]-carbamoyl}-pyridin-2-yl)-piperidine-4-carboxylic acid;
1-(3-chloro-5-{[4-(4-chlorothiophen-2-yl)-5-(5-cyclohexyl-2,5-diazabicyclo[2.2.1]heptan-2-yl)-thiazol-2-yl]-carbamoyl}-pyridin-2-yl)-piperidine-4-carboxylic acid;
3'-chloro-5'-[4-(4-chlorothiophen-2-yl)-5-(2,5-diazabicyclo[2.2.2]octan-2-yl)-thiazol-2-carbamoyl]-3,4,5,6-tetrahydro-2*H*-[1,2']bipyridine-4-carboxylic acid;
3'-chloro-5'-[4-(4-chlorothiophen-2-yl)-5-(5-methyl-2,5-diazabicyclo[2.2.2]octan-2-yl)-thiazol-2-carbamoyl]-3,4,5,6-tetrahydro-2*H*-[1,2']bipyridine-4-carboxylic acid;
3'-chloro-5'-[4-(4-chlorothiophen-2-yl)-5-(5-iso-propyl-2,5-diazabicyclo[2.2.2]octan-2-yl)-thiazol-2-carbamoyl]-3,4,5,6-tetrahydro-2*H*-[1,2']bipyridine-4-carboxylic acid;
3'-chloro-5'-[4-(4-chlorothiophen-2-yl)-5-(5-cyclobutyl-2,5-diazabicyclo[2.2.2]octan-2-yl)-thiazol-2-carbamoyl]-3,4,5,6-tetrahydro-2*H*-[1,2']bipyridine-4-carboxylic acid;
3'-chloro-5'-[4-(4-chlorothiophen-2-yl)-5-(5-cyclopentyl-2,5-diazabicyclo[2.2.2]octan-2-yl)-thiazol-2-carbamoyl]-3,4,5,6-tetrahydro-2*H*-[1,2']bipyridine-4-carboxylic acid;
3'-chloro-5'-[4-(4-chlorothiophen-2-yl)-5-(5-cyclohexyl-2,5-diazabicyclo[2.2.2]octan-2-yl)-thiazol-2-carbamoyl]-3,4,5,6-tetrahydro-2*H*-[1,2']bipyridine-4-carboxylic acid;
3'-chloro-5'-[4-(4-chlorothiophen-2-yl)-5-(5-cyclohexyl-octahydropyrrolo[3,4-c]pyridin-2-yl)-thiazol-2-carbamoyl]-3,4,5,6-tetrahydro-2*H*-[1,2']bipyridine-4-carboxylic acid;
3'-chloro-5'-[4-(4-chlorothiophen-2-yl)-5-(5-cyclopentyl-octahydropyrrol[3,4-c]pyridin-2-yl)-thiazol-2-carbamoyl]-3,4,5,6-tetrahydro-2*H*-[1,2']bipyridine-4-carboxylic acid;
3'-chloro-5'-[4-(4-chlorothiophen-2-yl)-5-(5-cyclohexyl-hexahydropyrrolo[3,4-b]pyrrol-1-yl)-thiazol-2-carbamoyl]-3,4,5,6-tetrahydro-2*H*-[1,2']bipyridine-4-carboxylic acid;
1-(3-chloro-5-{[4-(4-chloropyridin-2-yl)-5-(2-cyclohexyl-hexahydropyrrolo[3,4-c]pyrrol-5-yl)-thiazol-2-yl]-carbamoyl}-pyridin-2-yl)-pyridin-4-carboxylic acid;
3'-chloro-5'-[5-(1-cyclohexyl-hexahydropyrrolo[3,4-b]pyrrol-5-yl)-4-(3-trifluoromethylphenyl)-thiazol-2-carbamoyl]-3,4,5,6-tetrahydro-2*H*-[1,2']bipyridine-4-carboxylic acid;
(E)-3-{2,6-dichloro-4-[5-(1-cyclohexyl-hexahydropyrrolo[3,4-b]pyrrol-5-yl)-4-(3-trifluoromethylphenyl)-thiazol-2-carbamoyl]-phenyl}-2-methyl-acrylic acid;
(E)-3-(2,6-dichloro-4-{[4-(4-chlorothiophen-2-yl)-5-(1-cyclohexyl-hexahydropyrrolo[3,4-b]pyrrol-5(*1H*)-yl)-thiazol-2-yl]-carbamoyl}-phenyl)-2-methylacrylic acid;
(E)-3-(2,6-dichloro-4-{[4-(4-chlorothiophen-2-yl)-5-(1-cyclobutyl-hexahydropyrrolo[3,4-b]pyrrol-5(*1H*)-yl)-thiazol-2-yl]-carbamoyl}-phenyl)-2-methylacrylic acid;
(E)-3-[4-({5-[1-(3,3-difluorocyclobutyl)-hexahydropyrrolo[3,4-b]pyrrol-5(*1H*)-yl]-4-(4-chlorothiophen-2-yl)-thiazol-2-yl}-carbamoyl)-2,6-dichlorophenyl]-2-methylacrylic acid;
(E)-3-[4-({5-[1-(3-fluorocyclobutyl)-hexahydropyrrolo[3,4-b]pyrrol-5(*1H*)-yl]-4-(4-chlorothiophen-2-yl)-thiazol-2-yl}-carbamoyl)-2,6-dichlorophenyl]-2-methylacrylic acid;
(E)-3-(2,6-dichloro-4-{[4-(4-chlorothiophen-2-yl)-5-(1-cyclopentyl-hexahydropyrrolo[3,4-b]pyrrol-5(*1H*)-yl)-thiazol-2-yl]-carbamoyl}-phenyl)-2-methylacrylic acid;
(E)-3-[4-({5-[1-(3-methylcyclopentyl)-hexahydropyrrolo[3,4-b]pyrrol-5(*1H*)-yl]-4-(4-chlorothiophen-2-yl)-thiazol-2-yl}-carbamoyl)-2,6-dichlorophenyl]-2-methylacrylic acid;
(E)-3-[4-({5-[1-(N-methyl-piperidin-4-yl)-hexahydropyrrolo[3,4-b]pyrrol-5(1*H*)-yl]-4-(4-chlorothiophen-2-yl)-thiazol-2-yl}-carbamoyl)-2,6-dichlorophenyl]-2-methylacrylic acid;
(E)-3-[4-({5-[1-(4H-tetrahydropyran-4-yl)-hexahydropyrrolo[3,4-b]pyrrol-5(*1H*)-yl]-4-(4-chlorothiophen-2-yl)-thiazol-2-yl}-carbamoyl)-2,6-dichlorophenyl]-2-methylacrylic acid;
(E)-3-(4-{[5-(1-cycloheptyl-hexahydropyrrolo[3,4-b]pyrrol-5(*1H*)-yl)-4-(4-chlorothiophen -2-yl)-thiazol-2-yl]-carbamoyl}-2,6-dichlorophenyl)-2-methylacrylic acid;
(E)-3-[4-({5-[1-(bicyclo[2.2.1]hept-2-yl)-hexahydropyrrolo[3,4-b]pyrrol-5(*1H*)-yl]-4-(4-chlorothiophen-2-yl)-thiazol-2-yl}-carbamoyl)-2,6-dichlorophenyl]-2-methylacrylic acid;
(E)-3-{4-[(5-{1-[(1R,3R,5R,7R)-adamantan-2-yl]hexahydropyrrolo[3,4-b]pyrrol-5(*1H*)-yl} -4-(4-chlorothiophen-2-yl)thiazol-2-yl)carbamoyl]-2,6-dichlorophenyl}-2-methylacrylic acid;
(E)-3-{2,6-dichloro-4-[4-(4-chlorothiophen-2-yl)-5-(5-cyclohexyl-2,5-diazabicyclo[2.2.2] octan-2-yl)-thiazol-2-carbamoyl]-phenyl}-2-methyl-acrylic acid;
(E)-3-(2,6-dichloro-4-((4-(4-chlorothiophen-2-yl)-5-(5-cyclohexylhexahydro-1H-pyrrolo[3,2-c]pyridin-2(3H)-yl)thiazol-2-yl)carbamoyl)phenyl)-2-methylacrylic acid;
3'-chloro-5'-[5-(1-cyclohexyl-hexahydropyrrolo[3,4-b]pyrrol-5-yl)-4-(2-fluoro-3-trifluoromethyl phenyl)-thiazol-2-carbamoyl]-3,4,5,6-tetrahydro-*2H*-[1,2']bipyridine-4-carboxylic acid;
3-{2,6-dichloro-4-[5-(1-cyclohexyl-hexahydropyrrolo[3,4-b]pyrrol-5-yl)-4-(2-fluoro-3-trifluoromethylphenyl)-thiazol-2-carbamoyl]-phenyl}-2-methyl-acrylic acid;
(E)-3-{4-[(5-{(3aR,6aR)-1-[(1R,3R,5R,7R)-adamantan-2-yl]hexahydropyrrolo[3,4-b]pyrrol-5(*1H*)-yl}-4-(4-chlorothiophen-2-yl)thiazol-2-yl)carbamoyl]-2,6-dichlorophenyl}-2-methylacrylic acid;
(E)-3-{4-[(5-{(3aS,6aS)-1-[(1S,3S,5S,7S)-adamantan-2-yl]hexahydropyrrolo[3,4-b]pyrrol-5(*1H*)-yl}-4-(4-chlorothiophen-2-yl)thiazol-2-yl)carbamoyl]-2,6-dichlorophenyl}-2-methylacrylic acid;
or an isomer or racemate thereof, a salt thereof, an ester thereof, a solvate thereof, a chemically protected form thereof, a prodrug or metabolite thereof, a crystal form thereof, and a mixture thereof.

19. A compound represented by formula (IX), or an isomer or racemate thereof, a salt thereof, an ester thereof, a solvate thereof, a chemically protected form thereof, a prodrug or metabolite thereof, a crystal form thereof, and a mixture thereof: 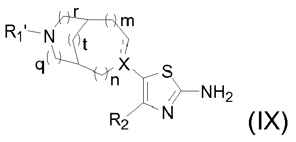
wherein m, n, q, and r are individually and separately selected from an integer of 0-4;
t is selected from an integer of 0-3;
X is N or C;
-̅-̅-̅ represents a single or double bond; when X is N, -̅-̅-̅ is a single bond;
R₁' is an amino protection group or hydrogen;
R₂ are defined as in claim 1.

20. The compound according to Claim 19, or an isomer or racemate thereof, a salt thereof, an ester thereof, a solvate thereof, a chemically protected form thereof, a prodrug or metabolite thereof, a crystal form thereof, and a mixture thereof, wherein the group 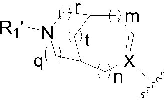 is formula (IV'): 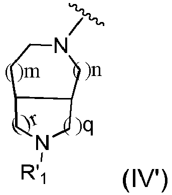 wherein m, n, q, and r are individually and separately selected from an integer of 0-4; R₁' is an amino protection group or hydrogen.

21. The compound according to Claim 19, or an isomer or racemate thereof, a salt thereof, an ester thereof, a solvate thereof, a chemically protected form thereof, a prodrug or metabolite thereof, a crystal form thereof, and a mixture thereof, wherein the group 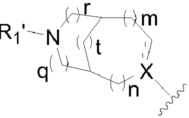 is formula (V'): 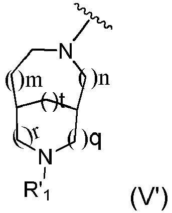
wherein m, n, q, and r are independently selected from an integer of 0-4;
t is an integer of 1-3;
R₁' is an amino protection group or hydrogen.

22. A compound, which is selected from:
tert-butyl 5-[2-amino-4-(4-chlorothiophen-2-yl)-thiazol-5-yl]-hexahydropyrrolo[3,4-b]pyrrole-1(*2H*)-carboxylate;
tert-butyl 5-[4-(4-chlorothiophen-2-yl)-2-(5,6-dichloro-pyridin-3-carbonylamino)-thiazol-5-yl]-hexahydropyrrolo[3,4-b]pyrrole-1(*2H*)-carboxylate;
tert-butyl 5-[2-{5-chloro-6-[4-(ethoxycarbonyl)-piperidin-1-yl]-nicotinamido}-4-(4-chlorothiophen-2-yl)-thiazol-5-yl]-hexahydropyrrolo[3,4-b]pyrrole-1(*2H*)-carboxylate;
ethyl 1-(3-chloro-5-{[4-(4-chlorothiophen-2-yl)-5-(hexahydropyrrolo[3,4-b]pyrrol-5(*1H*)-yl)-thiazol-2-yl]-carbamoyl}-pyridin-2-yl)-piperidine-4-carboxylate;
ethyl 1-(3-chloro-5-{[4-(4-chlorothiophen-2-yl)-5-(1-methyl-hexahydropyrrolo[3,4-b]pyrrol-5(*1 H*)-yl)-thiazol-2-yl]-carbamoyl}-pyridin-2-yl)-piperidine-4-carboxylate;
tert-butyl 6-[2-amino-4-(4-chlorothiophen-2-yl)thiazol-5-yl]octahydro-*1H*-pyrrolo[3,4-b]pyridinecarboxylate;
tert-butyl 6-(4-(4-chlorothiophen-2-yl)-2-(5,6-dichloronicotinamido)thiazol-5-yl)octahydro-1 H-pyrrolo[3,4-b]pyridine-1-carboxylate;
tert-butyl 6-(2-{5-chloro-6-[4-(ethoxycarbonyl)piperidin-1-yl]-nicotinamido}-4-(4-chlorothiophen-2-yl)-thiazol-5-yl)-octahydro-*1H*-pyrrolo[3,4-b]pyridin-carboxylate;
ethyl 1-(3-chloro-5-{[4-(4-chlorothiophen-2-yl)-5-(octahydropyrrolo[3,4-b]pyridin-6(*1H*)-yl)-thiazol-2-yl]-carbamoyl}pyridin-2-yl)-piperidine-4-carboxylate;
ethyl 1-(3-chloro-5-{[4-(4-chlorothiophen-2-yl)-5-(1-cyclopentyl-octahydropyrrolo[3,4-b]pyridin-6(*1H*)-yl)-thiazol-2-yl]-carbamoyl}-pyridin-2-yl)-piperidine-4-carboxylate;
tert-butyl 5-[2-amino-4-(4-chlorothiophen-2-yl)thiazol-5-yl]-octahydro-*1H*-pyrrolo[3,2-c]pyridin-1-carboxylate;
tert-butyl 5-[4-(4-chlorothiophen-2-yl)-2-(5,6-dichloronicotinamide)-thiazol-5-yl]-octahydro-*1H*-pyrrolo[3,2-c]pyridin-1-carboxylate;
tert-butyl 5-[2-{5-chloro-6-[4-(ethoxycarbonyl)-piperidin-1-yl]-nicotinamido}-4-(4-chlorothiophen-2-yl)-thiazol-5-yl]-octahydro-*1H*-pyrrolo[3,2-c]pyridin-1-carboxylate;
ethyl1-(3-chloro-5-{[4-(4-chlorothiophen-2-yl)-5-(octahydro-*1H*-pyrrol[3,2-c]-pyridin-5(*6H*)-yl )-thiazol-2-yl]-carbamoyl}-pyridin-2-yl)-piperidine-4-carboxylate;
ethyl 1-(3-chloro-5-{[4-(4-chlorothiophen-2-yl)-5-(1-cyclohexyloctahydro-*1H*-pyrrolo[3,2-c]pyridin-5(*6H*)-yl)-thiazol-2-yl]-aminoformamide}-pyridin-2-yl)-piperidine-4-carboxylate;
tert-butyl 6-[2-amino-4-(4-chloro-thiophen-2-yl)-thiazol-5-yl]-2,6-diazaspiro[3.3]heptane-2-carboxylate;
tert-butyl 6-(4-(4-chlorothiophen-2-yl)-2-(5,6-dichloronicotinamido)thiazol-5-yl)-2,6-diazaspiro[3.3]heptane-2-carboxylate;
ethyl 5'-[5-(6-tert-butyloxycarbonyl-2,6-diazaspiro[3.3]heptan-2-yl-4-(4-chloro-thiophen-2-yl)-thiazol-2-carbamoyl]-3'-chloro-3,4,5,6-tetrahydro-*2H*-[1,2']bipyridine-4-carboxylate;
ethyl 3'-chloro-5'-[4-(4-chloro-thiophen-2-yl)-5-(2,6-diazaspiro[3.3]heptan-2-yl)-thiazol-2-carbamoyl]-3,4,5,6-tetrahydro-*2H*-[1,2']bipyridine-4-carboxylate;
ethyl 3'-chloro-5'-[4-(4-chlorothiophen-2-yl)-5-(6-iso-propyl-2,6-diazaspiro[3.3]heptan-2-yl)-thiazol-2-carbamoyl]-3,4,5,6-tetrahydro-*2H*-[1,2']bipyridine-4-carboxylate;
tert-butyl 5-[2-amino-4-(4-chlorothiophen-2-yl)-thiazol-5-yl]-2,5-diazabicyclo[2.2.1]heptane-2-carboxylate;
tert-butyl 5-(2-amino-4-(4-chlorothiophen-2-yl)-thiazol-5-yl)-2,5-diazabicyclo[2.2.1]heptane-2-carboxylate;
tert-butyl 5-[4-(4-chlorothiophen-2-yl)-2-(5,6-dichloropyridin-3-formamide)-thiazol-5-yl]-2,5-diazabicyclo[2.2.1]heptane-2-carboxylate;
tert-butyl 5-(2-{5-chloro-6-[4-(ethoxycarbonyl)piperidin-1-yl]nicotinamido}-4-(4-chlorothiophen-2-yl)thiazol-5-yl)-2,5-diazabicyclo[2.2.1]heptane-2-carboxylate;
1-[5-({5-[5-(2-tert-butoxycarbonyl)-2,5-diazabicyclo[2.2.1]heptan-2-yl]-4-(4-chlorothiophen-2-yl)-thiazol-2-yl}carbamoyl)-3-chloropyridin-2-yl]piperidine-4-carboxylic acid;
ethyl 1-(5-{[5-(-2,5-diazabicyclo[2.2.1]heptan-2-yl)-4-(4-chlorothiophen-2-yl)-thiazol-2-yl]-carbamoyl}-3-chloropyridin-2-yl)-piperidine-4-carboxylate;
ethyl 1-(3-chloro-5-{[4-(4-chlorothiophen-2-yl)-5-(5-methyl-2,5-diazabicyclo[2.2.1]heptan-2-yl)thiazol-2-yl]carbamoyl}pyridin-2-yl)piperidine-4-carboxylate;
tert-butyl 5-[2-amino-4-(4-chloro-thiophen-2-yl)-thiazol-5-yl]-2,5-diazabicyclo[2.2.2]octane-2-carboxylate;
tert-butyl 5-(4-(4-chloro-thiophen-2-yl)-2-((5,6-dichloro-pyridin-3-carbonyl)-amino)-thiazol-5-yl)-2,5-diazabicyclo[2.2.2]octane-2-carboxylate;
ethyl 5'-[5-(5-tert-butyloxycarbonyl-2,5-diazabicyclo[2.2.2]octan-2-yl)-4-(4-chloro-thiophen-2-yl)-thiazol-2-carbamoyl]-3'-chloro-3,4,5,6-tetrahydro-*2H*-[1,2']bipyridine-4-carboxylate;
ethyl 5'-[5-(2,5-diazabicyclo[2.2.2]octan-2-yl)-4-(4-chloro-thiophen-2-yl)-thiazol-2-carbamoyl] -3'-chloro-3,4,5,6-tetrahydro-*2H*-[1,2']bipyridine-4-carboxylate;
ethyl 3'-chloro-5'-[4-(4-chlorothiophen-2-yl)-5-(5-methyl-2,5-diazabicyclo[2.2.2]octan-2-yl)-thiazol-2-carbamoyl]-3,4,5,6-tetrahydro-*2H*-[1,2']bipyridine-4-carboxylate;
tert-butyl 2-[2-amino-4-(4-chloro-thiophen-2-yl)-thiazol-5-yl]-octahydropyrrolo[3,4-c]pyridin-5-carboxylate;
tert-butyl 2-(4-(4-chlorothiophen-2-yl)-2-(5,6-dichloronicotinamido)thiazol-5-yl)hexahydro-1H-pyrrolo[3,4-c]pyridine-5(6H)-carboxylate;
ethyl 5'-[5-(5-tert-butyloxycarbonyl-octahydropyrrolo[3,4-c]pyridin-2-yl)-4-(4-chloro-thiophen-2-yl)-thiazol-2-carbamoyl]-3'-chloro-3,4,5,6-tetrahydro-*2H*-[1,2']bipyridine-4-carboxylate;
ethyl 3'-chloro-5'-[4-(4-chloro-thiophen-2-yl)-5-(octahydropyrrolo[3,4-c]pyridin-2-yl)-thiazol-2-carbamoyl]-3,4,5,6-tetrahydro-*2H*-[1,2']bipyridine-4-carboxylate;
ethyl 3'-chloro-5'-[4-(4-chloro-thiophen-2-yl)-5-(5-cyclohexyl-octahydropyrrolo[3,4-c]pyridin-2 -yl)-thiazol-2-carbamoyl]-3,4,5,6-tetrahydro-*2H*-[1,2']bipyridine-4-carboxylate;
tert-butyl 1-[2-amino-4-(4-chloro-thiophen-2-yl)-thiazol-5-yl]-hexahydropyrrolo[3,4-b]pyrrole-5-carboxylate;
tert-butyl-1-(4-(4-chlorothiophen-2-yl)-2-(5,6-dichloronicotinamido)thiazol-5-yl)hexahydropyrrolo[3,4-b]pyrrole-5(1H)-carboxylate;
ethyl 5'-[5-(5-tert-butyloxycarbonyl-hexahydropyrrolo[3,4-b]pyrrol-1-yl)-4-(4-chloro-thiophen-2-yl)-thiazol-2-carbamoyl]-3'-chloro-3,4,5,6-tetrahydro-*2H*-[1,2']bipyridine-4-carboxylate;
ethyl 3'-chloro-5'-[4-(4-chloro-thiophen-2-yl)-5-(hexahydropyrrolo[3,4-b]pyrrol-1-yl)-thiazol-2-carbamoyl]-3,4,5,6-tetrahydro-*2H*-[1,2']bipyridine-4-carboxylate;
ethyl 3'-chloro-5'-[4-(4-chloro-thiophen-2-yl)-5-(5-cyclohexyl-hexahydropyrrolo[3,4-b]pyrrol-1 -yl)-thiazol-2-carbamoyl]-3,4,5,6-tetrahydro-*2H*-[1,2']bipyridine-4-carboxylate;
tert-butyl 5-[2-amino-4-(4-chlorothiophen-2-yl)-thiazol-5-yl]hexahydropyrrolo[3,4-c]pyrrole-2(*1H)-*carboxylate;
tert-butyl 5-[4-(4-chlorothiophen-2-yl)-2-(5,6-dichloropyridin-3-formamide)-thiazol-5-yl]hexahydropyrrolo[3,4-c]pyrrole-2(*1H*)-carboxylate;
tert-butyl 5-{2-[5-chloro-6-(4-ethoxycarbonyl-piperidin-1-yl)nicotinamide]-4-(4-chlorothiophen-2-yl)thiazol-5-yl}hexahydropyrrolo[3,4-c]pyrrole-2(*1H*)-carboxylate;
ethyl 1-(3-chloro-5-{[4-(4-chlorothiophen-2-yl)-5-(hexahydropyrrolo[3,4-c]pyrrol-2(*1H*)-yl)-thiazol-2-yl]-carbamoyl}-pyridin-2-yl)-piperidine-4-carboxylate;
ethyl 1-(3-chloro-5-{[4-(4-chlorothiophen-2-yl)-5-(5-cyclohexyl-hexahydropyrrolo[3,4-c]pyrrol-2(*1H*)-yl)-thiazol-2-yl]carbamoyl}-pyridin-2-yl)-piperidine-4-carboxylate;
tert-butyl 5-[2-amino-4-(3-trifluoromethylphenyl)-thiazol-5-yl]-hexahydropyrrolo[3,4-b]pyrrole-1-carboxylate;
tert-butyl 5-[2-[(5,6-dichloro-pyridin-3-carbonyl)-amino]-4-(3-trifluoromethylphenyl)-thiazol-5-yl]-hexahydropyrrolo[3,4-b]pyrrole-1-carboxylate;
ethyl 5'-[5-(1-tert-butyloxycarbonyl-hexahydropyrrolo[3,4-b]pyrrol-5-yl)-4-(3-trifluoromethylphenyl)-thiazol-2-carbamoyl]-3'-chloro-3,4,5,6-tetrahydro-*2H*-[1,2']bipyridine-4-carboxylat e;
ethyl 3'-chloro-5'-[5-(hexahydropyrrolo[3,4-b]pyrrol-5-yl)-4-(3-trifluoromethylphenyl)-thiazol-2 -carbamoyl]-3,4,5,6-tetrahydro-*2H*-[1,2']bipyridine-4-carboxylate;
ethyl 3'-chloro-5'-[5-(1-cyclohexyl-hexahydropyrrolo[3,4-b]pyrrol-5-yl)-4-(3-trifluoromethylphenyl)-thiazol-2-carbamoyl]-3,4,5,6-tetrahydro-*2H*-[1,2']bipyridine-4-carboxylate;
tert-butyl (E)-5-[2-[3,5-dichloro-4-(2-ethoxycarbonyl-propenyl)-benzoylamino]-4-(3-trifluoromethyl-phenyl)-thiazol-5-yl]-hexahydropyrrolo[3,4-b]pyrrole-1-carboxylate;
(E)-3-{2,6-dichloro-4-[5-(hexahydropyrrolo[3,4-b]pyrrol-5-yl)-4-(3-trifluoromethylphenyl) -thiazol-2-carbamoyl]-phenyl}-2-methyl-acrylic acid ethyl ester;
(E)-3-{2,6-dichloro-4-[5-(1-cyclohexyl-hexahydropyrrolo[3,4-b]pyrrol-5-yl)-4-(3-trifluoromethylphenyl)-thiazol-2-carbamoyl]-phenyl}-2-methyl-acrylic acid ethyl ester; tert-butyl
(E)-5-{4-(4-chlorothiophen-2-yl)-2-[3,5-dichloro-4-(3-ethoxy-2-methyl-3-oxo-propenyl)-benzoylamino]-thiazol-5-yl}-hexahydropyrrolo[3,4-b]pyrrole-1(*2H*)-carboxylate;
(E)-3-(2,6-dichloro-4-{[5-(4-chlorothiophen-2-yl)-4-(hexahydropyrrolo[3,4-b]pyrrol-5(*1H* )-yl)-thiazol-2-yl]-carbamoyl}-phenyl)-2-methylacrylic acid ethyl ester;
(E)-3-(2,6-dichloro-4-{[4-(4-chlorothiophen-2-yl)-5-(1-cyclohexyl-hexahydropyrrolo[3,4-b]pyrrol-5(*1H*)-yl)-thiazol-2-yl]-carbamoyl}-phenyl)-2-methylacrylic acid ethyl ester;
tert-butyl (3aR,6aR)-5-[2-amino-4-(4-chlorothiophen-2-yl)-thiazol-5-yl]-hexahydropyrrolo[3,4-b]pyrrole-1(2*H*)-carboxylate;
tert-butyl (E)-(3aR,6aR)-5-{4-(4-chlorothiophen-2-yl)-2-[3,5-dichloro-4-(3-ethoxy-2-methyl-3-oxo-1-propenyl)-benzoylamino]thiazol-5-yl}hexahydropyrrolo[3,4-b]pyrrole-1(2*H*)-carboxylate;
(E)-(3aR,6aR)-3-(2,6-dichloro-4-{[4-(4-chlorothiophen-2-yl)-5-(hexahydropyrrolo[3,4-b] pyrrol-5(*1H*)-yl)-thiazol-2-yl]-carbamoyl}-phenyl)-2-methylacrylic acid ethyl ester;
(E)-3-{4-[(5-{(3aR,6aR)-1-[(1R,3R,5R,7R)-adamantan-2-yl]hexahydropyrrolo[3,4-b]pyrrol-5(*1H*)-yl}-4-(4-chlorothiophen-2-yl)-thiazol-2-yl)-carbamoyl]-2,6-dichlorophenyl}-2-methylacrylic acid ethyl ester;
tert-butyl (3aS,6aS)-5-[2-amino-4-(4-chlorothiophen-2-yl)-thiazol-5-yl]-hexahydropyrrolo[3,4-b]pyrrole-1(*2H*)-carboxylate;
(E)-(3aS,6aS)-5-{4-(4-chlorothiophen-2-yl)-2-[3,5-dichloro-4-(3-ethoxy-2-methyl-3-oxo-tert-butyl 1-propenyl)-benzoylamino]thiazol-5-yl}hexahydropyrrolo[3,4-b]pyrrole-1(2*H*)-carboxylate;
(E)-(3aS,6aS)-3-(2,6-dichloro-4-{[4-(4-chlorothiophen-2-yl)-5-(hexahydropyrrolo[3,4-b] pyrrol-5(*1H*)-yl)-thiazol-2-yl]-carbamoyl}-phenyl)-2-methylacrylic acid ethyl ester;
(E)-3-{4-[(5-{(3aS,6aS)-1-[(1S,3S,5S,7S)-adamantan-2-yl]hexahydropyrrolo[3,4-b]pyrrol-5(*1H*)-yl}-4-(4-chlorothiophen-2-yl)-thiazol-2-yl)-carbamoyl]-2,6-dichlorophenyl}-2-methylacrylic acid ethyl ester;
or an isomer or racemate thereof, a salt thereof, an ester thereof, a solvate thereof, a chemically protected form thereof, a prodrug or metabolite thereof, a crystal form thereof, and a mixture thereof.

23. A process for preparing the compound according to any of Claims 1-22, or an isomer or racemate thereof, a salt thereof, an ester thereof, a solvate thereof, a chemically protected form thereof, a prodrug or metabolite thereof, a crystal form thereof, and a mixture thereof, which comprises: 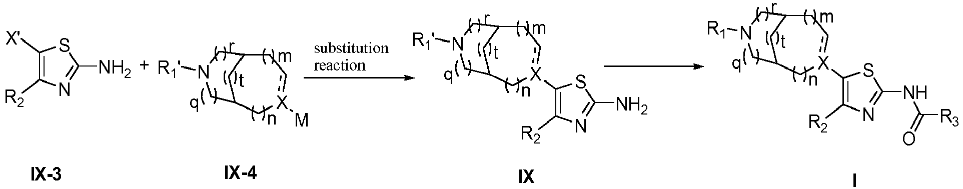
wherein M is H, Li, Na, K, Si, Mg, Zn, boric acid group or boric acid ester group; R₁, R₂, R₃, m, n, q, r and t are defined as in Claim 1-14, X' is halogen, R₁' is an amino protection group or hydrogen;
wherein compound IX-3 and compound IX-4 are subjected to a substitution reaction to produce compound IX; then the resulting compound IX is subjected to an acylation reaction, a substitution reaction, a deprotection reaction, a reductive amination reaction, a hydrolysis reaction or a combination thereof, to finally produce the desired compound I.

24. The process according to Claim 23, wherein said substitution reaction can be carried out in presence or absence of a metal catalyst.

25. A pharmaceutical composition, which contains the compound according to any of Claim 1-22 or an isomer or racemate thereof, a salt thereof, an ester thereof, a solvate thereof, a chemically protected form thereof, a prodrug or metabolite thereof, a crystal form thereof, and a mixture thereof, and a pharmaceutically acceptable carrier.

26. Use of the compound according to any of Claims 1-22, or an isomer or racemate thereof, a salt thereof, an ester thereof, a solvate thereof, a chemically protected form thereof, a prodrug or metabolite thereof, a crystal form thereof, and a mixture thereof, in manufacture of a thrombopoietin receptor agonist drug.

27. Use of the compound according to any of Claims 1-22, or an isomer or racemate thereof, a salt thereof, an ester thereof, a solvate thereof, a chemically protected form thereof, a prodrug or metabolite thereof, a crystal form thereof, and a mixture thereof in manufacture of a medicament for treatment and/or prevention of thrombopoietin receptor mediated diseases.

28. Use according to Claim 26 or 27, wherein said thrombopoietin receptor mediated disease is chronic idiopathic thrombocytopenic purpura.

29. A method for prevention and/or treatment of thrombopoietin receptor mediated diseases or conditions with the compound according to any of Claims 1-22, or an isomer or racemate thereof, a salt thereof, an ester thereof, a solvate thereof, a chemically protected form thereof, a prodrug or metabolite thereof, a crystal form thereof, and a mixture thereof, in combination with another drug, particularly with at least one of other thrombopoietin receptor agonist drugs.

30. Use of the compound according to any of Claims 1-22, or an isomer or racemate thereof, a salt thereof, an ester thereof, a solvate thereof, a chemically protected form thereof, a prodrug or metabolite thereof, a crystal form thereof, and a mixture thereof, in combination with another drug, particularly with at least one of other thrombopoietin receptor agonist drugs, in manufacture of a medicament for prevention and/or treatment of thrombopoietin receptor mediated diseases or conditions.
